# EUROPEAN PATENT APPLICATION

(11) **EP 4 582 410 A1**
(43) Date of publication of application: **09.07.2025**
(21) Application number: 23914120.3
(22) Date of filing: 19.01.2023
(51) Int. Cl.: C07C 229/16, C07C 229/12, C07C 323/12, C07C 67/08, C07C 67/11, C07C 69/63, C07C 227/08, C07C 213/02, C07C 219/06, C07C 319/24, C07C 319/22, C07C 319/20, A61K 9/51, A61K 47/18, A61P 31/00, A61P 35/00, A61P 37/02, A61P 31/10, A61P 25/00, A61P 9/00, A61P 13/12

(54) **CATIONIC LIPID COMPOUND WITH HIGH EFFICIENCY, LOW TOXICITY AND STABLE EXPRESSION, AND COMPOSITION COMPRISING SAME**

(30) Priority: 05.01.2023 CN 202310010915
(71) Applicant: Hangzhou Tianlong Pharmaceutical Co., Ltd., Hangzhou, Zhejiang 310009 (CN)
(72) Inventor: SONG, Gengshen, Beijing 100176 (CN); ZHANG, Honglei, Beijing 100176 (CN); WANG, Huanyu, Beijing 100176 (CN); CHEN, Xichao, Beijing 100176 (CN); HUANG, Dawei, Beijing 100176 (CN); YU, Xiaowen, Beijing 100176 (CN)
(74) Representative: Potter Clarkson
(86) International application number: PCT/CN2023/073229
(87) International publication number: WO 2024/145964

(57) **Abstract**

The present invention provides a cationic lipid compound with high efficiency, low toxicity and stable expression, which is a compound of formula (I) or an N-oxide, a solvate, a pharmaceutically acceptable salt or a stereoisomer thereof. Further provided are a composition comprising the described compound and a use thereof in delivering a therapeutic or prophylactic agent.

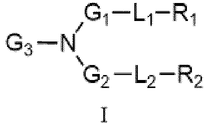

## Description

This application claims the priority to Chinese patent application No. 202310010915.X filed on January 5, 2023, the content of which is incorporated herein by reference as part of the present application.

### FIELD OF THE INVENTION

The present disclosure belongs to the field of medicine. The present disclosure specifically relates to a cationic lipid compound, a composition containing the same and uses thereof.

### BACKGROUND OF THE INVENTION

Effective targeted delivery of biologically active substances such as small molecule drugs, peptides, proteins and nucleic acids, especially nucleic acids, is a persistent medical problem. Nucleic acid therapeutics face great challenges due to low cell permeability and high susceptibility to degradation of certain nucleic acid molecules, including RNA.

It has been demonstrated that compositions, liposomes and liposome complexes (lipoplexes) containing a cationic lipid as delivery carriers can effectively deliver biologically active substances, such as small molecule drugs, polypeptides, proteins and nucleic acids, into cells and/or intracellular compartments. These compositions generally comprise one or more "cationic" and/or amino (ionizable) lipids, and also comprise a neutral lipid, a structured lipid, and a polymer-conjugated lipid. Cationic and/or ionizable lipids include, for example, amine-containing lipids that can be readily protonated. Although a variety of such lipid-containing nanoparticle compositions have been demonstrated, safety, efficacy and specificity remain to be improved. Notably, the increased complexity of lipid nanoparticles (LNPs) complicates their production and may increase their toxicity, which is a major concern that may limit their clinical application. For example, LNP siRNA particles such as patisiran require preadministration of steroids and antihistamines to eliminate unwanted immune responses (T. Coelho, D. Adams, A. Silva, et al., Safety and efficacy of RNAi therapy for transthyretin amyloidosis, N Engl J Med, 369 (2013) 819-829). Accordingly, there is still a need to develop improved cationic lipid compounds, and compositions comprising the same, that facilitate the delivery of therapeutic and/or prophylactic agents such as nucleic acids to cells.

### SUMMARY OF THE INVENTION

One aspect of the present disclosure provides a novel cationic lipid compound, which is a compound of formula (I) 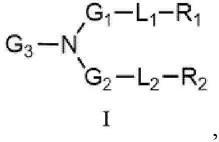 or an N-oxide, a solvate, a pharmaceutically acceptable salt or a stereoisomer thereof, wherein:
G₁ is C_{1~6} alkylene;
G₂ is C_{2~8} alkylene;
L₁ is -C(O)O- or -OC(O)-;
L₂ is -C(O)O- or -OC(O)-;
R₁ is C_{6~15} linear alkyl or R₃-S-R₄, R₅-S-S-R₆;
R₂ is C_{12~25} branched alkyl;
G₃ is: HO-R₇- or (CH₃)₂N(CH₂)₃C(O)O(CH₂)₂-;
wherein R₃ is C_{1~7} alkylene; R₄ is C_{1~7} alkyl;
R₅ is C_{1~7} alkylene; R₆ is C_{1~7} alkyl;
R₇ is C_{2~8} alkylene, C₅₋₆ cycloalkylene, -CH₂CH(OH)CH₂-, -(CH₂CH₂O)ₘ-(CH₂)ₙ- or -(CH₂)ₘ-O-(CH₂)ₙ-, where m and n are each independently 1, 2, 3 or 4.

For example, the compound of formula (I) has one of the following structures:

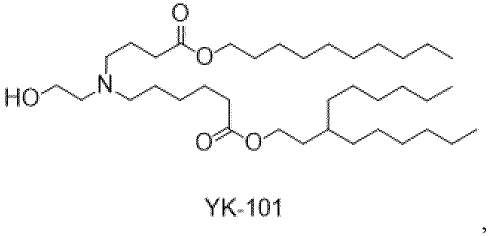

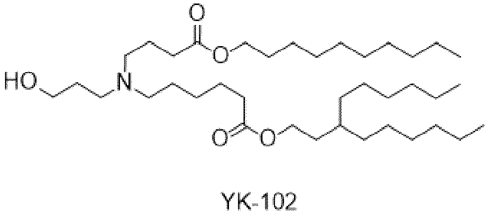

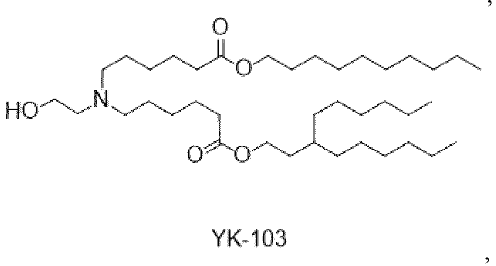

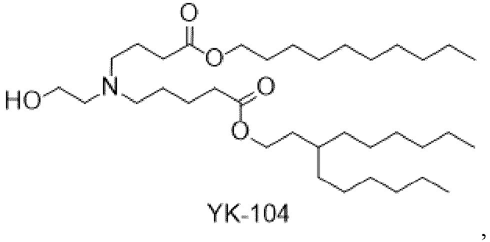

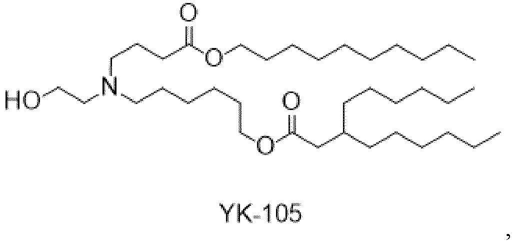

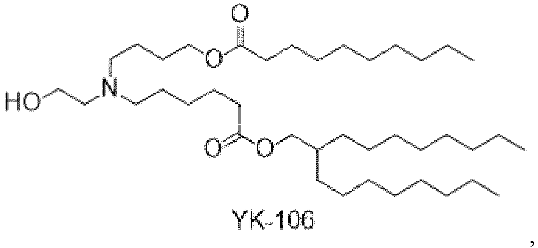

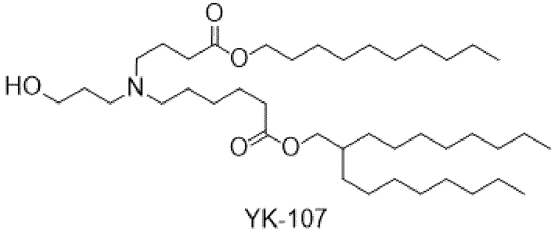

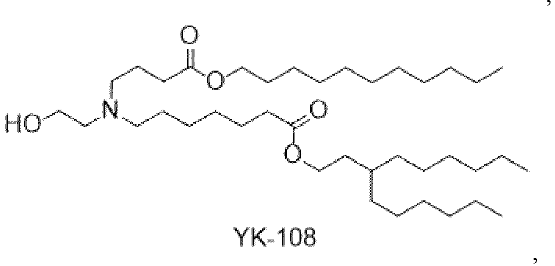

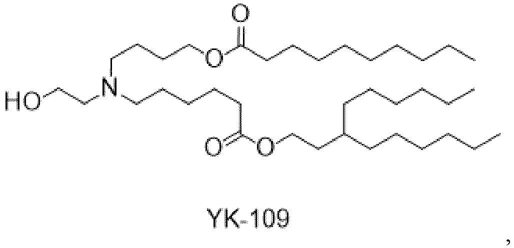

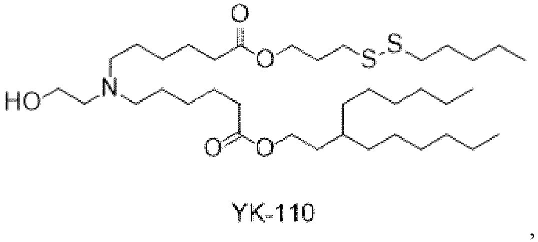

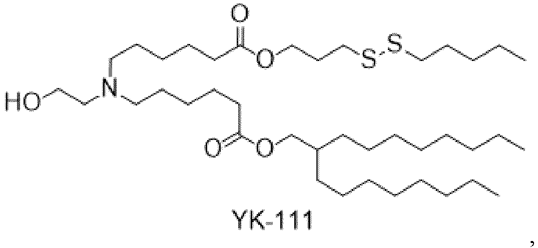

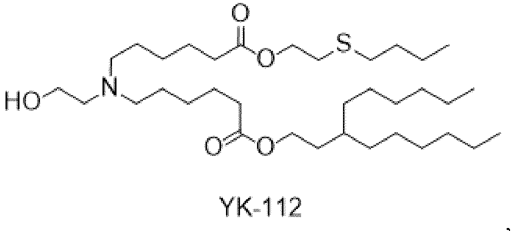

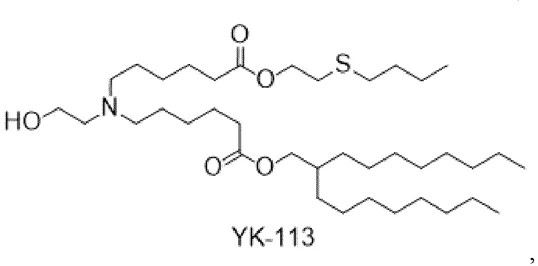

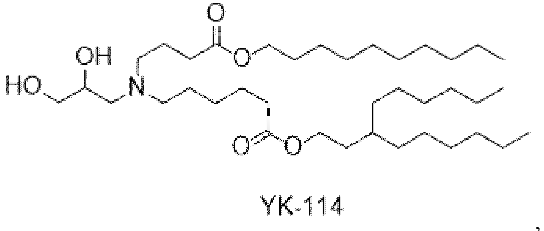

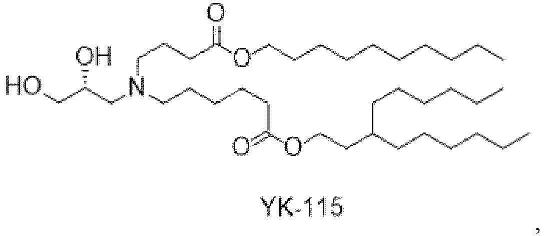

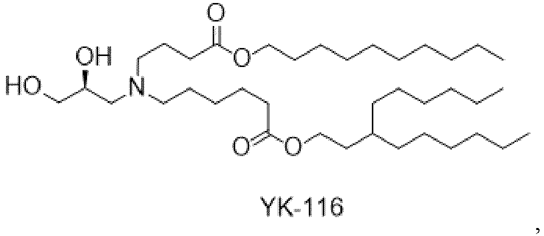

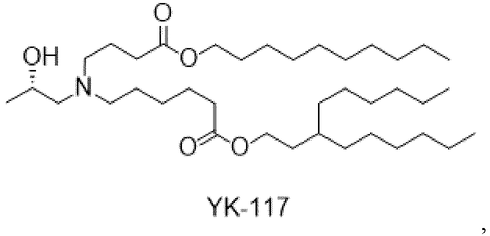

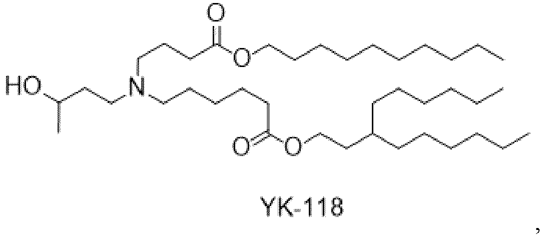

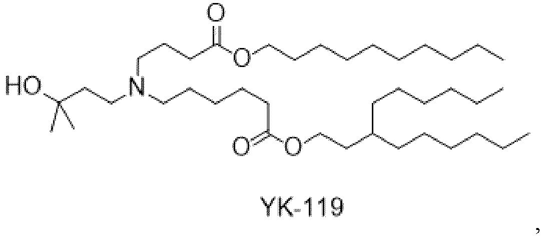

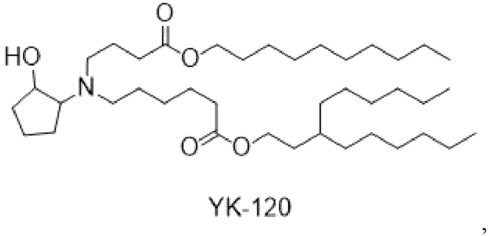

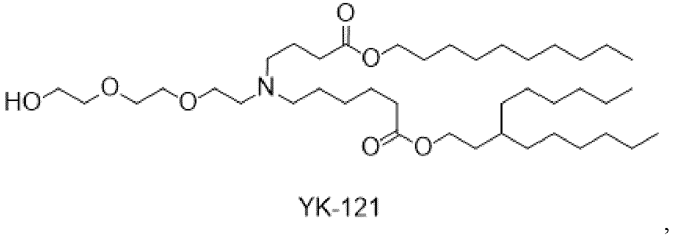

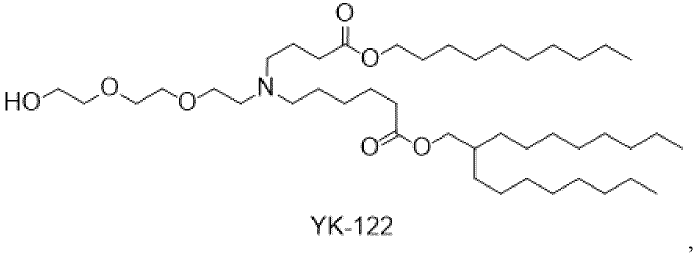

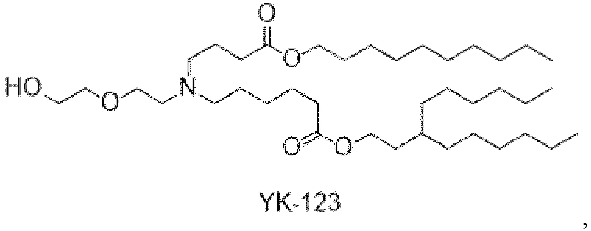

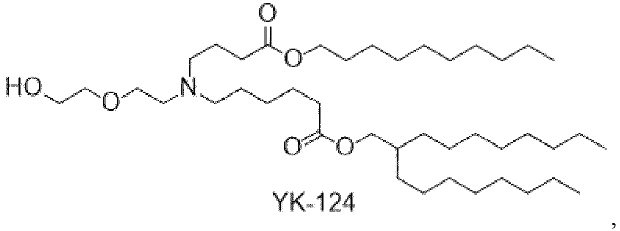

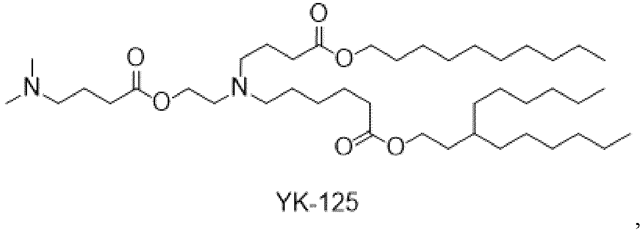

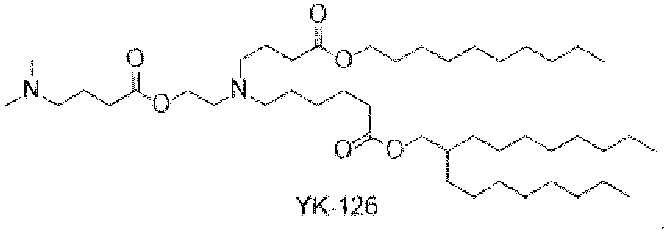

Another aspect of the present disclosure provides a composition, which comprises a carrier comprising a cationic lipid which comprises the compound of formula (I) described above or an N-oxide, a solvate, a pharmaceutically acceptable salt or a stereoisomer thereof.

Yet another aspect of the present disclosure provides the use of the compound of formula (I) described above or an N-oxide, a solvate, a pharmaceutically acceptable salt or a stereoisomer thereof or the composition described above in the manufacture of a nucleic acid medicine, a gene vaccine medicine, a small molecule medicine, a peptide or protein medicine.

Yet another aspect of the present disclosure provides the use of the compound of formula (I) described above or an N-oxide, a solvate, a pharmaceutically acceptable salt or a stereoisomer thereof or the composition described above in the manufacture of a medicament for treating a disease or condition in a mammal in need thereof.

### BRIEF DESCRIPTION OF THE DRAWINGS

Fig. 1 shows the results of cell transfection tests with different weight ratios of the carrier to mRNA used in the preparation of LNP formulations, wherein ***a*** has carrier : mRNA of 5:1, ***b*** has carrier : mRNA of 15: 1, c has carrier : mRNA of 35: 1, and ***d*** is a blank control.
Fig. 2 shows the results of cell transfection tests with different molar ratios of the cationic lipid to the neutral lipid DSPC used in the preparation of LNP formulations, wherein ***a*** is 3: 1, ***b*** is 4: 1, ***c*** is 4.9:1, and ***d*** is a blank control.
Fig. 3 shows the results of cell transfection tests with different molar ratios of polymer-conjugated lipid to carrier in the preparation of LNP formulations, wherein ***a*** is 1.5%, ***b*** is 10%, and c is a blank control.
Fig. 4 shows the results of cell transfection tests with different ratios of the cationic lipid, the neutral lipid DSPC, the structural lipid cholesterol and the polymer-conjugated lipid DMG-PEG2000 in the carrier in the preparation of LNP formulations, wherein ***a*** is 30:10: 58.5:1.5, ***b*** is 40:10:48.5:1.5, c is 49:10:39.5:1.5, ***d*** is a blank control.
Fig. 5 shows the fluorescence absorption intensity of LNP formulations of Fluc-mRNA prepared from different cationic lipids (a: YK-101; b: YK-107; c: YK-108; d: SM-102).
Fig. 6 shows the fluorescence absorption intensity of LNP formulations of Fluc-mRNA prepared from different cationic lipids (a: YK-102; b: YK-104; c: YK-106; d: compound 21).
Fig. 7 shows the fluorescence absorption intensity of LNP formulations of Fluc-mRNA prepared from different cationic lipids (a: YK-101; b: YK-108; c: YK-112; d: YK-119).
Fig. 8 shows the cell survival rate after introducing the LNP formulations of Fluc-mRNA prepared from different cationic lipids (YK-101, YK-107, YK-108, YK-009, SM-102, ALC-0315, compound 21, compound 23 and HHMA) and a Lipofectamine 3000 formulation containing Fluc-mRNA into the cell culture medium and culturing for 24 hours.
Fig. 9 shows the cell survival rate after introducing the LNP formulations of Fluc-mRNA prepared from different cationic lipids (YK-101, YK-107, YK-108, YK-102, YK-103, YK-104, YK-105, YK-106, YK-109, SM-102, ALC-0315, compound 21, compound 23 and HHMA) and a Lipofectamine 3000 formulation containing Fluc-mRNA into the cell culture medium and culturing for 24 hours.
Fig. 10 shows the cell survival rate after introducing the LNP formulations of Fluc-mRNA prepared from different cationic lipids (YK-101, YK-107, YK-108, YK-110, YK-111, YK-112, YK-113, YK-114, YK-115, YK-116, YK-117, YK-118, YK-119, YK-120, SM-102, ALC-0315, compound 21, compound 23 and HHMA) and a Lipofectamine 3000 formulation containing Fluc-mRNA into the cell culture medium and culturing for 24 hours.
Fig. 11 shows the cell survival rate after introducing the LNP formulations of Fluc-mRNA prepared from different cationic lipids (YK-101, YK-107, YK-108, YK-121, YK-122, YK-123, YK-124, YK-125, YK-126, SM-102, ALC-0315, compound 21, compound 23 and HHMA) and a Lipofectamine 3000 formulation containing Fluc-mRNA into the cell culture medium and culturing for 24 hours.
Fig. 12 shows the results of mouse *in vivo* imaging tests of LNP formulations of Fluc-mRNA prepared from different cationic lipids (YK-101, YK-102, YK-109, YK-120, SM-102, ALC-0315, compound 21, compound 23 and HHMA).
Fig. 13 shows the results of mouse *in vivo* imaging tests of LNP formulations of Fluc-mRNA prepared from different cationic lipids (YK-106, YK-107, YK-111, YK-123, SM-102, ALC-0315, compound 21, compound 23 and HHMA).
Fig. 14 shows the results of mouse *in vivo* imaging tests of LNP formulations of Fluc-mRNA prepared from different cationic lipids (YK-104, YK-108, YK-114, YK-126, YK-009, SM-102, ALC-0315, compound 21, compound 23 and HHMA).

### DETAILED DESCRIPTION OF THE INVENTION

In order to make the purpose, technical solutions and advantages of the examples of the present disclosure clearer, the technical solutions of the examples of the present disclosure will be clearly and completely described below in conjunction with the drawings of the examples of the present disclosure. Apparently, the described examples are some of the examples of the present disclosure, not all of them. Based on the described examples of the present disclosure, all other examples obtained by persons of ordinary skill in the art without creative efforts shall fall within the protection scope of the present disclosure.

The present disclosure can be conducted in other specific forms without departing from essential attributes of the present disclosure. It should be understood that any and all embodiments of the present disclosure can be combined with technical features in any other embodiment(s) to obtain additional embodiments under the premise of no conflict. The present disclosure includes additional embodiments obtained from such combinations.

All publications and patents mentioned in this disclosure are incorporated herein by reference in their entirety. If usage or terminology used in any publications and patents incorporated by reference conflicts with usage or terminology used in the present disclosure, the usage and terminology in the present disclosure shall prevail.

The section headings used herein are for the purpose of organizing the article only and should not be construed as limitations on the subject matter described.

Unless defined otherwise, all technical and scientific terms used herein have their ordinary meanings in the art to which the claimed subject matter belongs. In the event that more than one definitions exist for a term, the definition herein prevails.

Except in the working examples or where otherwise indicated, all numbers stating quantitative properties such as dosages in the specification and claims are to be understood as modified in all instances by the term "about". It is also to be understood that any numerical range recited herein is intended to include all sub-ranges within that range and any combination of various endpoints of such ranges or sub-ranges.

The words "comprising", "including" or "containing" and similar words used in the present disclosure mean that the element appearing before the word covers the elements listed after the word and their equivalents, and does not exclude unrecited elements. The terms "comprising" or " including (containing)" used herein can be open, semi-closed and closed. In other words, the terms also include "consisting essentially of", or "consisting of".

The term "pharmaceutically acceptable" in this application means that a compound or composition is chemically and/or toxicologically compatible with the other ingredients making up the formulation and/or with the human or mammal in which it is used to prevent or treat a disease or condition.

The term "subject" or "patient" as used herein refers to mammals, including but not limited to: humans; non-human primates; farm animals, such as cattle, horses, sheep, goats, and pigs; domestic animals, such as rabbits, dogs, and cats; laboratory animals, including rodents such as rats, mice, and guinea pigs; etc. In some embodiments, the subject or patient is a human.

The term "treating" as used herein refers to the administration of one or more drug substances to a patient or subject suffering from a disease or symptoms of the disease in order to cure, alleviate, relieve, ameliorate or affect the disease or symptoms of the disease. In the context of this application, unless specifically stated to the contrary, the term "treating" may also include prophylaxis.

The term "solvate" in this application refers to a complex formed by combining a compound of formula (I) or a pharmaceutically acceptable salt thereof with a solvent (e.g., ethanol or water). It should be understood that any solvate of a compound of formula I for use in the treatment of a disease or condition may provide different properties including pharmacokinetic properties, however will result in the compound of formula I upon absorption into a subject, such that the use of the compound of formula I encompasses the use of any solvate of the compound of formula I respectively.

The term "hydrate" refers to the situation where the solvent in the above term "solvate" is water.

It should be further understood that a compound of formula I, or a pharmaceutically acceptable salt thereof may be isolated in the form of a solvate, and therefore any such solvate is included within the scope of the present disclosure. For example, a compound of formula I, or a pharmaceutically acceptable salt thereof may exist in an unsolvated form as well as a solvated form with a pharmaceutically acceptable solvent such as water, ethanol, or the like.

The term "pharmaceutically acceptable salt" refers to a relatively non-toxic, inorganic or organic acid addition salt of a compound of the present disclosure. For example, see S.M. Berge et al. "Pharmaceutical Salts", J. Pharm. Sci. 1977, 66, 1-19. The inorganic acid is for example hydrochloric, hydrobromic, hydroiodic, sulfuric, phosphoric, or nitric acid; and the organic acid is for example formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, picric, pivalic, 2-hydroxyethanesulfonate, itaconic, sulfamic, trifluoromethanesulfonic, dodecyl sulfuric, ethansulfonic, benzenesulfonic, para-toluenesulfonic, methansulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, or sulfosalicylic acid. For example, a pharmaceutically acceptable salt may be formed by using a compound of formula I and HCl (or hydrochloric acid), HBr (or hydrobromic acid), methanesulfonic acid, sulfuric acid, tartaric acid or fumaric acid.

The nitrogen-containing compounds of formula (I) of the present disclosure may be converted to N-oxides by treatment with an oxidizing agent (e.g., m-chloroperbenzoic acid, hydrogen peroxide, ozone). Therefore, under the conditions allowed by the valence state and structure, the compounds claimed in this application include not only the nitrogen-containing compounds shown in the structural formulas, but also their N-oxide derivatives.

Certain compounds of the present disclosure may exist as one or more stereoisomers. Stereoisomers include geometric isomers, diastereomers and enantiomers. Accordingly, the compounds claimed in this disclosure also include racemic mixtures, single stereoisomers, and optically active mixtures. It will be understood by those skilled in the art that one stereoisomer may have better efficacy and/or lower side effects than other stereoisomers. Single stereoisomers and optically active mixtures can be obtained by methods such as chiral source synthesis, chiral catalysis, and chiral resolution. The racemate can be chirally resolved by chromatographic resolution or chemical resolution. For example, chiral tartaric acid, chiral malic acid or other chiral acid resolution reagents may be added to form a salt with the compound of the present disclosure, and the product may be separated by utilizing the different physical and chemical properties of the product, such as solubility.

The present disclosure also includes all suitable isotopic variations of the disclosed compounds. An isotopic variant is defined as a compound in which at least one atom is replaced by an atom of the same atomic number but with an atomic mass different from that commonly found or predominantly found in nature. Examples of isotopes that may be incorporated into compounds of the present disclosure include isotopes of hydrogen, carbon, nitrogen, and oxygen, such as ²H (deuterium), ³H (tritium), ¹¹C, ¹³C, ¹⁴C, ¹⁵N, ¹⁷O, and ¹⁸O, respectively.

The term "alkyl" is used in this disclosure to include branched and linear saturated aliphatic monovalent hydrocarbon groups having the specified number of carbon atoms. The term "alkylene" is used in this disclosure to include branched and linear saturated aliphatic divalent hydrocarbon groups having the specified number of carbon atoms. C_{n~m} refers to groups having n to m carbon atoms. For example, C_{2~8} alkylene includes C₂ alkylene, C₃ alkylene, C₄ alkylene, C₅ alkylene, C₆ alkylene,C₇ alkylene, and C₈ alkylene. C₅₋₆ cycloalkylene refers to cyclopentylene or cyclohexylene.

An alkyl (or, alkylene or cycloalkylene) group can be unsubstituted, or an alkyl (or, alkylene or cycloalkylene) group can be substituted wherein at least one hydrogen is replaced by another chemical group such as hydroxyl, halogen, etc.. For example, C₃ alkylene includes -(CH₂)₃-, -CH₂CH(OH)CH₂-, -CH(CH₃)CH₂- and the like. C₄ alkylene includes -(CH₂)₄-, -CH₂CH(CH₃)CH₂-, -CH(CH₃)CH₂CH₂- and the like.

A "therapeutically effective amount" is an amount of a therapeutic agent being administered which will improve a disease or condition in a patient. A "prophylactically effective amount" is an amount of a prophylactic agent being administered which will prevent a disease or condition in a subject. The "therapeutically effective amount" for a therapeutic agent or the "prophylactically effective amount" for a prophylactic agent may vary with the therapeutic/prophylactic agent, the disease state and its severity, the age and weight of a patient/subject to be treated/prevented, etc. The therapeutically effective amount and the prophylactically effective amount can be routinely determined by one of ordinary skill in the art based on his knowledge and this disclosure.

In this application, when the name of the compound is inconsistent with the structural formula, the structural formula shall prevail.

It should be understood that the term "compound of the present disclosure" used in the present application may include the compound of formula (I), N-oxide thereof, solvate thereof, pharmaceutically acceptable salt thereof, stereoisomer thereof, or mixture thereof according to the context.

The term "cationic lipid" as used herein refers to a lipid that is positively charged at a selected pH value. Cationic lipids are prone to bind to negatively charged nucleic acids, that is, to form lipid nanoparticles (LNPs) by interacting with negatively charged phosphate groups present in nucleic acids through electrostatic forces.

After screening a large number of compounds, the inventors found that it is very difficult to screen out a suitable cationic lipid compound that meet the following conditions: having both very high transfection efficiency and extremely low cytotoxicity, ultra-high and sustained expression in mice. The inventors have discovered that some compounds, such as YK-101, YK-107 and YK-108, can deliver nucleic acids with significantly improved intracellular transfection efficiencies, significantly reduced cytotoxicities, and significantly improved expressions and sustained expression in animals as compared with compounds in the prior art.

Herein, we selected SM-102 (compound 25 in WO2017049245A2) for comparison with the compounds we designed. SM -102 is now widely used for mRNA delivery.

The chemical structure of SM -102:

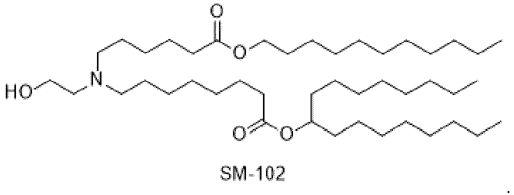

In addition, we also compared the compound of the present disclosure with ALC -0315 (compound 3 disclosed in CN108368028B), cationic lipid compound 21 and compound 23 disclosed in WO2021055833A1, and HHMA (compound 1 disclosed in CN112979483B).

The chemical structure of ALC-0315:

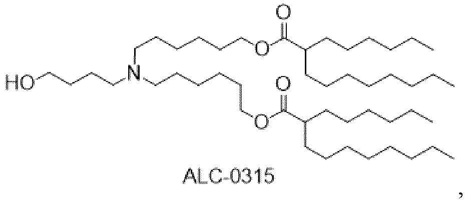

The chemical structure of compound 21:

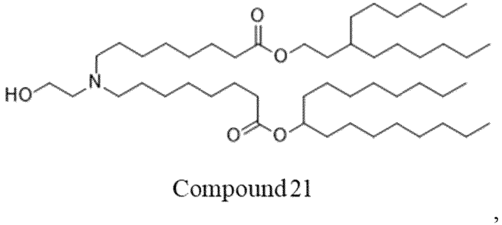

The chemical structure of compound 23:

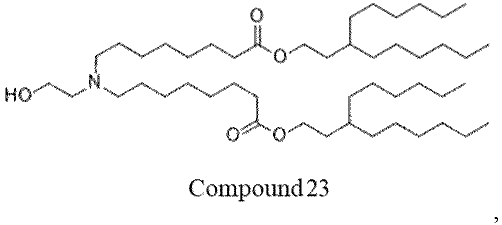

The chemical structure of HHMA:

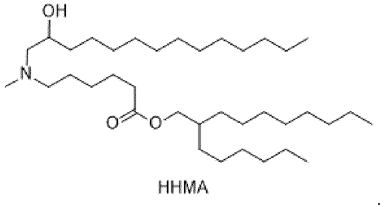

Among a series of designed compounds, LNP preparations prepared from YK-101, YK-107 and YK-108 have significantly improved cell transfection activities, significantly reduced cytotoxicities, and significantly improved expression amounts and durations of mRNA in mice, compared with similar or used cationic lipids in the prior art. For example, YK-101, YK-107 and YK-108 achieved cell transfection activities that can be up to 16 times that of SM-102 (compound 25 disclosed in WO2017049245A2), and 19 times that of compound 23 (compound 23 disclosed in WO2021055833A1); cell survival rates that are 39% higher than that of ALC-0315 (compound 3 disclosed in CN108368028B), and 22% higher than that of both SM-102 and HHMA (compound 1 disclosed in CN112979483B); expression amounts of mRNA in mice that can be up to 11 times that of SM-102, 12 times that of compound 21 (compound 21 disclosed in WO2021055833A1) and compound 23.

Compounds with small structural differences have very large differences in transfection efficiency and/or toxicity to cells and high intracellular expression. For example, for compounds YK-108 and YK-109 of the present application, the cell transfection efficiency of YK-108 can be up to 34 times that of YK-109, the cell survival rate of YK-108 is 20 % higher than that of YK-109, the expression of mRNA in mice of YK-108 can be up to 15 times that of YK-109; YK-101, YK-107 and YK-108 have cell transfection activities that are more than 10,000 times higher than that of YK-112, and cell survival rates that are 80 % higher than that of YK-112; The expression of the mRNA carried by compound YK-107 in animals is 62 times that of YK-126.

We found that the compounds of the present disclosure are significantly improved in terms of the transfection efficiency and/or toxicity to cells. For example, YK-101, YK-107 and YK-108 achieved cell transfection activities that can be up to 16 times that of SM-102, 19 times of compound 23; cell survival rates that are 39% higher than that of ALC-0315 (compound 3 disclosed in CN108368028B), and 22% higher than those of both SM-102 and HHMA (compound 1 disclosed in CN112979483B); expression amounts of mRNA in mice that can be up to 11 times that of SM-102 and 12 times those of compound 21 and compound 23.

We also found that there is no obvious corresponding relationship between the structure of cationic lipid compounds and intracellular transfection efficiency, toxicity to cells, and the high expression and sustained expression in animals of mRNA in an LNP formulation prepared from the cationic lipid compounds. It is likely that compounds with small structural differences have very large differences in transfection efficiency and/or toxicity to cells and high intracellular expression. For example, for the compounds YK-108 and YK-109 of the present application, the cell transfection efficiency of YK-108 is 34 times that of YK-109, the cell survival rate of YK-108 is 20 % higher than that of YK-109, and the expression of mRNA in mice of YK-108 can be up to 15 times that of YK-109; YK-101, YK-107 and YK-108 have cell transfection activities that are more than 10,000 times higher than that of YK-112, cell survival rates that are 80% higher than that of YK-112; the expression of mRNA carried by compound YK-107 in animals is 62 times that of YK-126. Therefore, it is very difficult to select suitable cationic lipid compounds that can simultaneously have high transfection efficiency and low toxicity to cells, as well as high and sustained expression of mRNA in mice.

Through unique design and extensive screening, the present disclosure discovered some compounds, such as YK-101, YK-107, YK-108, YK-103, YK-104, YK-105 and YK-106, can deliver nucleic acids with significantly improved cell transfection efficiencies, significantly reduced cytotoxicities, and significantly improved expression and sustained expression in animals, compared with other compounds in the prior art, achieving unexpected technical effects.
1. The cell transfection efficiency is significantly improved compared to the activity of the cationic lipids in the prior art and those designed by us
   1) Through various designs of compound structures and a lot of creative work, we screened out cationic lipid compounds with high cell transfection efficiency, such as YK-101, YK-107 and YK-108.

The LNP formulations prepared from YK-101, YK-107 and YK-108 have the best cell transfection activities, which are significantly improved compared with similar or used cationic lipids in the prior art. For example, the cell transfection activity of YK-108 can be up to 16 times that of SM-102 and 19 times that of compound 23.

YK-101, YK-107 and YK-108 have the highest cell transfection activities among the series of compounds we designed with the most similar structures, which can be up to 34 times that of other compounds, such as YK-109.

YK-101, YK-107 and YK-108 have the highest transfection activities compared to compounds with some minor differences in structure only in the G₁, G₃ or R₁ group. They can be up to 10,000 times higher than other compounds, such as YK-112.

YK-101, YK-107 and YK-108 have the highest transfection activities compared with compounds in which only an ether bond is introduced into the G₃ group or the G₃ group is changed to a 4-(dimethylamino)butyryl group. For example, they can be up to more than 300 times higher than that of YK-124.
2) There is no correspondence between the structure of a compound and intracellular transfection efficiency. Compounds with small structural differences are likely to have very large differences in transfection efficiency.
3) Changing one identical group to another identical group in different compounds may not necessarily have the same impact on activity, and may even be opposite, that is, changes in activity cannot be inferred based on changes in chemical structure. Therefore, screening out cationic lipid compounds with high transfection efficiency requires multiple designs and a lot of creative work.
2. Cytotoxicity is significantly reduced compared with the cationic lipids in the prior art and those designed by us
   1) We measured the cell survival rate of LNP formulations prepared from a series of designed compounds and screened out cationic lipid compounds with low cytotoxicity, such as YK-101, YK-107 and YK-108.

The LNP formulations prepared from YK-101, YK-107 and YK-108 have the lowest cytotoxicities and significantly reduced cytotoxicities compared with similar or used cationic lipids in the prior art. For example, the cell survival rates of the three are 39% higher than that of ALC-0315 (compound 3 disclosed in CN108368028B), and 22% higher than those of both SM-102 and HHMA (compound 1 disclosed in CN112979483B).

YK-101, YK-107 and YK-108 have the lowest cytotoxicities among the compounds we designed that have the most similar structures, that is, only individual groups, such as G₁, G₂ and R₁, differ by 1-2 C. The cell survival rates of the three compounds can be 45% higher than that of other compounds, such as YK-103.

YK-101, YK-107 and YK-108 have the lowest cytotoxicities compared to compounds with some minor differences in structure only in the G₁, G₃ or R₁ group. The cell survival rates of the three compounds can be 80% higher than that of other compounds, such as YK-112.

YK-101, YK-107 and YK-108 have the lowest cytotoxicities compared with compounds in which only an ether bond is introduced into the G3 group or the G3 group is changed to a 4-(dimethylamino)butyryl group. For example, the cell survival rates of the three can be 60% higher than YK-126.
2) In addition, there is no correspondence between the structure of a compound and its cytotoxicity. Even compounds with small structural differences will likely have very large differences in cytotoxicity.
3) Cytotoxicity cannot be predicted based on chemical structure. It is very difficult to screen out compounds with high transfection efficiency and low cytotoxicity, which requires a lot of creative work.
3. The expression amount and expression time in animals are significantly improved compared with those cationic lipids in the prior art and designed by us
   1) We conducted a testing of performance of *in vivo* delivery of carriers in animals on LNP formulations prepared from a series of designed compounds, and screened out cationic lipid compounds with high and sustained expression of mRNA in mice, such as YK-101, YK -107 and YK-108.

The LNP formulations prepared from YK-101, YK-107 and YK-108 have high and sustained expressions of mRNA in mice, which are significantly higher than similar or used cationic lipids in the prior art. For example, YK-108 can be up to 11 times that of SM-102, and 12 times that of compound 21 and compound 23.

YK-101, YK-107 and YK-108 have the highest mRNA expression amounts and durations of mRNA in mice among the compounds we designed that have the most similar structures, that is, only individual groups, such as G₁, G₂ and R₁, differ by 1-2 C. The expression amount of YK-108 can be 14 times higher than that of other compounds, such as YK-109.

YK-101, YK-107 and YK-108 have the highest *in vivo* mRNA expression amounts and durations of mRNA in mice compared to compounds with some minor differences in structure only in the G₁, G₃ or R₁ group. For example, the expression amount of YK-108 can be up to 15 times that of YK-114.

YK-101, YK-107 and YK-108 have the highest mRNA expression amounts and durations of mRNA in mice compared with compounds in which only an ether bond is introduced into the G3 group or the G3 group is changed to a 4-(dimethylamino)butyryl group. For example, the expression amount of YK-108 can be up to 67 times higher than that of YK-126.
2) There is no correspondence between the structure of cationic lipids and the high expression and sustained expression of mRNA in mice. Even if the structural differences of cationic lipid compounds are very small, the expression *in vivo* of mRNA in animals of the LNP formulations prepared from them will be likely to be very different.
3) It is impossible to predict whether mRNA will be highly and continuously expressed in animals based on the chemical structure of cationic lipids. Therefore, it is very difficult and requires a lot of creative work to screen out cationic lipid compounds with high and sustained expression of mRNA.

One aspect of the present disclosure provides novel cationic lipid compounds for the delivery of a therapeutic agent or a prophylactic agent. The cationic lipid compounds of the present disclosure can be used to deliver nucleic acid molecules, small molecule compounds, polypeptides or proteins. Compared with known cationic lipid compounds, the cationic lipid compounds of the present disclosure exhibit higher transfection efficiencies and less cytotoxicities, and thus improve delivery efficiencies and safeties.

The present disclosure provides a cationic lipid, which is a compound of formula (I) 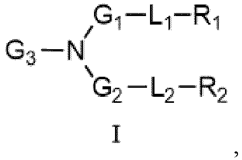 or an N-oxide, a solvate, a pharmaceutically acceptable salt or a stereoisomer thereof, wherein
G₁ is C_{1~6} alkylene, preferably unsubstituted C_{2~5} alkylene, more preferably unsubstituted C₃, C₅ or C₄ alkylene, still more preferably unsubstituted C₃, C₅ or C₄ linear alkylene;
G₂ is C_{2~8} alkylene, preferably unsubstituted C_{4~6} alkylene, more preferably unsubstituted C₅, C₆ or C₄ alkylene, still more preferably unsubstituted C₅, C₆ or C₄ linear alkylene;
G₃ is HO-R₇- or (CH₃)₂N(CH₂)₃C(O)O(CH₂)₂-, wherein R₇ is C_{2~8} alkylene, C₅₋₆ cycloalkylene, -CH₂CH(OH)CH₂-, -(CH₂CH₂O)ₘ-(CH₂)ₙ- or -(CH₂)ₘ-O-(CH₂)ₙ-, where m and n are each independently 1, 2, 3 or 4; R₇ is, e.g. : -(CH₂)₂-, -(CH₂)₃-, -CH₂CH(OH)CH₂-, -CH(CH₃)CH₂-, -CH(CH₃)(CH₂)₂-, -C(CH₃)₂(CH₂)₂-, -(CH₂)₂O(CH₂)₂O(CH₂)₂-, -(CH₂)₂O(CH₂)₂- or 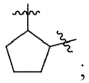
G₃ is preferably HO(CH₃)₂- or HO(CH₃)₃-;
L₁ is -C(O)O- or -OC(O)-;
L₂ is -C(O)O- or -OC(O)-;
R₁ is C_{6~15} linear alkyl or R₃-S-R₄ or R₅-S-S-R₆, wherein R₃ is C_{1~7} alkylene, preferably -CH₂CH₂-, R₄ is C_{1~7} alkyl, preferably C₄ linear alkyl, R₅ is C_{1~7} alkylene, preferably -CH₂CH₂CH₂-, R₆ is C_{1~7} alkyl, preferably C₅ linear alkyl;
R₁preferably unsubstituted C_{8~12} linear alkyl, more preferably unsubstituted C₁₀, C₁₁, C₉ or C₈ linear alkyl;
R₂ is C_{12~25} branched alkyl, preferably unsubstituted C_{14~22} branched alkyl, more preferably unsubstituted C₁₅, C₁₈ and C₁₄ branched alkyl.

In one embodiment, G₁ is unsubstituted C₃ alkylene, for example, -(CH₂)₃-.

In one embodiment, G₁ is unsubstituted C₅ alkylene, for example, -(CH₂)₅-.

In one embodiment, G₁ is unsubstituted C₄ alkylene, for example, -(CH₂)₄-.

In one embodiment, G₂ is unsubstituted C₅ alkylene, for example, -(CH₂)₅-.

In one embodiment, G₂ is unsubstituted C₆ alkylene, for example, -(CH₂)₆-.

In one embodiment, G₂ is unsubstituted C₄ alkylene, for example, -(CH₂)₄-.

In one embodiment, G₃ is HO(CH₂)₂-.

In one embodiment, G₃ is HO(CH₂)₃-.

In one embodiment, G₃ is HOCH₂CH(OH)CH₂-.

In one embodiment, G₃ is HOCH(CH₃)CH₂-.

In one embodiment, G₃ is HOCH(CH₃)(CH₂)₂-.

In one embodiment, G₃ is HOC(CH₃)₂(CH₂)₂-.

In one embodiment, G₃ is HO(CH₂)₂O(CH₂)₂O(CH₂)₂-.

In one embodiment, G₃ is HO(CH₂)₂O(CH₂)₂-.

In one embodiment, G₃ is

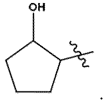

In one embodiment, L₁ is -C(O)O-.

In one embodiment, L₁ is -OC(O)-.

In one embodiment, L₂ is -C(O)O-.

In one embodiment, L₂ is -OC(O)-.

In one embodiment, R₁ is unsubstituted C_{8~12} linear alkyl, preferably unsubstituted C₁₀ linear alkyl, i.e., -(CH₂)₉CH₃.

In one embodiment, R₁ is unsubstituted C_{8~12} linear alkyl, preferably unsubstituted C₁₁ linear alkyl, i.e., -(CH₂)₁₀CH₃.

In one embodiment, R₂ is unsubstituted C_{14~22} branched alkyl, preferably unsubstituted C₁₄, C₁₅ or C₁₈ branched alkyl. e.g., R₂ is:

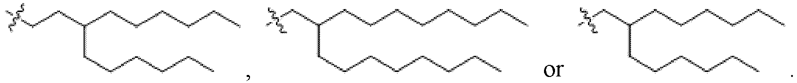

In one embodiment, G₁ is -(CH₂)₃-, G₂ is -(CH₂)₅-, G₃ is HO(CH₂)₂-, L₁ is -C(O)O-, L₂ is -C(O)O-, R₁ is -(CH₂)₉CH₃, R₂ is:

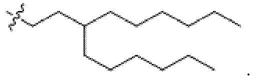

In one embodiment, G₁ is -(CH₂)₃-, G₂ is -(CH₂)₅-, G₃ is HO(CH₂)₃-, L₁ is -C(O)O-, L₂ is -C(O)O-, R₁ is -(CH₂)₉CH₃, R₂ is:

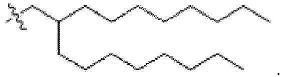

In one embodiment, G₁ is -(CH₂)₃-, G₂ is -(CH₂)₆-, G₃ is HO(CH₂)₂-, L₁ is -C(O)O-, L₂ is -C(O)O-, R₁ is -(CH₂)₁₀CH₃, R₂ is :

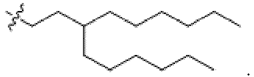

In an exemplary embodiment, the present disclosure provides a compound of formula (I) selected from the following, or an N-oxide, a solvate, a pharmaceutically acceptable salt or a stereoisomer thereof:

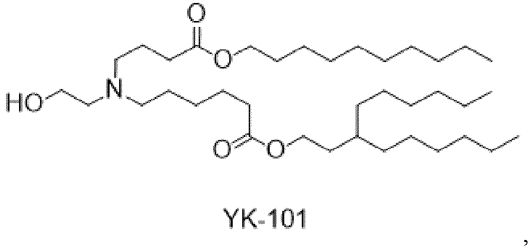

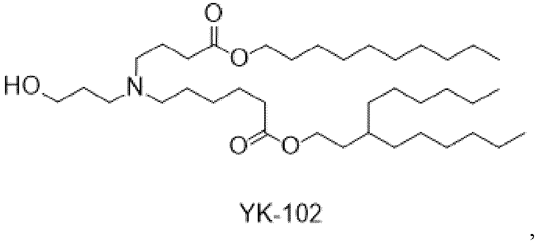

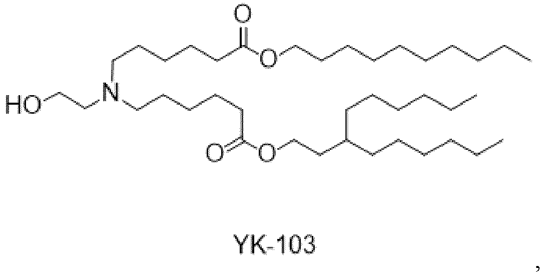

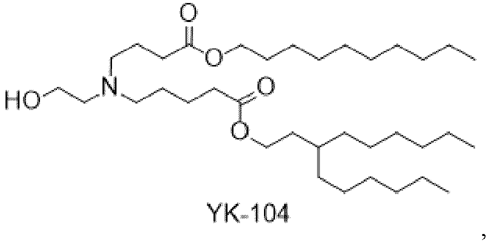

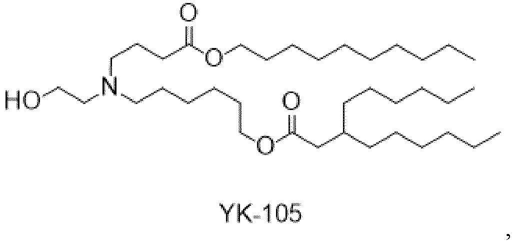

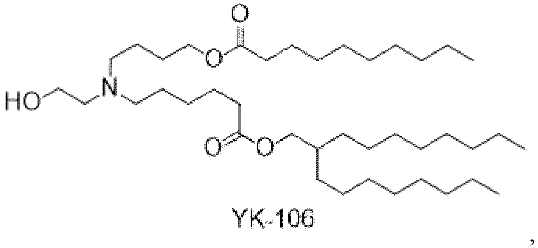

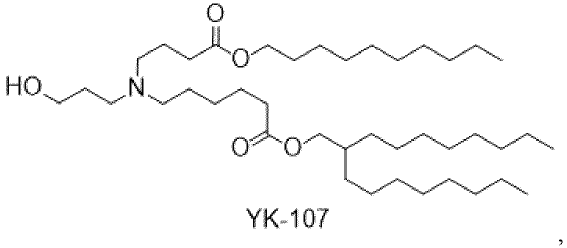

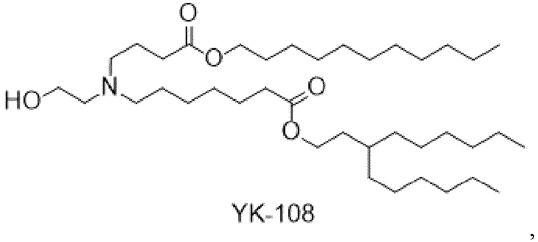

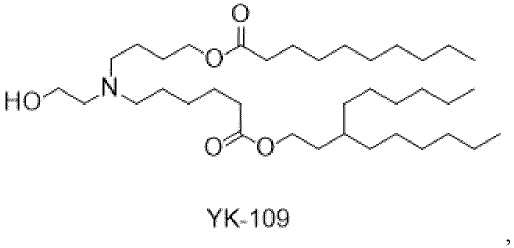

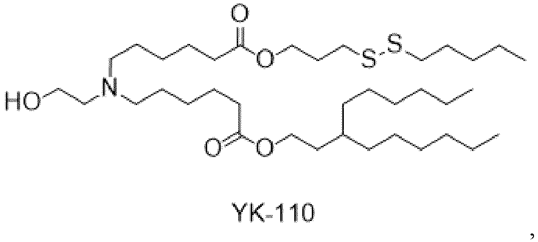

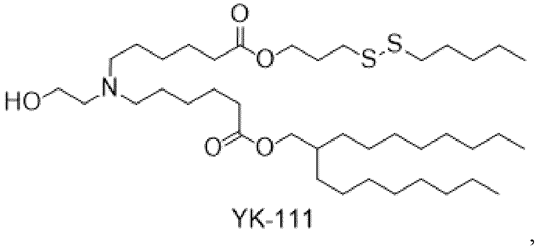

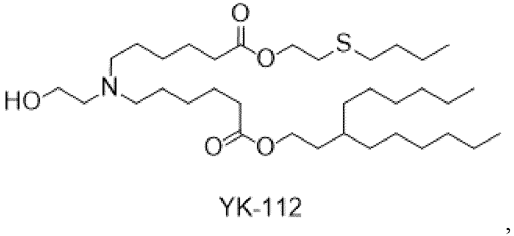

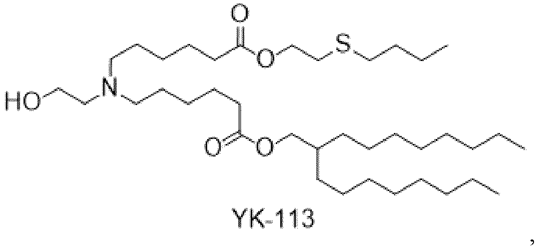

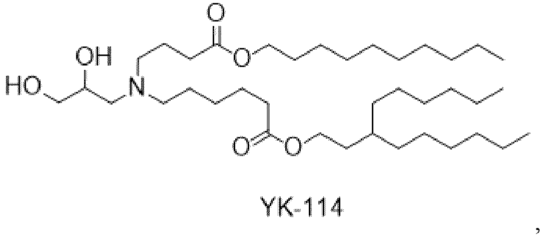

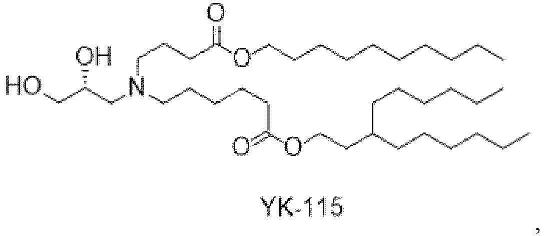

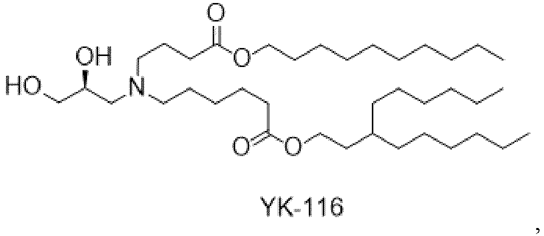

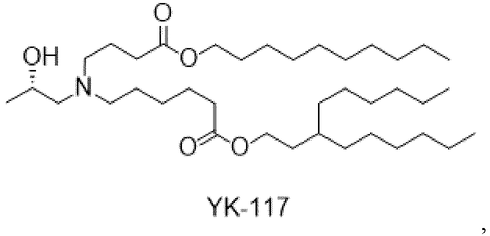

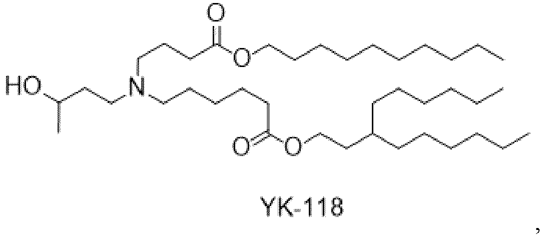

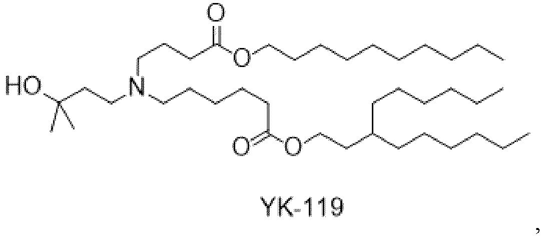

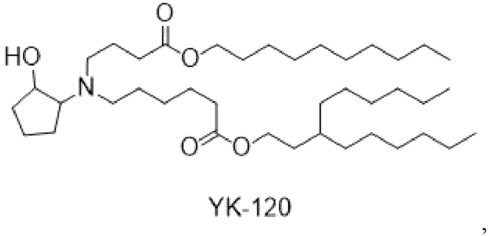

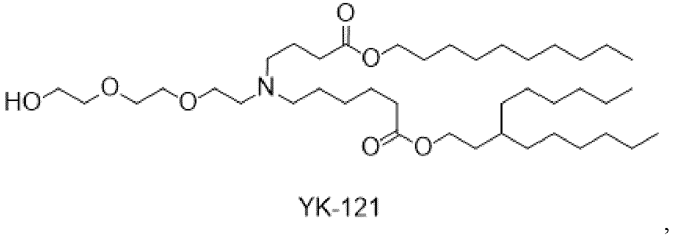

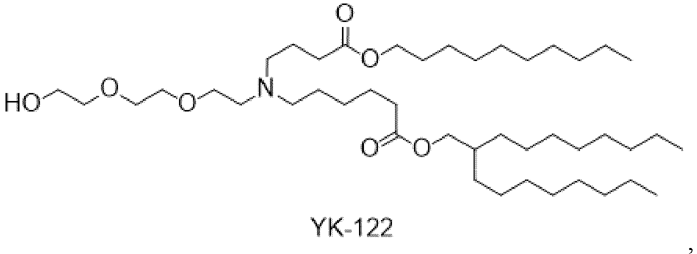

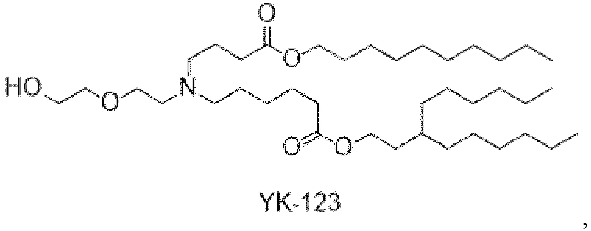

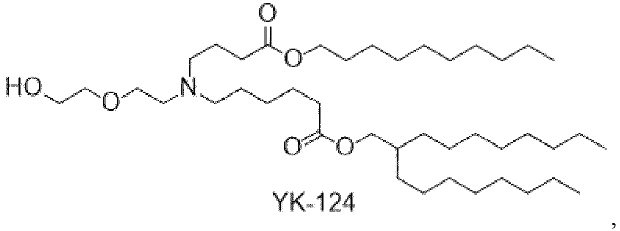

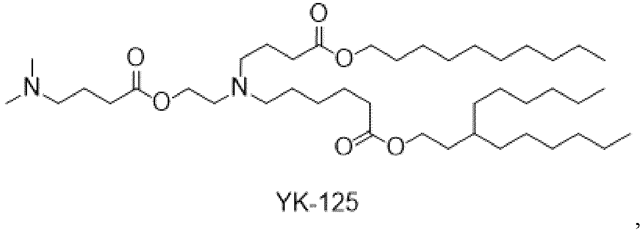

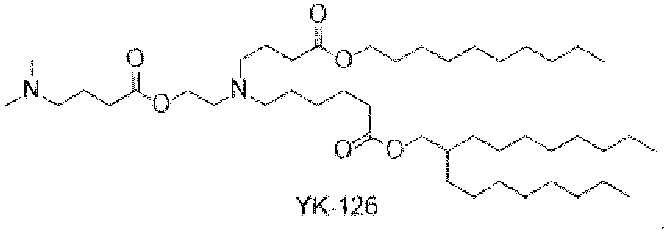

Another aspect of the present disclosure provides a composition comprising a carrier, wherein the carrier comprises a cationic lipid, and the cationic lipid comprises the above compound of formula (I) or an N-oxide, a solvate, a pharmaceutically acceptable salt or a stereoisomer thereof.

In one embodiment, the composition is a nanoparticle formulation, wherein the average size of the nanoparticle formulation is 10nm to 300nm, preferably 90nm to 260nm, more preferably 90nm to 200nm; and the polydispersity coefficient of the nanoparticle formulation is ≤50%, preferably ≤40%, more preferably ≤30%.

### Cationic lipid

In one embodiment of the composition/carrier of the present disclosure, the cationic lipid is one or more selected from the above compound of formula (I) or an N-oxide, a solvate, a pharmaceutically acceptable salt or a stereoisomer thereof. In one embodiment, the cationic lipid is selected from the compounds of formula (I) described above. For example, the cationic lipid is compound YK-101, YK-102, YK-103, YK-104, YK-105, YK-106, YK-107, YK-108, YK-109, YK-110, YK-111, YK-112, YK-113, YK-114, YK-115, YK-116, YK-117, YK-118, YK-119, YK-120, YK-121, YK-122, YK-123, YK-124, YK-125 or YK-126. In a preferred embodiment, the cationic lipid is compound YK-101. In another preferred embodiment, the cationic lipid is compound YK-107. In another preferred embodiment, the cationic lipid is compound YK-108.

In another embodiment of the composition/carrier of the present disclosure, the cationic lipid comprises: (*a*) one or more selected from the above compound of formula (I) or an N-oxide, a solvate, a pharmaceutically acceptable salt or a stereoisomer thereof; and (*b*) one or more other ionizable lipid compound(s) different from (*a*)*.* The cationic lipid compound (*b*) can be a commercially available cationic lipid, or a cationic lipid compound reported in literatures. For example, the cationic lipid compound (b) can be SM-102 (compound 25 in WO2017049245A2), and can also be ALC-0315 (compound 3 in CN108368028B), or compound 21 and compound 23 in WO2021055833, or HHMA (compound 1 in CN112979483B).

In one embodiment, the molar ratio of the cationic lipid to the carrier is about 25%~75%, such as about 30%, 40%, 49%, 55%, 60%, 65% or 70%.

The carrier can be used to deliver an active ingredient such as a therapeutic agent or a prophylactic agent. The active ingredient can be sealed within the carrier or bound to the carrier.

For example, the therapeutic agent or prophylactic agent comprises one or more of nucleic acid molecules, small molecule compounds, polypeptides or proteins. Such nucleic acids include, but are not limited to, single-chained DNA, double-chained DNA, and RNA. Suitable RNAs include, but are not limited to, small interfering RNA (siRNA), asymmetric interfering RNA (aiRNA), microRNA (miRNA), Dicer-substrate RNA (dsRNA), small hairpin RNA (shRNA), messenger RNA (mRNA) and a mixture thereof.

### Neutral lipid

The carrier may comprise a neutral lipid. Neutral lipid in this disclosure refers to an auxiliary lipid that is uncharged or exists in a zwitterionic form at a selected pH value. The neutral lipid may regulate the flow of nanoparticles into a lipid bilayer structure and improve efficiency by promoting lipid phase transition, while also possibly affecting target organ specificity.

In one embodiment, the molar ratio of the cationic lipid to the neutral lipid is about 1:1~15:1, for example about 14:1, 13:1, 12:1, 11:1, 10:1, 9:1, 8:1, 7:1, 6:1, 5:1, 4:1, 3:1 and 2:1. In a preferred embodiment, the molar ratio of the cationic lipid to the neutral lipid is about 4.9:1. In another preferred embodiment, the molar ratio of the cationic lipid to the neutral lipid is about 4:1.

For example, neutral lipids may comprise one or more of phosphatidylcholine, phosphatidylethanolamine, sphingomyelin, ceramide, sterol, and derivatives thereof.

The carrier component of a composition comprising the cationic lipid may comprise one or more neutral lipid-phospholipids, such as one or more (poly)unsaturated lipids. Phospholipids can be assembled into one or more lipid bilayers. In general, a phospholipid can comprise a phospholipid moiety and one or more fatty acid moieties.

The neutral lipid moiety may be selected from the non-limiting group consisting of phosphatidylcholine, phosphatidylethanolamine, phosphatidylglycerol, phosphatidylserine, phosphatidic acid, 2-lysophosphatidylcholine, and sphingomyelin. The fatty acid moiety may be selected from the non-limiting group consisting of lauric acid, myristic acid, myristoleic acid, palmitic acid, palmitoleic acid, stearic acid, oleic acid, linoleic acid, alpha-linolenic acid, erucic acid, phytanic acid, arachidic acid, arachidonic acid, eicosapentaenoic acid, behenic acid, docosapentaenoic acid, and docosahexaenoic acid. Also contemplated are non-natural species which include natural species with modifications and substitutions such as branching, oxidation, cyclization and alkynes. For example, a phospholipid can be functionalized with or cross-linked with one or more alkynes (e.g., an alkenyl group in which one or more double bonds are replaced by a triple bond). Under appropriate reaction conditions, alkynyl groups may undergo copper-catalyzed cycloaddition reactions when exposed to azides. These reactions can be used to functionalize the lipid bilayer of the composition to facilitate membrane penetration or cell recognition, or to couple the composition to useful components such as targeting or imaging moieties (e.g., dyes).

Neutral lipids useful in these compositions can be selected from the non-limiting group consisting of: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and a mixture thereof.

In some embodiments, the neutral lipid comprises DSPC. In certain embodiments, the neutral lipid comprises DOPE. In some embodiments, the neutral lipid comprises both DSPC and DOPE.

### Structured lipid

The carrier of the composition comprising the cationic lipid may also comprise one or more structured lipid(s). Structured lipids in this disclosure refer to lipids that enhance the stability of nanoparticles by filling the gaps between lipids.

In one embodiment, the molar ratio of the cationic lipid to the structured lipid is about 0.6:1~3:1, for example, about 0.6:1, 0.7:1, 0.8:1, 0.9:1, 1.0:1, 1.1:1, 1.2:1, 1.3:1, 1.4:1, 1.5:1, 1.6:1, 1.7:1, 1.8:1, 1.9:1, 2.0:1.

Structured lipids may be selected from, but are not limited to, the group consisting of cholesterol, nonsterol, sitosterol, ergosterol, campesterol, stigmasterol, brassicasterol, tomatine, tomatine, ursolic acid, alpha-tocopherol, corticosteroid and a mixture thereof. In some embodiments, the structured lipid is cholesterol. In some embodiments, the structured lipid comprises cholesterol, corticosteroid (e.g., prednisolone, dexamethasone, prednisone, and hydrocortisone) or a combination thereof.

### Polymer-conjugated lipid

The carrier of the composition comprising the cationic lipid may also comprise one or more polymer-conjugated lipid(s). Polymer-conjugated lipids mainly refer to lipids modified with polyethylene glycol (PEG). Hydrophilic PEG stabilizes LNPs, regulates nanoparticle size by limiting lipid fusion, and increases nanoparticle half-life by reducing nonspecific interactions with macrophages.

In one embodiment, the molar ratio of the polymer-conjugated lipid to the carrier is 0.5% to 10%, preferably 1.5%.

In one embodiment, the polymer-conjugated lipid is selected from one or more of PEG-modified phosphatidylethanolamine, PEG-modified phosphatidic acid, PEG-modified ceramide, PEG-modified dialkylamine, PEG-modified diacylglycerol, and PEG-modified dialkylglycerol. The molecular weight of PEG for the PEG modification is usually 350-5000Da.

For example, the polymer-conjugated lipid is selected from one or more of distearoyl phosphatidylethanolamine polyethylene glycol 2000 (DSPE-PEG2000), dimyristoylglycero-3-methoxy polyethylene glycol 2000 (DMG-PEG2000) and methoxypolyethylene glycol ditetradecylacetamide (ALC-0159).

In one embodiment of the composition/carrier of the present disclosure, the polymer-conjugated lipid is DMG-PEG2000.

In one embodiment of the composition/carrier of the present disclosure, the carrier comprises a neutral lipid, a structured lipid and a polymer-conjugated lipid, wherein the molar ratio of the cationic lipid, the neutral lipid, the structured lipid, and the polymer-conjugated lipids is (25~75): (5~25): (15~65): (0.5~10), for example (30~49):(7.5~15):(35~55):(1~5).

In one embodiment of the composition/carrier of the present disclosure, the carrier comprises a neutral lipid, a structured lipid and a polymer-conjugated lipid, wherein the molar ratio of the cationic lipid, the neutral lipid, the structured lipid, and the polymer-conjugated lipid is 40:10:48.5:1.5 or 49:10:39.5:1.5, preferably, 40:10:48.5:1.5.

### Therapeutic and/or prophylactic agent

The composition may comprise one or more therapeutic and/or prophylactic agent(s). In one embodiment, the mass ratio of the carrier to the therapeutic agent or prophylactic agent is 10:1 to 30:1, for example 12:1, 13:1, 14:1, 15:1, 16:1, 17:1, 18:1, 19:1,20:1,21:1,22:1,23:1,24:1,25:1.

In one embodiment, the mass ratio of the carrier to the therapeutic agent or prophylactic agent is 12.5:1~20:1, preferably 15:1.

The therapeutic agent or prophylactic agent includes, but is not limited to, one or more of a nucleic acid molecule, a small molecule compound, a polypeptide or a protein.

For example, the therapeutic agent or prophylactic agent is a vaccine or compound capable of eliciting an immune response.

The carriers of the present disclosure can deliver therapeutic and/or prophylactic agents to mammalian cells or organs, and thus the present disclosure also provides methods of treating diseases or conditions in mammals in need thereof, which comprises administering to the mammal a composition comprising a therapeutic agent and/or prophylactic agent and/or contacting mammalian cells with the composition.

Therapeutic and/or prophylactic agents include biologically active substances and are referred to alternatively as "active agents." A therapeutic and/or prophylactic agent may be a substance that, after delivery to a cell or organ, causes a desired change in the cell or organ or in other tissues or systems of the body. Such species can be used to treat one or more diseases, disorders or conditions. In some embodiments, the therapeutic and/or prophylactic agent is a small molecule drug useful in the treatment of a particular disease, disorder or condition. Examples of drugs that can be used in the composition include, but are not limited to, antineoplastic agents (e.g., vincristine, doxorubicin, mitoxantrone, camptothecin, cisplatin, bleomycin, cyclophosphamide, methotrexate, and streptozotocin), antitumor agents (e.g., actinomycin D, vincristine, vinblastine, cytosine arabinoside, anthracycline, alkylating agents, platinum compounds, antimetabolites, and nucleoside analogs such as methotrexate and purine and pyrimidine analogs), anti-infectives, local anesthetics (e.g., dibucaine and chlorpromazine), beta-adrenergic blockers (e.g., propranolol, timolol and labetalol), antihypertensives (e.g., clonidine and hydralazine), antidepressants (e.g., imipramine, amitriptyline, and doxepin), anticonvulsants (e.g., phenytoin), antihistamines (e.g., diphenhydramine, chlorpheniramine, and promethazine), antibiotics/antibacterials (e.g., gentamycin, ciprofloxacin, and cefoxitin), antifungals (e.g., miconazole, terconazole, econazole, isoconazole, butaconazole, clotrimazole, itraconazole, nystatin, naftifine, and amphotericin B), antiparasitic agents, hormones, hormone antagonists, immunomodulators, neurotransmitter antagonists, antiglaucoma drugs, vitamins, sedatives, and imaging agents.

In some embodiments, the therapeutic agent and/or prophylactic agent is a cytotoxin, a radioactive ion, a chemotherapeutic agent, a vaccine, a compound that elicits an immune response, and/or another therapeutic agent and/or prophylactic agent. A cytotoxin or cytotoxic agent includes any agent that is harmful to cells. Examples include, but are not limited to, taxol, cytochalasin B, gramicidin D, ethidium bromide, emetine, mitomycin, etoposide, teniposide, vincristine, vinblastine, colchicine, doxorubicin, daunorubicin, dihydroxy anthracin dione, mitoxantrone, mithramycin, actinomycin D, 1-dehydrotestosterone, glucocorticoid, procaine, tetracaine, lidocaine, propranolol, puromycin, maytansinoids such as maytansinol, rachelmycin (CC-1065), and their analogs or homologues. Radioactive ions include, but are not limited to, iodine (e.g., iodine 125 or iodine 131), strontium 89, phosphorus, palladium, cesium, iridium, phosphate, cobalt, yttrium 90, samarium 153, and praseodymium. Vaccines include compounds and formulations that confer immunity against one or more conditions associated with infectious diseases such as influenza, measles, human papillomavirus (HPV), rabies, meningitis, pertussis, tetanus, plague, hepatitis and tuberculosis, and may include mRNA encoding infectious disease-derived antigens and/or epitopes. Vaccines may also include compounds and formulations that lead to an immune response against cancer cells and may include mRNA encoding tumor cell-derived antigens, epitopes and/or neo-epitopes. Compounds that elicit an immune response may include vaccines, corticosteroids (e.g., dexamethasone), and other species. In some embodiments, vaccines and/or compounds capable of eliciting an immune response are administered by intramuscular administration of a composition comprising a compound according to formula (I), (IA), (IB), (II), (IIa), (IIb), (IIc), (IId), (IIe), (IIf), (IIg) or (III) (e.g., compound 3, 18, 20, 25, 26, 29, 30, 60, 108-112 or 122). Other therapeutic and/or prophylactic agents include, but are not limited to, antimetabolites (e.g., methotrexate, 6-mercaptopurine, 6-thioguanine, cytarabine, 5-fluorouracil, and dacarbazine), alkylating agents (e.g., mechlorethamine, thiotepa, chlorambucil, lazithromycin (CC-1065), melphalan, carmustine (BSNU), lomustine (CCNU), cyclophosphamide, busulfan, dibromomannitol, streptozotocin, mitomycin C, and cis-dichlorodiamino platinum (II) (DDP), cisplatin), anthracyclines (e.g., daunorubicin (formerly known as daunomycin) and doxorubicin), antibiotics (e.g., dactinomycin (formerly known as actinomycin), bleomycin, mithramycin, and anthramycin (AMC)) and antimitotic agents (e.g., vincristine, vinblastine, paclitaxel, and maytansinoid).

In other embodiments, the therapeutic and/or prophylactic agent is a protein. Therapeutic proteins that can be used in the nanoparticles of the present disclosure include, but are not limited to, gentamicin, amikacin, insulin, erythropoietin (EPO), granulocyte colony stimulating factor (G-CSF), granulocyte-macrophage colony-stimulating factor (GM-CSF), factor VIR, luteinizing hormone-releasing hormone (LHRH) analogs, interferon, heparin, hepatitis B surface antigen, typhoid vaccines and cholera vaccines.

In some embodiments, the therapeutic and/or prophylactic agent is a polynucleotide or nucleic acid (e.g., ribonucleic acid or deoxyribonucleic acid). The term "polynucleotide" in its broadest sense includes any compound and/or substance which is an oligonucleotide chain or can be incorporated into an oligonucleotide chain. Exemplary polynucleotides for use in accordance with the present disclosure include, but are not limited to, one or more of deoxyribonucleic acid (DNA); ribonucleic acid (RNA), including messenger mRNA (mRNA), hybrids thereof; RNAi-inducing factors; RNAi factors; siRNA; shRNA; miRNA; antisense RNA; ribozyme; catalytic DNA; RNA that induces triple helix formation; aptamer, etc. In some embodiments, the therapeutic and/or prophylactic agent is RNA. RNAs useful in the compositions and methods described herein can be selected from but not limited to the group consisting of shortmer, antagomir, antisense RNA, ribozymes, small interfering RNA (siRNA), asymmetric interfering RNA (aiRNA), microRNA (miRNA), Dicer-substrate RNA (dsRNA), small hairpin RNA (shRNA), transfer RNA (tRNA), messenger RNA (mRNA) and a mixture thereof. In certain embodiments, the RNA is mRNA.

In certain embodiments, the therapeutic agent and/or prophylactic agent is mRNA. The mRNA can encode any polypeptide of interest, including any naturally or non-naturally occurring or otherwise modified polypeptide. The polypeptide encoded by the mRNA can be of any size and can have any secondary structure or activity. In some embodiments, the polypeptide encoded by an mRNA may have a therapeutic effect when expressed in a cell.

In other embodiments, the therapeutic agent and/or prophylactic agent is siRNA. The siRNA can selectively reduce the expression of a gene of interest or downregulate the expression of that gene. For example, the siRNA can be selected such that a gene associated with a particular disease, disorder or condition is silenced upon administration of a composition comprising the siRNA to a subject in need thereof. siRNA can comprise a sequence that is complementary to an mRNA sequence encoding a gene or protein of interest. In some embodiments, the siRNA can be an immunomodulatory siRNA.

In certain embodiments, the therapeutic agent and/or prophylactic agent is sgRNA and/or cas9 mRNA. sgRNA and/or cas9 mRNA can be used as gene editing tools. For example, sgRNA-cas9 complexes can affect mRNA translation of cellular genes.

In some embodiments, the therapeutic agent and/or prophylactic agent is shRNA or a vector or plasmid encoding shRNA. shRNA can be produced inside a target cell after an appropriate construct is delivered into the nucleus. The constructs and mechanisms associated with shRNA are well known in the related art.

### Disease or condition

The composition/carrier of the present disclosure can deliver a therapeutic agent or a prophylactic agent to a subject or patient. The therapeutic agent or prophylactic agent includes, but is not limited to, one or more of nucleic acid molecules, small molecular compounds, polypeptides or proteins. Therefore, the composition of the present disclosure can be used to prepare nucleic acid medicine, gene vaccine, small molecule medicine, polypeptide or protein medicine. Due to the wide variety of therapeutic or prophylactic agents described above, the composition of the present disclosure can be used to treat or prevent a variety of diseases or conditions.

In one embodiment, the disease or condition is characterized by dysfunctional or abnormal protein or polypeptide activity.

For example, the disease or condition is selected from the group consisting of infectious diseases, cancer and proliferative diseases, genetic diseases, autoimmune diseases, diabetes, neurodegenerative diseases, cardiovascular and renovascular diseases, and metabolic diseases.

In one embodiment, the infectious disease is selected from diseases caused by coronavirus, influenza virus, or HIV virus, infantile pneumonia, Rift Valley fever, yellow fever, rabies, and various herpes.

### Other components

The composition may comprise one or more component(s) other than those described in the preceding sections. For example, the composition may comprise one or more small hydrophobic molecules, such as vitamins (e.g., vitamin A or vitamin E) or sterols.

The composition may also comprise one or more permeability enhancing molecules, carbohydrates, polymers, surface-altering agents or other components. The permeability enhancing molecule can be, for example, molecules described in US Patent Application Publication No. 2005/0222064. The carbohydrates can include simple sugars such as glucose and polysaccharides such as glycogen and derivatives and analogs thereof.

Surface-altering agents may include, but are not limited to, anionic proteins such as bovine serum albumin, surfactants for example cationic surfactants such as dimethyldioctadecylammonium bromide, sugars or sugar derivatives (e.g., cyclodextrins), nucleic acids, polymers (e.g., heparin, polyethylene glycol, and poloxamers), mucolytics (e.g., acetylcysteine, mugwort, bromelain, papain, clerodendrum, bromhexine, carbocisteine, eprazinone, mesna, ambroxol, sobrerol, domiodol, letosteine, stepronin, tiopronin, gelsolin, thymosin β4, dornase alfa, neltenexine and erdosteine) and DNases (e.g., rhDNase). Surface-altering agents can be disposed within and/or on the surface of the nanoparticles of the composition (e.g., by coating, adsorption, covalent attachment, or other methods).

The composition may also comprise one or more functionalized lipids. For example, lipids can be functionalized with alkyne groups that may undergo cycloaddition reactions when exposed to azide under appropriate reaction conditions. Specifically, lipid bilayers can be functionalized in this way with one or more groups that effectively facilitate membrane penetration, cell recognition, or imaging. The surface of the composition may also be coupled to one or more useful antibodies. Functional groups and conjugates useful for targeted cell delivery, imaging and membrane penetration are well known in the art.

In addition to these components, the composition may comprise any material that can be used in pharmaceutical compositions. For example, a composition may include one or more pharmaceutically acceptable excipients or auxiliary ingredients, such as, but not limited to, one or more of solvents, dispersion media, diluents, dispersion aids, suspension aids, granulation aids, disintegrants, fillers, glidants, liquid vehicles, binders, surfactants, isotonic agents, thickeners, emulsifiers, buffering agents, lubricants, oils, preservatives, flavoring agents, colorants, etc. Excipients are, for example, starch, lactose or dextrin. Pharmaceutically acceptable excipients are well known in the art (see for example Remington's The Science and Practice of Pharmacy, 21st Edition, A. R. Gennaro; Lippincott, Williams & Wilkins, Baltimore, MD, 2006).

Examples of diluents may comprise, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dry starch, corn starch, powdered sugar and/or a combination thereof.

In some embodiments, compositions comprising one or more lipids described herein may also comprise one or more adjuvants, such as glucopyranosyl lipid adjuvant (GLA), CpG oligodeoxyribonucleotide (e.g., class A or class B), poly(I:C), aluminum hydroxide, and Pam3CSK4.

The compositions of the present disclosure can be formulated into a preparation in solid, semi-solid, liquid or gaseous form, such as tablets, capsules, ointments, elixirs, syrups, solutions, emulsions, suspensions, injections, and aerosols. The compositions of the present disclosure can be prepared by methods well known in the art of pharmacy. For example, sterile injectable solutions can be prepared by incorporating into an appropriate solvent such as sterile distilled water the therapeutic agent or prophylactic agent in the required amount with various other ingredients enumerated above as required and then filtered sterilization. Surfactants may also be added to promote the formation of a uniform solution or suspension.

For example, the composition of the present disclosure may be administered intravenously, intramuscularly, intradermally, subcutaneously, intranasally, or by inhalation. In one embodiment, the composition is administered subcutaneously.

The compositions of the present disclosure are administered in therapeutically effective amounts which may vary not only with the particular agent chosen, but also with the route of administration, the nature of the disease being treated, and the age and condition of the patient, and may ultimately be at the discretion of the attending physician or clinician. For example, a dose of about 0.001 mg/kg to about 10 mg/kg of the therapeutic agent or prophylactic agent can be administered to a mammal (e.g., a human).

The present disclosure includes, but is not limited to, the following embodiments:
1. A compound of formula (I) 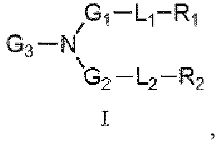 or an N-oxide, a solvate, a pharmaceutically acceptable salt or a stereoisomer thereof, wherein
   G₁ is C_{1~6} alkylene;
   G₂ is C_{2~8} alkylene;
   L₁ is -C(O)O- or -OC(O)-;
   L₂ is -C(O)O- or -OC(O)-;
   R₁ is C_{6~15} linear alkyl or R₃-S-R₄ or R₅-S-S-R₆;
   R₂ is C_{12~25} branched alkyl;
   G₃ is: HO-R₇- or (CH₃)₂N(CH₂)₃C(O)O(CH₂)₂-;
   wherein R₃ is C_{1~7} alkylene; R₄ is C_{1~7} alkyl;
   R₅ is C_{1~7} alkylene; R₆ is C_{1~7} alkyl;
   R₇ is C_{2~8} alkylene, C₅₋₆ cycloalkylene, -CH₂CH(OH)CH₂-, -(CH₂CH₂O)ₘ-(CH₂)ₙ- or -(CH₂)ₘ-O-(CH₂)ₙ-, where m and n are each independently 1, 2, 3 or 4.
2. The compound of formula (I) or an N-oxide, a solvate, a pharmaceutically acceptable salt or a stereoisomer thereof according to embodiment 1, wherein G₁ is unsubstituted C_{2~5} alkylene.
3. The compound of formula (I) or an N-oxide, a solvate, a pharmaceutically acceptable salt or a stereoisomer thereof according to embodiment 2, wherein G₁ is unsubstituted C₃ alkylene.
4. The compound of formula (I) or an N-oxide, a solvate, a pharmaceutically acceptable salt or a stereoisomer thereof according to embodiment 2, wherein G₁ is unsubstituted C₅ alkylene.
5. The compound of formula (I) or an N-oxide, a solvate, a pharmaceutically acceptable salt or a stereoisomer thereof according to embodiment 2, wherein G₁ is unsubstituted C₄ alkylene.
6. The compound of formula (I) or an N-oxide, a solvate, a pharmaceutically acceptable salt or a stereoisomer thereof according to any one of the preceding embodiments, wherein G₂ is unsubstituted C_{4~6} alkylene.
7. The compound of formula (I) or an N-oxide, a solvate, a pharmaceutically acceptable salt or a stereoisomer thereof according to embodiment 6, wherein G₂ is unsubstituted C₅ alkylene.
8. The compound of formula (I) or an N-oxide, a solvate, a pharmaceutically acceptable salt or a stereoisomer thereof according to embodiment 6, wherein G₂ is unsubstituted C₆ alkylene.
9. The compound of formula (I) or an N-oxide, a solvate, a pharmaceutically acceptable salt or a stereoisomer thereof according to embodiment 6, wherein G₂ is unsubstituted C₄ alkylene.
10. The compound of formula (I) or an N-oxide, a solvate, a pharmaceutically acceptable salt or a stereoisomer thereof according to any one of embodiments 1-9, wherein L₁ is -C(O)O-.
11. The compound of formula (I) or an N-oxide, a solvate, a pharmaceutically acceptable salt or a stereoisomer thereof according to any one of embodiments 1-9, wherein L₁ is -OC(O)-.
12. The compound of formula (I) or an N-oxide, a solvate, a pharmaceutically acceptable salt or a stereoisomer thereof according to any one of embodiments 1-11, wherein L₂ is -C(O)O-.
13. The compound of formula (I) or an N-oxide, a solvate, a pharmaceutically acceptable salt or a stereoisomer thereof according to any one of embodiments 1-11, wherein L₂ is -OC(O)-.
14. The compound of formula (I) or an N-oxide, a solvate, a pharmaceutically acceptable salt or a stereoisomer thereof according to any one of embodiments 1-13, wherein R₁ is unsubstituted C_{8~12} linear alkyl.
15. The compound of formula (I) or an N-oxide, a solvate, a pharmaceutically acceptable salt or a stereoisomer thereof according to embodiment 14, wherein R₁ is unsubstituted C₁₀ linear alkyl.
16. The compound of formula (I) or an N-oxide, a solvate, a pharmaceutically acceptable salt or a stereoisomer thereof according to embodiment 14, wherein R₁ is unsubstituted C₁₁ linear alkyl.
17. The compound of formula (I) or an N-oxide, a solvate, a pharmaceutically acceptable salt or a stereoisomer thereof according to embodiment 14, wherein R₁ is unsubstituted C₉ linear alkyl.
18. The compound of formula (I) or an N-oxide, a solvate, a pharmaceutically acceptable salt or a stereoisomer thereof according to embodiment 14, wherein R₁ is unsubstituted C₈ linear alkyl.
19. The compound of formula (I) or an N-oxide, a solvate, a pharmaceutically acceptable salt or a stereoisomer thereof according to any one of embodiments 1-13, wherein R₃ is -CH₂CH₂-.
20. The compound of formula (I) or an N-oxide, a solvate, a pharmaceutically acceptable salt or a stereoisomer thereof according to embodiment 19, wherein R₄ is C₄ linear alkyl.
21. The compound of formula (I) or an N-oxide, a solvate, a pharmaceutically acceptable salt or a stereoisomer thereof according to any one of embodiments 1-13, wherein R₅ is -CH₂CH₂CH₂-.
22. The compound of formula (I) or an N-oxide, a solvate, a pharmaceutically acceptable salt or a stereoisomer thereof according to embodiment 21, wherein R₆ is C₅ linear alkyl.
23. The compound of formula (I) or an N-oxide, a solvate, a pharmaceutically acceptable salt or a stereoisomer thereof according to any one of the preceding embodiments, wherein R₂ is unsubstituted C_{14~22} branched alkyl.
24. The compound of formula (I) or an N-oxide, a solvate, a pharmaceutically acceptable salt or a stereoisomer thereof according to embodiment 23, wherein R₂ is unsubstituted C₁₅ branched alkyl.
25. The compound of formula (I) or an N-oxide, a solvate, a pharmaceutically acceptable salt or a stereoisomer thereof according to embodiment 23, wherein R₂ is unsubstituted C₁₈ branched alkyl.
26. The compound of formula (I) or an N-oxide, a solvate, a pharmaceutically acceptable salt or a stereoisomer thereof according to embodiment 23, wherein R₂ is unsubstituted C₁₄ branched alkyl.
27. The compound of formula (I) or an N-oxide, a solvate, a pharmaceutically acceptable salt or a stereoisomer thereof according to embodiment 23, wherein R₂ is: 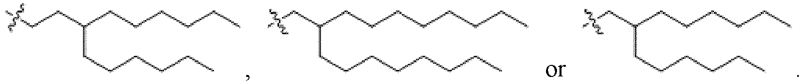
28. The compound of formula (I) or an N-oxide, a solvate, a pharmaceutically acceptable salt or a stereoisomer thereof according to any one of the preceding embodiments, wherein R₇ is: -(CH₂)₂-, -(CH₂)₃-, -CH₂CH(OH)CH₂-, -CH(CH₃)CH₂-, -CH(CH₃)(CH₂)₂-, -C(CH₃)₂(CH₂)₂-,
   -(CH₂)₂O(CH₂)₂O(CH₂)₂-, -(CH₂)₂O(CH₂)₂- or 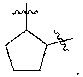
29. The compound of formula (I) or an N-oxide, a solvate, a pharmaceutically acceptable salt or a stereoisomer thereof according to embodiment 28, wherein R₇ is: -(CH₂)₂-.
30. The compound of formula (I) or an N-oxide, a solvate, a pharmaceutically acceptable salt or a stereoisomer thereof according to embodiment 28, wherein R₇ is: -(CH₂)₃-.
31. The compound of formula (I) or an N-oxide, a solvate, a pharmaceutically acceptable salt or a stereoisomer thereof according to embodiment 1, wherein the compound of formula (I) has one of the following structures: 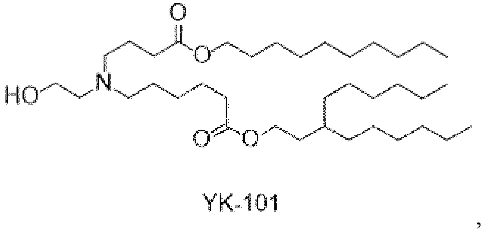 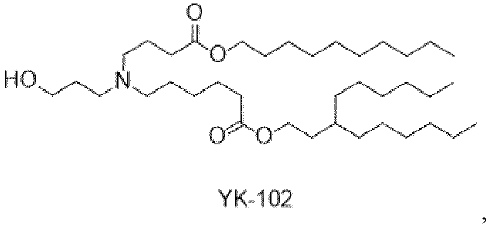 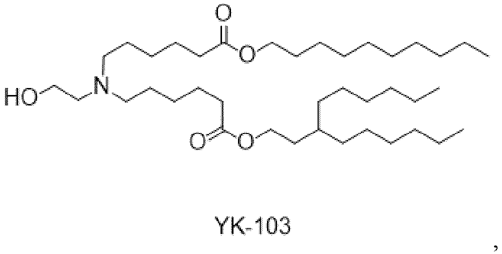 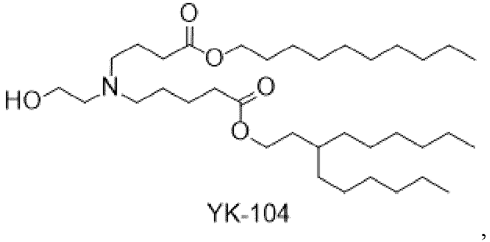 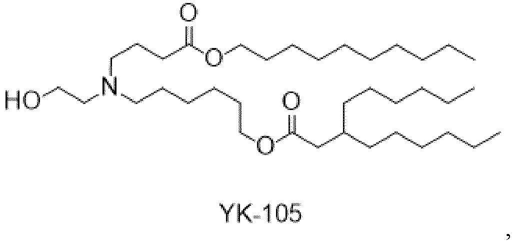 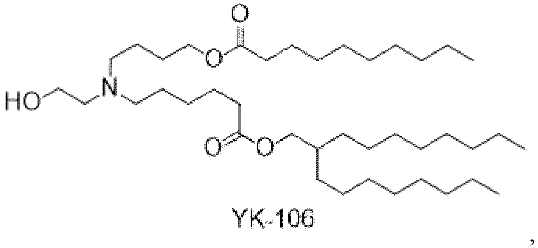 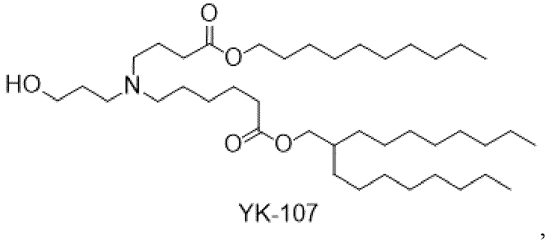 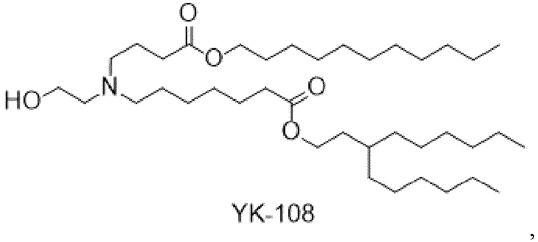 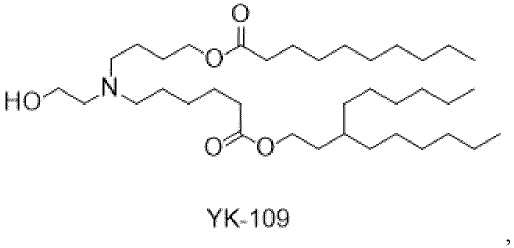 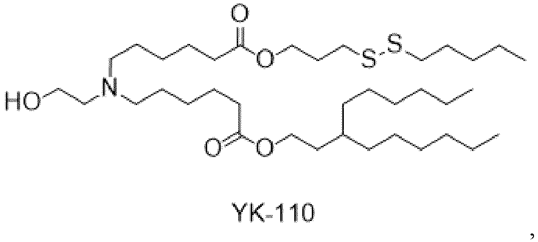 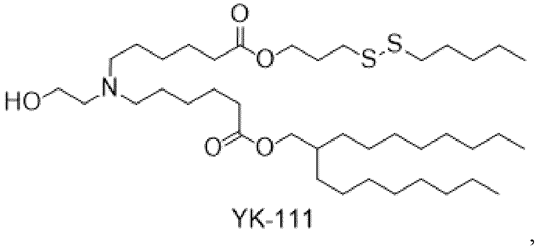 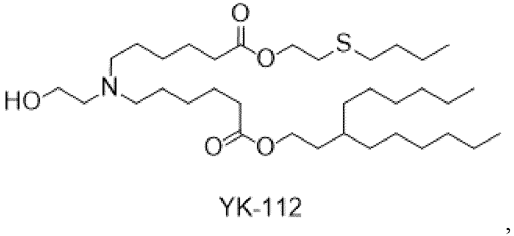 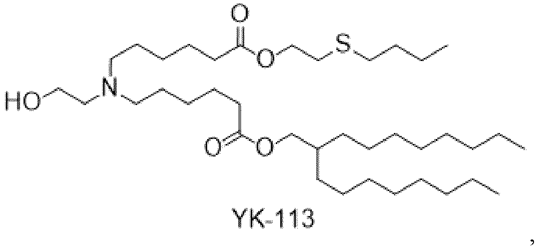 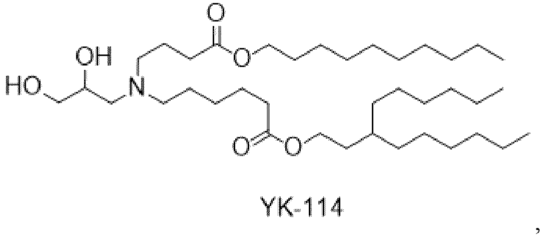 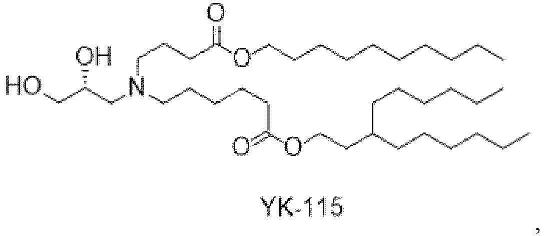 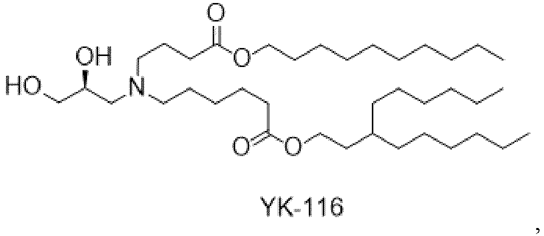 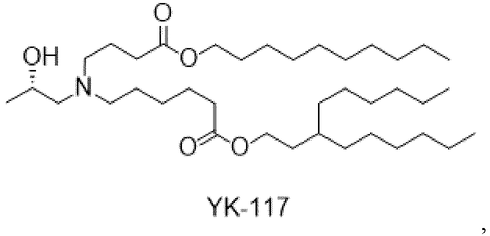 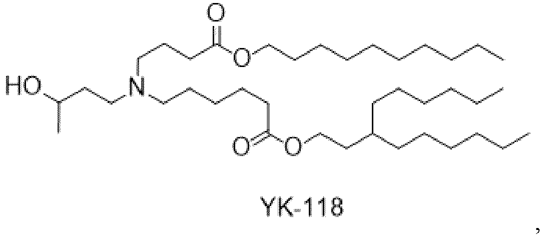 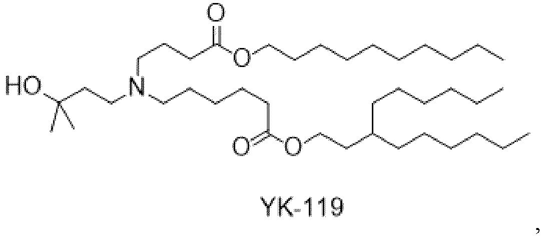 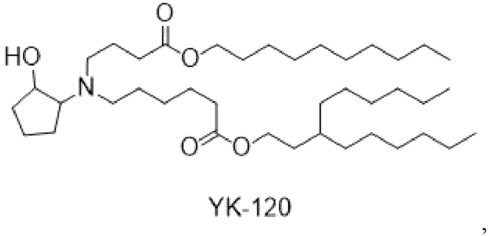 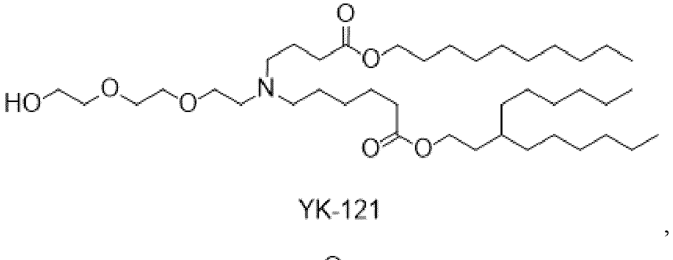 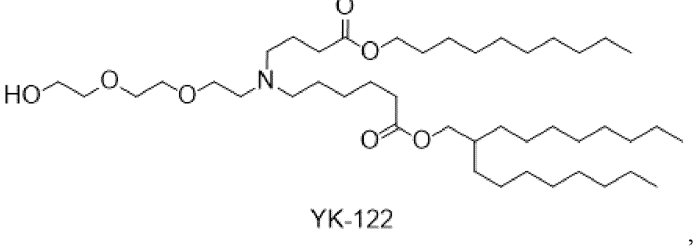 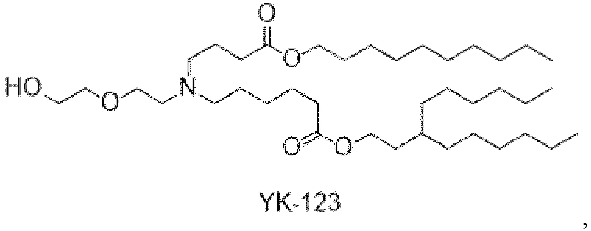 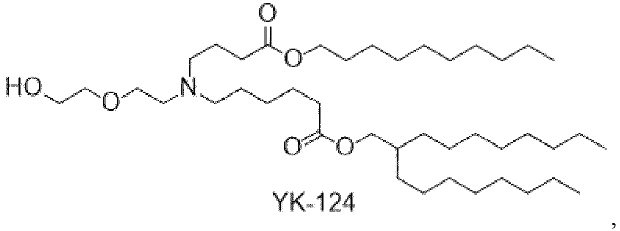 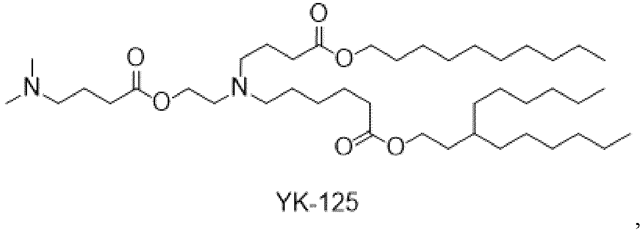 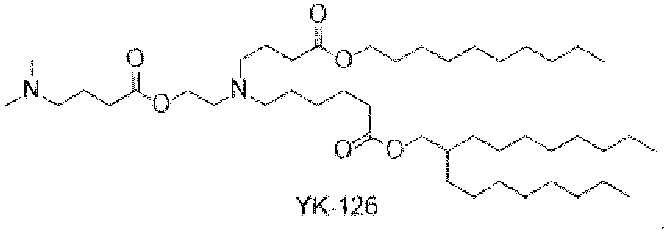
32. The compound of formula (I) or an N-oxide, a solvate, a pharmaceutically acceptable salt or a stereoisomer thereof according to embodiment 31, wherein the compound of formula (I) is compound YK-101 with the following structure: 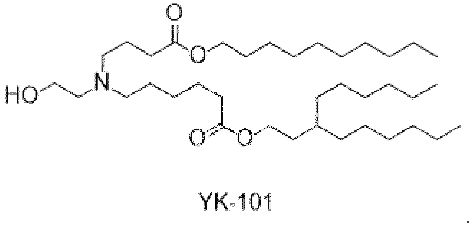
33. The compound of formula (I) or an N-oxide, a solvate, a pharmaceutically acceptable salt or a stereoisomer thereof according to embodiment 31, wherein the compound of formula (I) is compound YK-107 with the following structure: 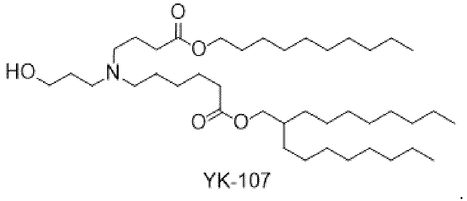
34. The compound of formula (I) or an N-oxide, a solvate, a pharmaceutically acceptable salt or a stereoisomer thereof according to embodiment 31, wherein the compound of formula (I) is compound YK-108 with the following structure: 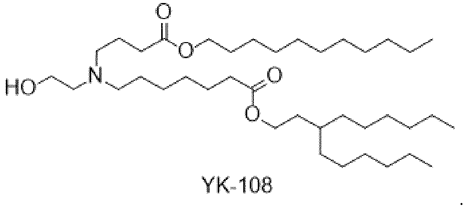
35. A composition comprising a carrier, wherein the carrier comprises a cationic lipid, and the cationic lipid comprises the compound of formula (I) or an N-oxide, a solvate, a pharmaceutically acceptable salt or a stereoisomer thereof according to any one of the preceding embodiments.
36. The composition according to embodiment 35, wherein the molar ratio of the cationic lipid to the carrier is 25% to 75%.
37. The composition according to any one of embodiments 35-36, wherein the carrier further comprises a neutral lipid.
38. The composition according to embodiment 37, wherein the molar ratio of the cationic lipid to the neutral lipid is 1:1 to 15:1, preferably 4:1.
39. The composition according to any one of embodiments 37-38, wherein the neutral lipid comprises one or more of phosphatidylcholine, phosphatidylethanolamine, sphingomyelin, ceramide, sterol and a derivative thereof.
40. The composition according to any one of embodiments 37-39, wherein the neutral lipid is selected from one or more of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and a mixture thereof.
41. The composition according to embodiment 40, wherein the neutral lipid is DOPE and/or DSPC.
42. The composition according to any one of embodiments 35-41, wherein the carrier further comprises a structured lipid.
43. The composition according to embodiment 42, wherein the molar ratio of the cationic lipid to the structured lipid is 0.6:1 to 3:1.
44. The composition according to any one of embodiments 42-43, wherein the structured lipid is selected from one or more of cholesterol, nonsterol, sitosterol, ergosterol, campesterol, stigmasterol, brassicasterol, tomatine, tomatine, ursolic acid, alpha-tocopherol, and corticosteroid.
45. The composition according to any one of embodiments 42-44, wherein the structured lipid is cholesterol.
46. The composition according to any one of embodiments 35-45, wherein the carrier further comprises a polymer-conjugated lipid.
47. The composition according to embodiment 46, wherein the molar ratio of the polymer-conjugated lipid to the carrier is 0.5% to 10%, preferably 1.5%.
48. The composition according to any one of embodiments 46-47, wherein the polymer-conjugated lipid is selected from one or more of PEG-modified phosphatidylethanolamine, PEG-modified phosphatidic acid, PEG-modified ceramide, PEG-modified dialkylamine, PEG-modified diacylglycerol, and PEG-modified dialkylglycerol.
49. The composition according to any one of embodiments 46-48, wherein the polymer-conjugated lipid is selected from one or more of distearoyl phosphatidylethanolamine polyethylene glycol 2000 (DSPE-PEG2000), dimyristoylglycero-3-methoxy polyethylene glycol 2000 (DMG-PEG2000) and methoxypolyethylene glycol ditetradecylacetamide (ALC-0159).
50. The composition according to any one of embodiments 35-49, wherein the carrier comprises a neutral lipid, a structured lipid and a polymer-conjugated lipid, and the molar ratio of the cationic lipid, the neutral lipid, the structured lipid and the polymer-conjugated lipid is (25 to 75):(5 to 25):(15 to 65):(0.5 to 10).
51. The composition according to embodiment 50, wherein the molar ratio of the cationic lipid, the neutral lipid, the structured lipid, and the polymer-conjugated lipid is (35 to 49):(7.5 to 15):(35 to 55):(1 to 5).
52. The composition according to any one of embodiments 50-51, wherein the molar ratio of the cationic lipid, the neutral lipid, the structured lipid, and the polymer-conjugated lipid is 40:10:48.5:1.5.
53. The composition according to any one of embodiments 35-52, wherein the composition is a nanoparticle formulation which has an average particle size of 10 nm to 300 nm and a polydispersity coefficient less than or equal to 50%.
54. The composition according to embodiment 53, wherein the nanoparticle formulation has an average particle size of 90 nm to 260 nm and a polydispersity coefficient less than or equal to 40 %.
55. The composition according to any one of embodiments 35-54, wherein the cationic lipid further comprises one or more other ionizable lipid compound(s).
56. The composition according to any one of embodiments 35-55, further comprising a therapeutic agent or a prophylactic agent.
57. The composition according to embodiment 56, wherein the mass ratio of the carrier to the therapeutic agent or prophylactic agent is 10:1 to 30:1.
58. The composition according to embodiment 57, wherein the mass ratio of the carrier to the therapeutic agent or prophylactic agent is 12.5:1 to 20:1.
59. The composition according to embodiment 58, wherein the mass ratio of the carrier to the therapeutic agent or prophylactic agent is 15:1.
60. The composition according to any one of embodiments 56-59, wherein the therapeutic agent or prophylactic agent comprises one or more of a nucleic acid molecule, a small molecule compound, a polypeptide or a protein.
61. The composition according to any one of embodiments 56-60, wherein the therapeutic agent or prophylactic agent is a vaccine or compound capable of eliciting an immune response.
62. The composition according to any one of embodiments 56-61, wherein the therapeutic agent or prophylactic agent is a nucleic acid.
63. The composition according to any one of embodiments 56-62, wherein the therapeutic agent or prophylactic agent is ribonucleic acid (RNA).
64. The composition according to any one of embodiments 56-62, wherein the therapeutic agent or prophylactic agent is deoxyribonucleic acid (DNA).
65. The composition according to embodiment 63, wherein the RNA is selected from the group consisting of small interfering RNA (siRNA), asymmetric interfering RNA (aiRNA), microRNA (miRNA), Dicer-substrate RNA (dsRNA), small hairpin RNA (shRNA), messenger RNA (mRNA), and a mixture thereof.
66. The composition according to embodiment 65, wherein the RNA is mRNA.
67. The composition according to any one of embodiments 35-66, wherein the composition further comprises one or more of pharmaceutically acceptable excipients or diluents.
68. Use of the compound of formula (I), or an N-oxide, a solvate, a pharmaceutically acceptable salt or a stereoisomer thereof according to any one of embodiments 1 to 34, or the composition according to any one of embodiments 35 to 67 in the manufacture of a nucleic acid medicine, a gene vaccine, a small molecule medicine, a polypeptide or protein medicine.
69. Use of the compound of formula (I), or an N-oxide, a solvate, a pharmaceutically acceptable salt or a stereoisomer thereof according to any one of embodiments 1 to 34, or the composition according to any one of embodiments 35 to 67 in the manufacture of a medicament for treating a disease or condition in a mammal in need thereof.
70. The use according to embodiment 69, wherein the disease or condition is characterized by dysfunctional or abnormal protein or polypeptide activity.
71. The use according to any one of embodiments 69-70, wherein the disease or condition is selected from the group consisting of infectious diseases, cancer and proliferative diseases, genetic diseases, autoimmune diseases, diabetes, neurodegenerative diseases, cardiovascular and renovascular diseases, and metabolic diseases.
72. The use according to embodiment 71, wherein the infectious disease is selected from: diseases caused by coronavirus, influenza virus or HIV virus, infantile pneumonia, Rift Valley fever, yellow fever, rabies, and various herpes.
73. The use according to any one of embodiments 69-72, wherein the mammal is a human.
74. The use according to any one of embodiments 69-73, wherein the composition is administered intravenously, intramuscularly, intradermally, subcutaneously, intranasally or by inhalation.
75. The use according to embodiment 74, wherein the composition is administered subcutaneously.
76. The use according to any one of embodiments 69-75, wherein the therapeutic agent or prophylactic agent is administered to the mammal at a dose of about 0.001 mg/kg to about 10 mg/kg.

### Examples

The present disclosure will be further described below in conjunction with examples. However, the present disclosure is not limited to the following examples. The implementation conditions used in the examples can be further adjusted according to different requirements for specific use. Unspecified implementation conditions are conventional conditions in the industry. In the specific examples of the present disclosure, the raw materials used are all commercially available. Unless otherwise stated, percentages in this context are by weight and all temperatures are given in degrees Celsius. The technical features involved in the various embodiments of the present disclosure can be combined with each other as long as they do not conflict with each other.

The following abbreviations represent the following reagents:
DCM: dichloromethane; EDCI: 1-(3-dimethylaminopropyl)-3-ethylcarbodiimide hydrochloride; DMAP: 4-dimethylaminopyridine; DCC: N,N'- dicyclohexylcarbodiimide

### Example 1: Synthesis of Cationic Lipid Compounds

### 1. Intermediate 1 (INT-1): Synthesis of n-decyl 4-bromobutyrate

The synthesis route is as follows:

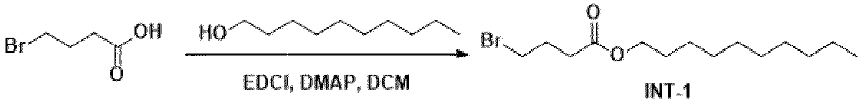

4-Bromobutyric acid (15.00g, 89.82mmol) and 1-decanol (12.90g, 81.50mmol) were dissolved in DCM (100mL), and EDCI (18.70g, 97.55mmol) and DMAP(500mg, 4.09mmol) were added to the above solution. The mixture was stirred and reacted at 30-35 °C for 8 hours. After the reaction was completed, the reaction solution was washed with saturated sodium carbonate, washed with saturated brine, and dried over Na₂SO₄. The mixture was filtered and the filtrate was concentrated under reduced pressure in vacuo. The residue was purified by silica gel chromatography (ethyl acetate/n-hexane) to obtain n-decyl 4-bromobutyrate (INT-1)(10.52g, 34.24mmol, 42.01%).

¹H NMR (400 MHz, CDCl₃) δ 4.12 (t, *J =* 6.8 Hz, 2H), 3.51 (t, *J =* 6.5 Hz, 2H), 2.54 (t, *J* = 7.2 Hz, 2H), 2.22 (p, *J =* 6.8 Hz, 2H), 1.66 (dd, *J =* 14.5, 7.3 Hz, 2H), 1.35 (dd, *J* = 19.1, 15.8 Hz, 14H), 0.92 (t, *J =* 6.9 Hz, 3H).

### 2. Intermediate 2 (INT-2): Synthesis of 3-hexylnonyl 6-bromohexanoate

The synthesis route is as follows:

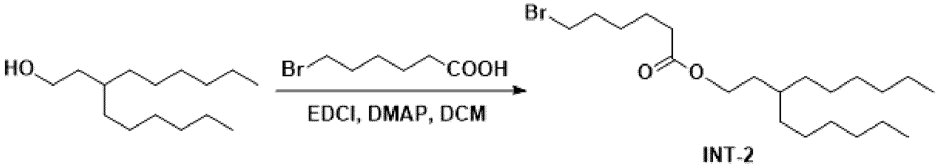

6-Bromocaproic acid (5.85g, 30.00mmol) and 3-hexylnonanol (6.24g, 27.30mmol) were dissolved in DCM (60mL), and EDCI (5.41g, 35.50mmol) and DMAP(333mg, 2.73mmol) were added to the above solution. The mixture was stirred and reacted at 30-35 °C for 9 hours. After the reaction was completed, the reaction solution was washed with saturated sodium carbonate, washed with saturated brine, and dried over Na₂SO₄. The mixture was filtered and the filtrate was concentrated under reduced pressure in vacuo. The residue was purified by silica gel chromatography (ethyl acetate/n-hexane) to obtain 3-hexylnonyl 6-bromohexanoate (INT-2)(7.89g, 19.46mmol, 71.3%).

¹H NMR (400 MHz, CDCl₃) δ 4.13 (t, *J =* 7.1 Hz, 2H), 3.45 (t, *J =* 6.8 Hz, 2H), 2.35 (t, *J =* 7.4 Hz, 2H), 1.98- 1.87 (m, 2H), 1.70 (dt, *J =* 20.6, 7.4 Hz, 2H), 1.65 - 1.57 (m, 2H), 1.52 (ddd, *J =* 8.6, 6.9, 4.2 Hz, 2H), 1.44 (s, 1H), 1.37- 1.24 (m, 20H), 0.93 (t, *J =* 6.8 Hz, 6H).

### 3. Synthesis of 3-hexylnonyl-6-(4-(decyloxy)-4-oxobutyl)((2-hydroxyethyl)amino)hexanoate (YK-101)

The synthesis route is as follows:

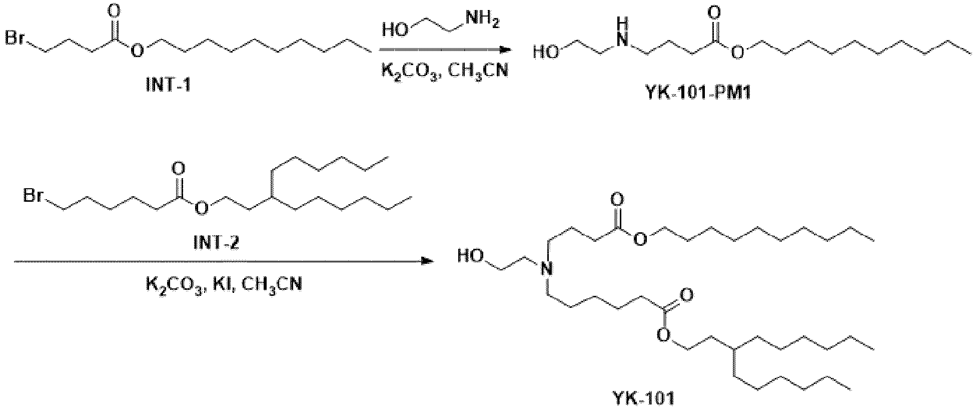

### Step 1: Synthesis of n-decyl 4-(2-hydroxyethylamino)butyrate (YK-101-PM1)

n-Decyl 4-bromobutyrate (4.30g, 14.00mmol) and ethanolamine (2.56g, 42.00mmol) were dissolved in acetonitrile (150mL). Potassium carbonate (1.94g, 14.00mmol) was added to the above system and the mixture was heated to 70 °C and stirred to react for 2 hours. After the reaction was completed, the reaction solution was cooled to room temperature and then filtered. The filtrate was concentrated under vacuum to remove the solvent. The residue was purified by silica gel chromatography (methanol / dichloromethane) to obtain n-decyl 4-((2-hydroxyethyl)amino)butyrate (2.01g, 6.97mmol, 49.72%), C₁₆H₃₃NO₃, MS(ES): m/z(M+H⁺)288.3.

### Step 2: Synthesis of 3-hexylnonyl-6-(4-(decyloxy)-4-oxobutyl)((2-hydroxyethyl)amino)hexanoate (YK-101)

3-Hexylnonyl 6-bromohexanoate (1.15g, 2.83mmol) and n-decyl 4-((2-hydroxyethyl)amino)butyrate (1.72g, 6mmol) were dissolved in acetonitrile (40mL). Potassium carbonate (1.56g, 11.32mmol) and potassium iodide (46mg, 0.28mmol) were added to the above system and the mixture was heated to 70 °C and stirred to react for 2 hours. The reaction solution was cooled to room temperature and then filtered. The filtrate was concentrated under vacuum to remove the solvent. The residue was purified by silica gel chromatography (ethyl acetate/n-hexane) to obtain the target compound (1.21g, 1.98mmol, 70.0%). For C₃₇H₇₃NO₅, MS(ES):m/z(M+H⁺)612.8.

¹H NMR (400 MHz, CDCl₃) δ 4.13 - 3.89 (m, 4H), 3.77 (dd, *J =* 10.2, 6.0 Hz, 2H), 3.53 - 3.30 (m, 2H), 2.97 - 2.81 (m, 1H), 2.77 (dd, *J =* 15.4, 7.3 Hz, 3H), 2.43 (t, *J =* 8.1 Hz, 1H), 2.31 (dd, *J =* 16.7, 9.4 Hz, 3H), 2.14 - 1.94 (m, 1H), 1.76 - 1.46 (m, 9H), 1.46 - 1.08 (m, 38H), 0.87 (t, *J =* 6.8 Hz, 9H).

### 4. Synthesis of 3-hexylnonyl-6-(4-(decyloxy)-4-oxobutyl)((3-hydroxypropyl)amino)hexanoate (YK-102)

The synthesis route is as follows:

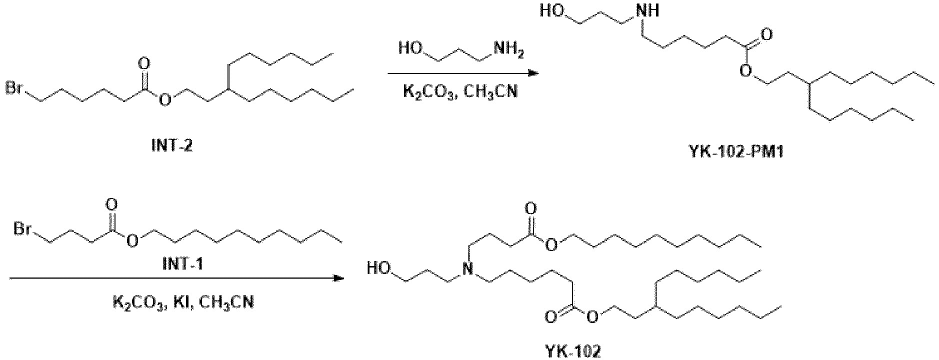

### Step 1: Synthesis of 3-hexylnonyl-6-((3-hydroxypropyl)amino)hexanoate (YK-102-PM1)

According to the method for preparing YK-101-PM2, INT-2(500mg, 1.23mmol) and 3-aminopropan-1-ol (282mg, 3.69mmol) were used as raw materials and subjected to purification by silica gel chromatography (methanol/dichloromethane) to give 3-hexylnonyl-6-((3-hydroxypropyl)amino)hexanoate 405mg, 1.01mmol, 82.4%. C₂₄H₄₉NO₃, MS(ES): m/z(M+H⁺)400.3.

### Step 2: Synthesis of 3-hexylnonyl-6-(4-(decyloxy)-4-oxobutyl)((3-hydroxypropyl)amino)hexanoate (YK-102)

According to the method for preparing YK-101, 3-hexylnonyl-6-((3-hydroxypropyl)amino)hexanoate (200mg, 0.50mmol) obtained from the above purification and INT-1 (199mg, 0.65mmol) were used as raw materials and subjected to purification by silica gel chromatography (ethyl acetate/n-hexane) to give the target compound (70mg, 0.11mmol, 22.4%). C₃₈H₇₅NO₅, MS(ES): m/z(M+H⁺)626.7.

¹H NMR (400 MHz, CDCl₃) δ 4.11 (q, *J =* 6.9 Hz, 4H), 3.88 - 3.73 (m, 2H), 2.78 (t, *J* = 5.8 Hz, 2H), 2.67 - 2.48 (m, 4H), 2.42 - 2.24 (m, 4H), 1.89 (dt, *J* = 14.9, 7.3 Hz, 2H), 1.78 (dt, *J =* 10.7, 5.5 Hz, 2H), 1.75 - 1.49 (m, 8H), 1.46 - 1.18 (m, 37H), 0.92 (t, *J =* 6.7 Hz, 9H).

### 5. Synthesis of 3-hexylnonyl-6-((6-(decyloxy)-6-oxohexyl)(2-hydroxyethyl)amino)hexanoate (YK-103)

The synthesis route is as follows:

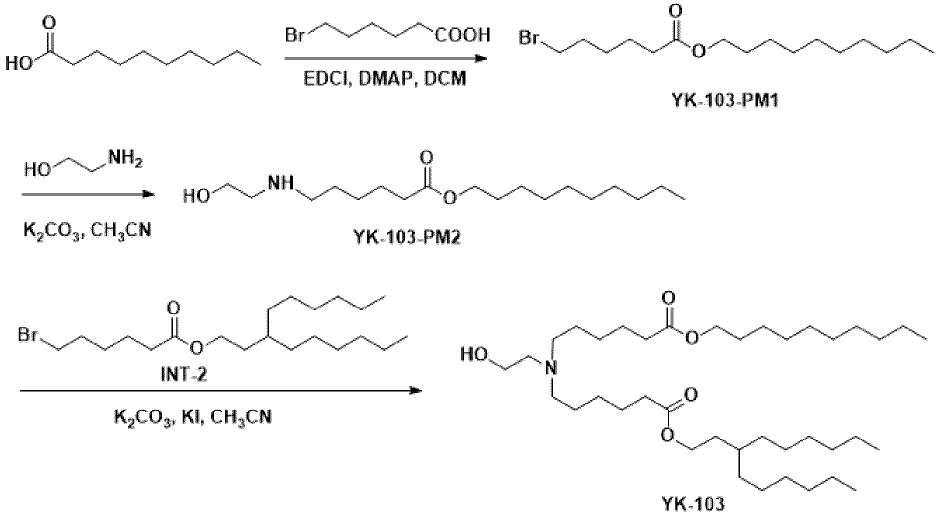

### Step 1: Synthesis of n-decyl 6-bromohexanoate (YK-103-PM1)

According to the method for preparing INT-1, 6-bromohexanoic acid (1.12g, 10.00mmol) and n-decanol (1.45g, 9.16mmol) were used as raw materials and subjected to purification by silica gel chromatography (ethyl acetate/n-hexane) to give n-decyl 6-bromohexanoate (2.8g, 8.35mmol, 91.2%).

### Step 2: Synthesis of n-decyl 6-((2-hydroxyethyl)amino)hexanoate (YK-103-PM2)

n-Decyl 6-bromohexanoate (1.12g, 3.47mmol) obtained through the above purification and ethanolamine (3.18g, 52.05mmol) were dissolved in ethanol (15mL), and the reaction mixture was stirred at room temperature for 16 hours. The reaction solution was concentrated under reduced pressure to remove the solvent, diluted with ethyl acetate (80 mL), and washed three times with brine (50 mL). The organic phase was concentrated under reduced pressure and purified by silica gel chromatography (methanol/ dichloromethane) to obtain n-decyl 6-((2-hydroxyethyl)amino)hexanoate (0.81g, 2.56mmol, 74.0%). C₁₈H₃₇NO₃, MS(ES):m/z(M+H⁺)316.4.

### Step 3: Synthesis of 3-hexylnonyl-6-((6-(decyloxy)-6-oxohexyl)(2-hydroxyethyl)amino)hexanoate (YK-103)

According to the method for preparing YK-101, n-decyl 6-((2-hydroxyethyl)amino)hexanoate (159mg, 0.51mmol) and INT-2 (202mg, 0.50mmol) were used as raw materials to give the target compound (81mg, 0.13mmol, 25.3%). C₃₉H₇₇NO₅, MS(ES): m/z(M+H⁺)640.8.

¹H NMR (400 MHz, CDCl₃) δ 4.08 (dt, *J =* 9.3, 7.0 Hz, 4H), 3.66 (t, *J =* 5.1 Hz, 2H), 2.74 (t, *J =* 4.8 Hz, 2H), 2.68 - 2.44 (m, 4H), 2.31 (td, *J =* 7.4, 2.9 Hz, 4H), 1.76 - 1.45 (m, 11H), 1.49 - 0.99 (m, 40H), 0.89 (t, *J =* 6.8 Hz, 9H).

### 6. Synthesis of 3-hexylnonyl-5-((4-(decyloxy)-4-oxobutyl)((2-hydroxyethyl)amino)pentanoate (YK-104)

The synthesis route is as follows:

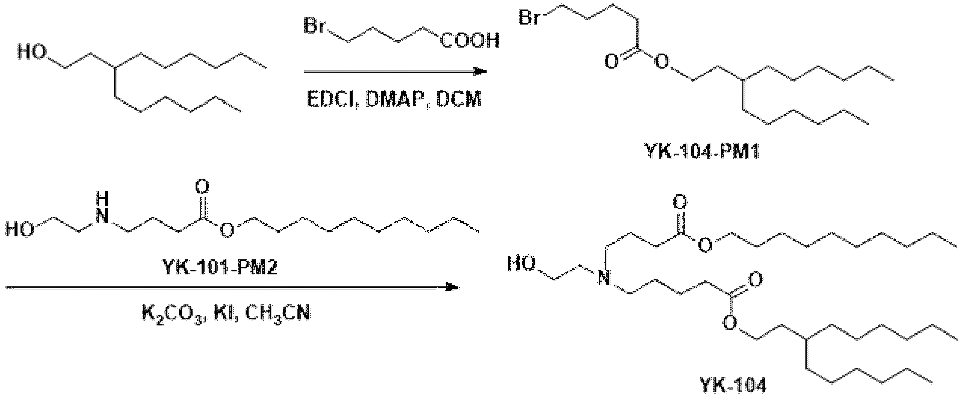

### Step 1: Synthesis of 3-hexylnonyl 5-bromopentanoate (YK-104-PM1)

According to the method for preparing INT-2, 3-hexylnonanol (1.14g, 5.00mmol) and 5-bromopentanoic acid (0.91g, 5.03mmol) were used as raw materials and subjected to purification by silica gel chromatography (ethyl acetate/n-hexane) to give 3-hexylnonyl 5-bromopentanoate (1.40g, 3.58mmol, 71.5%).

### Step 2: Synthesis of 3-hexylnonyl-5-((4-(decyloxy)-4-oxobutyl)((2-hydroxyethyl)amino)pentanoate (YK-104)

According to the method for preparing YK-101, 3-hexylnonyl 5-bromopentanoate (196mg, 0.50mmol) and n-decyl 4-((2-hydroxyethyl)amino)butyrate (145mg, 0.51mmol) were used as raw materials and subjected to purification by silica gel chromatography (ethyl acetate/n-hexane) to give the target compound (178mg, 0.30mmol, 59.5%). C₃₆H₇₁NO₅, MS(ES):m/z(M+H⁺)598.3.

¹H NMR (400 MHz, CDCl₃) δ 4.06 (dt, *J =* 8.5, 7.0 Hz, 4H), 3.71 (d, *J =* 7.0 Hz, 1H), 3.56 (t, *J =* 5.2 Hz, 2H), 2.62 (t*, J =* 5.2 Hz, 2H), 2.52 (dd, *J =* 14.9, 7.7 Hz, 4H), 2.31 (td, *J =* 7.3, 5.5 Hz, 4H), 1.85 - 1.73 (m, 2H), 1.66 - 1.41 (m, 6H), 1.43 - 1.11 (m, 37H), 0.87 (dd, *J* = 7.2, 6.4 Hz, 9H).

### 7. Synthesis of Hexyl 6-(((4-(decyloxy)-4-oxobutyl)(2-hydroxyethyl)amino)-3-hexylnonanoate (YK-105)

The synthesis route is as follows:

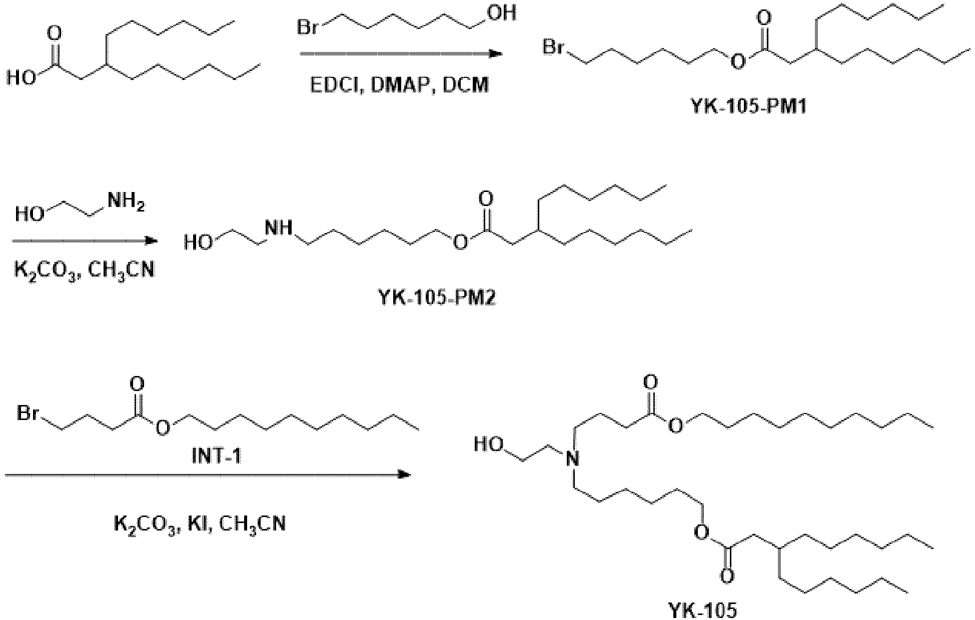

### Step 1: Synthesis of 6-bromohexyl 3-hexylnonanoate (YK-105-PM1)

According to the method for preparing INT-1, 6-bromohexanol (412mg, 2.18mmol) and 3-hexylnonanoic acid (460mg, 1.90mmol) were used as raw materials and subjected to purification by silica gel chromatography (ethyl acetate/n-hexane) to give 6-bromohexyl 3-hexylnonanoate (393mg, 0.97mmol, 51.0%).

### Step 2: Synthesis of 6-(2-hydroxyethyl-amino)hexyl 3-hexylnonanoate (YK-105-PM2)

According to the method for synthesizing YK-101-PM2, 6-bromohexyl 3-hexylnonanoate (393mg, 0.97mmol) and ethanolamine (178mg, 2.91mmol) were used as raw materials to give 6-(2-hydroxyethyl-amino)hexyl 3-hexylnonanoate (312mg, 0.81mmol, 83.4%), C₂₃H₄₇NO₃, MS(ES): m/z(M+H⁺)386.6.

### Step 3: Synthesis of hexyl 6-(((4-(decyloxy)-4-oxobutyl)(2-hydroxyethyl)amino)-3-hexylnonanoate (YK-105)

According to the method for preparing YK-101, 6-(2-hydroxyethyl-amino)hexyl 3-hexylnonanoate (312mg, 0.85mmol) and INT-1 (447mg, 1.54mmol) were used as raw materials to give the target compound (329mg, 0.54mmol, 63.2%). C₃₇H₇₃NO₅, MS(ES): m/z(M+H⁺)612.3.

¹H NMR (400 MHz, CDCl₃) δ 5.34 (s, 1H), 4.10 (td, *J =* 6.7, 5.1 Hz, 4H), 3.62 (d, *J* = 5.5 Hz, 2H), 2.62 (d, *J* = 44.1 Hz, 5H), 2.37 (t, *J =* 7.2 Hz, 2H), 2.26 (d, *J =* 6.9 Hz, 2H), 1.97 - 1.76 (m, 3H), 1.74 - 1.59 (m, 4H), 1.53 (s, 2H), 1.48 - 1.06 (m, 39H), 0.92 (t, *J=* 6.7 Hz, 9H).

### 8. Synthesis of 3-hexylnonyl-6-((10-oxodecyl-10-oxy-4-butyl)((2-hydroxyethyl)amino) hexanoate (YK-106)

The synthesis route is as follows:

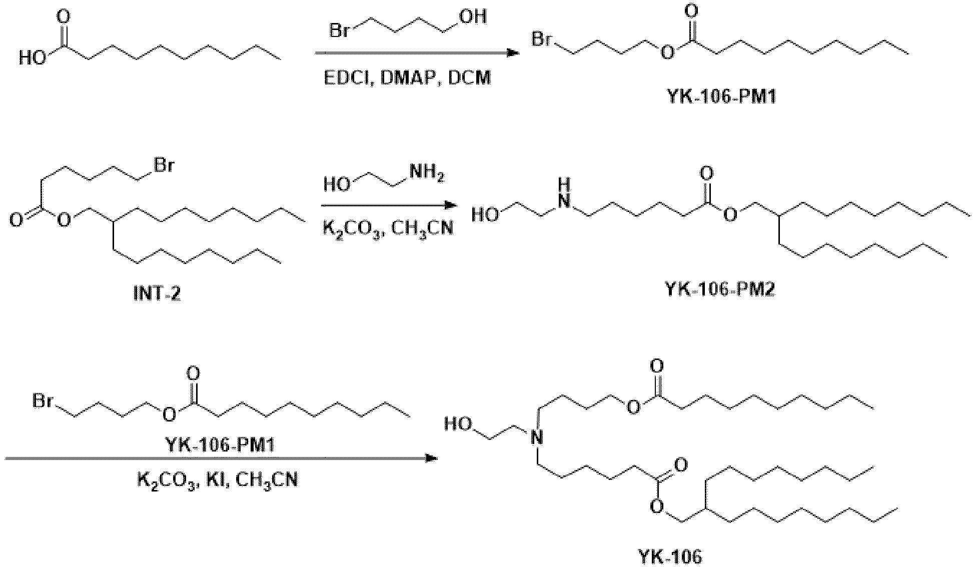

### Step 1: Synthesis of 4-bromobutyl n-decanoate (YK-106-PM1)

4-Bromobutanol (888mg, 5.80mmol) and n-decanoic acid (1.00g, 5.81mmol) were dissolved in DCM (20mL), and EDCI (1.46g, 7.62mmol) and DMAP (71mg, 0.58mmol) were added to the above solution. The mixture was stirred and reacted at 30-35 °C for 7 hours. After the reaction was completed, the reaction solution was washed once with saturated sodium carbonate and saturated brine in sequence. The organic phase was dried over Na₂SO₄ and filtered. The filtrate was concentrated under vacuum and reduced pressure. The residue was purified by silica gel chromatography (ethyl acetate/n-hexane) to obtain 4-bromobutyl n-decanoate (600mg, 1.95mmol, 33.7%).

### Step 2: Synthesis of 2-octyldecyl 6-((2-hydroxyethyl)amino)hexanoate (YK-106-PM2)

According to the method for synthesizing YK-101-PM2, INT-2(200mg, 0.45mmol) and 2-aminoethanol (81.8mg, 1.34mmol) were used as raw materials and subjected to purification by silica gel chromatography (ethyl acetate/n-hexane) to give 2-octyldecyl 6-((2-hydroxyethyl)amino)hexanoate (106mg, 0.25mmol, 55.1%). C₂₆H₅₃NO₃, MS(ES): m/z(M+H⁺)428.5.

### Step 3: Synthesis of 2-octyldecyl 6-((10-oxodecyl-10-oxy-4-butyl)((2-hydroxyethyl)amino)hexanoate (YK-106)

According to the method for preparing YK-101, 2-octyldecyl 6-((2-hydroxyethyl)amino)hexanoate (140mg, 0.33mmol) and 4-bromobutyl n-decanoate (181mg, 0.59mmol) were used as raw materials to give the target compound (60mg, 0.09mmol, 27.8%). C₄₀H₇₉NO₅, MS(ES):m/z(M+H⁺)654.5.

¹H NMR (400 MHz, CDCl₃) δ 4.13 - 3.93 (m, 2H), 3.90 (d, *J =* 5.8 Hz, 2H), 3.76 - 3.56 (m, 2H), 2.88 - 2.53 (m, 6H), 2.24 (q, *J =* 7.4 Hz, 4H), 1.58 (dddd, *J =* 23.9, 21.6, 10.9, 5.4 Hz, 13H), 1.41 - 1.01 (m, 40H), 0.81 (td, *J* = 6.9, 1.3 Hz, 9H).

### 9. Synthesis of 2-octyldecyl 6-(4-(decyloxy)-4-oxobutyl)((3-hydroxypropyl)amino)hexanoate (YK-107)

The synthesis route is as follows:

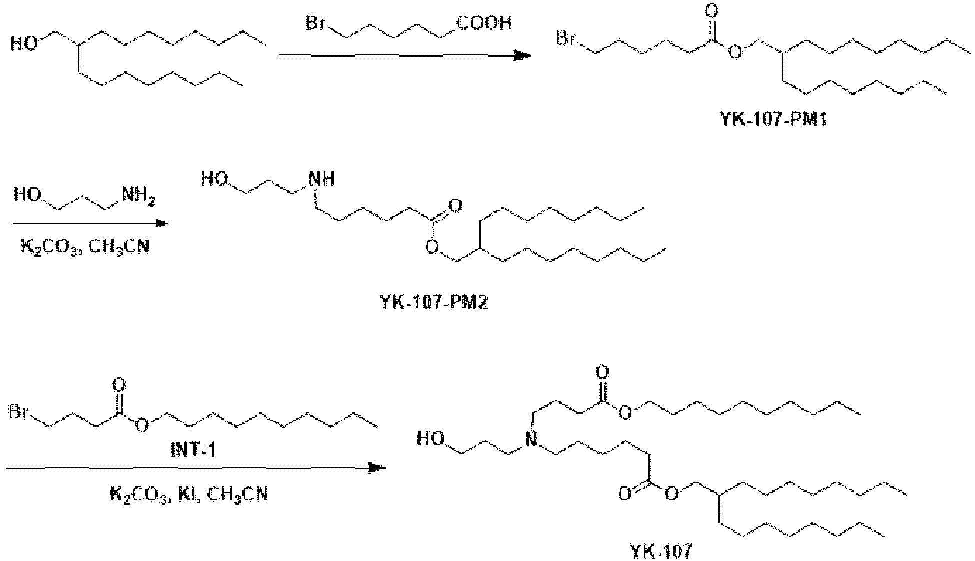

### Step 1: Synthesis of 2-octyldecyl 6-bromohexanoate (YK-107-PM1)

According to the method for preparing INT-1, 2-octyldecanol (3.00g, 11.09mmol) and 6-bromohexanoic acid (2.60g, 13.33mmol) were used as raw materials to give 2-octyldecyl 6-bromohexanoate (3.05g, 6.82mmol, 61.50%).

### Step 2: Synthesis of 2-octyldecyl 6-((3-hydroxypropyl)amino)hexanoate (YK-107-PM2)

According to the method for synthesizing YK-101-PM2, 2-octyldecyl 6-bromohexanoate (200mg, 0.45mmol) and 3-aminopropan-1-ol (102mg, 1.34mmol) were used as raw materials to give 2-octyldecyl 6-((3-hydroxypropyl)amino)hexanoate 126mg, 0.29mmol, 63.4%. C₂₇H₅₅NO₃, MS(ES): m/z(M+H⁺)442.3.

### Step 3: Synthesis of 2-octyldecyl 6-(4-(decyloxy)-4-oxobutyl)((3-hydroxypropyl)amino)hexanoate (YK-107)

According to the method for preparing YK-101, 2-octyldecyl 6-((3-hydroxypropyl)amino)hexanoate (106mg, 0.24mmol) and INT-1 (118mg, 0.38mmol) were used as raw materials to give the target compound (88mg, 0.13mmol, 54.9%). C₄₁H₈₁NO₅, MS(ES): m/z(M+H⁺)668.8.

¹H NMR (400 MHz, CDCl₃) δ 4.89 (t, *J =* 6.2 Hz, 1H), 4.72 (s, 2H), 4.08 (t, *J =* 6.8 Hz, 2H), 3.57 (s, 2H), 2.44 (s, 3H), 2.28 (td, *J =* 7.6, 3.3 Hz, 4H), 1.81 - 1.41 (m, 15H), 1.30 (d, *J* = 4.1 Hz, 41H), 0.92 (td, *J* = 6.8, 1.6 Hz, 9H).

### 10. Synthesis of 3-hexylnonyl 7-(4-(undecyloxy)-4-oxobutyl)((2-hydroxyethyl)amino)heptanoate (YK-108)

The synthesis route is as follows:

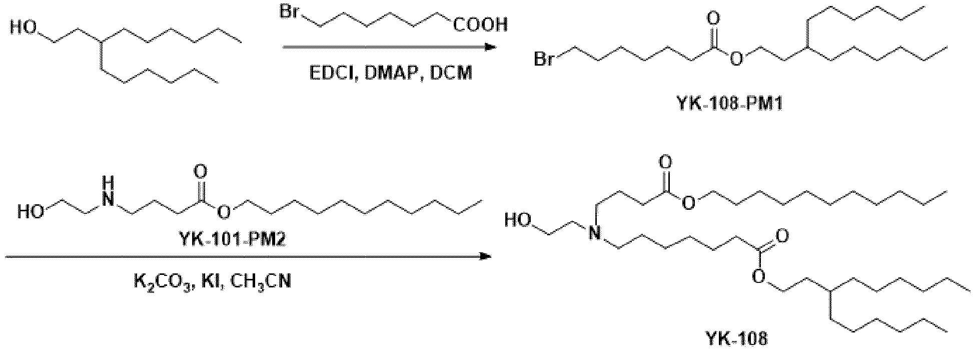

### Step 1: Synthesis of 3-hexylnonyl 7-bromoheptanoate (YK-108-PM1)

7-Bromoheptanoic acid (4.48g, 21.43mmol) and 3-hexylnonanol (5.00g, 21.89mmol) were dissolved in DCM (150mL). EDCI (4.86g, 25.35mmol) and DMAP(261mg, 2.14mmol) were added to the above solution. The mixture was stirred and reacted at 30-35 °C for 10 hours. After the reaction was completed, the reaction solution was washed with saturated sodium carbonate, washed with saturated brine, and dried over Na₂SO₄. The mixture was filtered and the filtrate was concentrated under reduced pressure in vacuo. The residue was purified by silica gel chromatography (ethyl acetate/n-hexane) to obtain 3-hexylnonyl 7-bromoheptanoate (5.98g, 14.26mmol, 66.5%).

### Step 2: Synthesis of 3-hexylnonyl 7-(4-(undecyloxy)-4-oxobutyl)((2-hydroxyethyl)amino)heptanoate (YK-108)

n-Undecyl 4-(2-hydroxyethylamino)butyrate (2.01g, 6.67mmol) and 3-hexylnonyl 7-bromoheptanoate (3.06g, 7.29mmol) were dissolved in acetonitrile (100mL). Potassium carbonate (3.65g, 26.41mmol) and potassium iodide (110 mg, 0.66mmol) were added to the above system and the mixture was heated to 70 °C and stirred to react for 2 hours. The reaction solution was cooled to room temperature and then filtered. The filtrate was concentrated under vacuum to remove the solvent. The residue was purified by silica gel chromatography (ethyl acetate/n-hexane) to obtain the target compound (1.52g, 2.37mmol, 35.5%). C₃₉H₇₇NO₅, MS(ES):m/z(M+H⁺)640.8.

¹H NMR (400 MHz, CDCl₃) δ 4.08 (q, *J* = 7.1 Hz, 4H), 3.55 (t, *J* = 5.3 Hz, 2H), 2.60 (t, *J =* 5.2 Hz, 2H), 2.57 - 2.41 (m, 4H), 2.31 (dt, *J* = 14.7, 7.4 Hz, 4H), 1.79 (p, *J =* 7.2 Hz, 2H), 1.61 (ddt, *J* = 20.9, 13.8, 7.0 Hz, 5H), 1.51 - 1.37 (m, 3H), 1.40 - 1.10 (m, 42H), 0.99 - 0.78 (m, 9H).

### 11. Synthesis of 3-hexylnonyl 6-((10-oxodecyl-10-oxy-4-butyl)((2-hydroxyethyl)amino)hexanoate (YK-109)

The synthesis route is as follows:

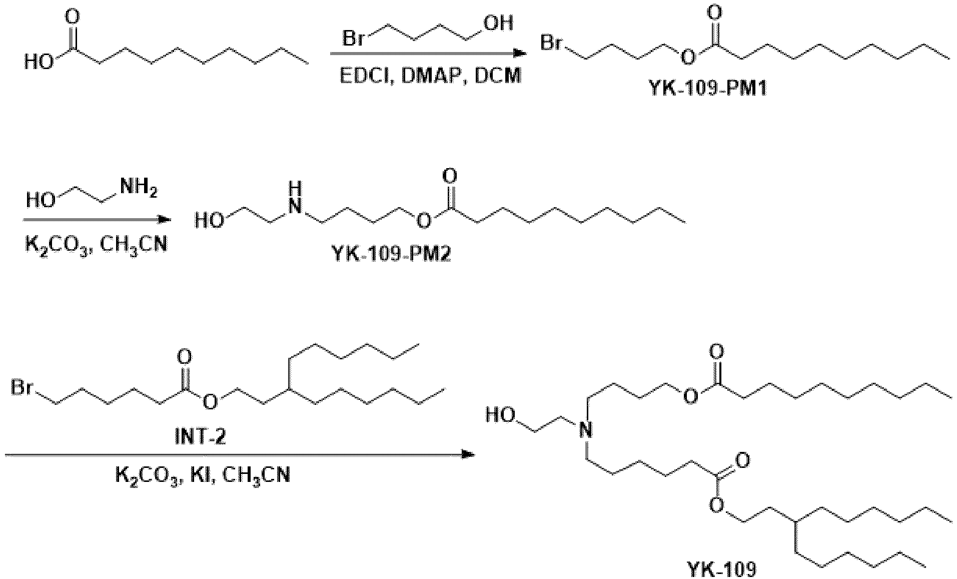

### Step 1: Synthesis of 4-bromobutyl n-decanoate (YK-109-PM1)

4-Bromobutanol (888mg, 5.80mmol) and n-decanoic acid (1.00g, 5.81mmol) were dissolved in DCM (20mL), and EDCI (1.46g, 7.62mmol) and DMAP (71mg, 0.58mmol) were added to the above solution. The mixture was stirred and reacted at 30-35 °C for 7 hours. After the reaction was completed, the reaction solution was washed once with saturated sodium carbonate and saturated brine in sequence. The organic phase was dried over Na₂SO₄ and filtered. The filtrate was concentrated under vacuum and reduced pressure. The residue was purified by silica gel chromatography (ethyl acetate/n-hexane) to obtain 4-bromobutyl n-decanoate (600mg, 1.95mmol, 33.7%).

### Step 2: Synthesis of 4-(2-hydroxyethyl-amino)butyl n-decanoate (YK-109-PM2)

The above purified 4-bromobutyl n-decanoate (600mg, 1.96mmol), ethanolamine (360mg, 5.88mmol) were dissolved in acetonitrile (6mL). Potassium carbonate (813mg, 5.88mmol) was added to the above system and the mixture was heated to 70 °C and stirred to react for 2 hours. After the reaction was completed, the reaction solution was cooled to room temperature and then filtered. The filtrate was concentrated under vacuum to remove the solvent. The residue was purified by silica gel chromatography (methanol / dichloromethane) to obtain 4-(2-hydroxyethyl-amino)butyl n-decanoate (250mg, 0.87mmol, 44.4%), C₁₆H₃₃NO₃, MS(ES): m/z(M+H⁺)288.4.

### Step 3: Synthesis of 3-hexylnonyl 6-((10-oxodecyl-10-oxy-4-butyl)((2-hydroxyethyl)amino)hexanoate (YK-109)

The purified 4-(2-hydroxyethyl-amino)butyl n-decanoate (107mg, 0.35mmol), 3-hexylnonyl 6-bromohexanoate (300mg, 0.75mmol) were dissolved in acetonitrile (5mL). Potassium carbonate (193mg, 1.40mmol) and potassium iodide (6 mg, 0.04mmol) were added to the above system and the mixture was heated to 70 °C and stirred to react for 2 hours. The reaction solution was cooled to room temperature and then filtered. The filtrate was concentrated under vacuum to remove the solvent. The residue was purified by silica gel chromatography (ethyl acetate/n-hexane) to obtain the target compound (150mg, 0.25mmol, 70.0%). C₃₇H₇₃NO₅, MS(ES):m/z(M+H⁺)612.8.

¹H NMR (400 MHz, CDCl₃) δ 4.09 (dt, *J =* 12.9, 7.0 Hz, 4H), 3.96 (d, *J =* 5.8 Hz, 1H), 3.63 (t, *J* = 5.2 Hz, 2H), 2.89 - 2.45 (m, 6H), 2.43 - 2.18 (m, 4H), 1.79 - 1.43 (m, 11H), 1.45 - 1.05 (m, 36H), 0.87 (td, *J* = 6.7, 1.6 Hz, 9H).

### 12. Synthesis of 3-hexylnonyl 6-(6-(2-(butylthio)ethoxy)-6-oxohexyl)((2-hydroxyethyl)amino)hexanoate (YK-110)

The synthesis route is as follows:

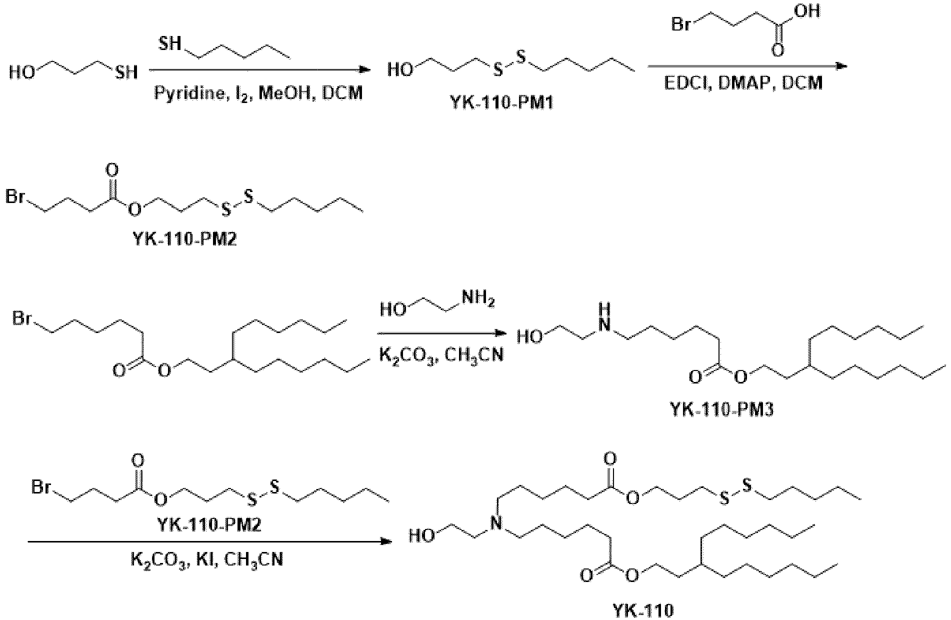

### Step 1: Synthesis of 3-(5-pentyl-dithio)propan-1-ol (YK-110-PM1)

3-Sulfydrylpropan-1-ol (1.00g, 10.85mmol), n-pentylthiol (1.13g, 10.85mmol), and pyridine (1.71g, 21.62mmol) were added in sequence to a mixture of DCM (50mL) and methanol (50mL). Iodine particles (2.74 g, 10.85 mmol) were added to the above solution in two portions under nitrogen, and the mixture was stirred to react at room temperature for 20 hours. After the reaction was completed, 50 mL of DCM was added. The mixture was washed with saturated sodium carbonate and saturated brine, and dried over Na₂SO₄. The mixture was filtered and the filtrate was concentrated under reduced pressure and in vacuo. The residue was purified by silica gel chromatography (ethyl acetate/n-hexane) to obtain 3-(5-pentyl-dithio)propan-1-ol (0.66g, 3.40mmol, 31.3%).

### Step 2: Synthesis of 3-(5-pentyl-dithio)propyl 4-bromobutyrate (YK-110-PM2)

According to the method for preparing INT-1, 4-bromobutyric acid (119mg, 0.61mmol) and 3-(5-pentyl-dithio)propan-1-ol (100mg, 0.51mmol) were used as raw materials and subjected to purification by silica gel chromatography (ethyl acetate/n-hexane) to give 3-(5-pentyl-dithio)propyl 4-bromobutyrate (140mg, 0.41mmol, 80.0%).

### Step 3: Synthesis of 3-hexylnonyl 6-((2-hydroxyethyl)amino)hexanoate (YK-110-PM3)

According to the method for YK-101-PM2, INT-2(500mg, 1.23mmol) and 2-aminoethanol (226mg, 3.70mmol) were used as raw materials and subjected to purification by silica gel chromatography (ethyl acetate/n-hexane) to give 3-hexylnonyl 6-((2-hydroxyethyl)amino)hexanoate (248mg, 0.64mmol, 52.3%). C₂₃H₄₇NO₃, MS(ES): m/z(M+H⁺)386.5.

### Step 4: Synthesis of 3-hexylnonyl 6-(4-(2-(butylthio)ethoxy)-4-oxobutyl)((2-hydroxyethyl)amino)hexanoate (YK-110)

According to the method for preparing YK-101, 3-hexylnonyl-6-((2-hydroxyethyl)amino)hexanoate (200mg, 0.52mmol) and 3-(5-pentyl-dithio)propyl 4-bromobutyrate (349mg, 0.94mmol) were used as raw materials to give the target compound (110mg, 0.16mmol, 31.3%). C₃₅H₆₉NO₅S, MS(ES): m/z(M+H⁺)676.4.

¹H NMR (400 MHz, CDCl₃) δ 4.21 (t, *J* = 6.3 Hz, 2H), 4.12 (t, *J* = 7.1 Hz, 2H), 3.70 (s, 2H), 2.86 - 2.52 (m, 4H), 2.45 - 2.21 (m, 4H), 2.08 (dt, *J* = 13.3, 6.5 Hz, 2H), 1.77 - 1.50 (m, 15H), 1.50 - 1.18 (m, 32H), 0.94 (dt, *J* = 9.3, 7.0 Hz, 9H).

### 13. Synthesis of 2-octyldecyl 6-(6-(2-(butylthio)ethoxy)-6-oxohexyl)((2-hydroxyethyl)amino)hexanoate (YK-111)

The synthesis route is as follows:

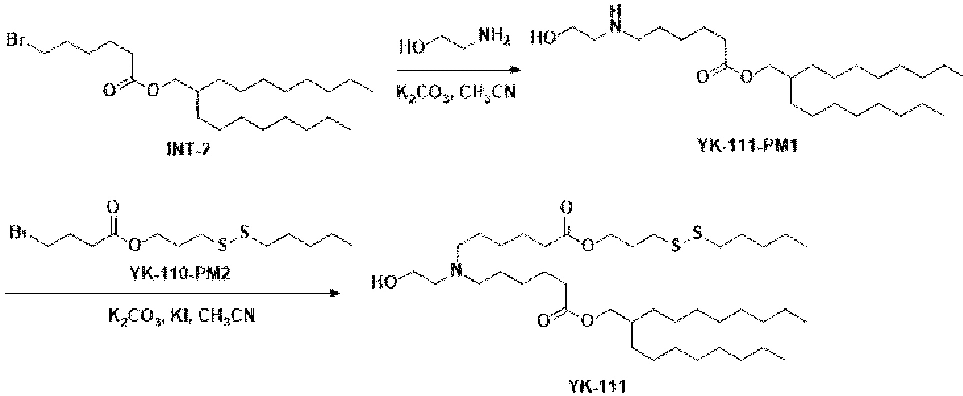

### Step 1: Synthesis of 2-octyldecyl 6-((2-hydroxyethyl)amino)hexanoate (YK-111-PM1)

According to the method for synthesizing YK-101-PM2, INT-2(200mg, 0.45mmol) and 2-aminoethanol (81.8mg, 1.34mmol) were used as raw materials and subjected to purification by silica gel chromatography (ethyl acetate/n-hexane) to give 2-octyldecyl 6-((2-hydroxyethyl)amino)hexanoate (106mg, 0.25mmol, 55.1%). C₂₆H₅₃NO₃, MS(ES): m/z(M+H⁺)428.5.

### Step 2: Synthesis of 2-octyldecyl-6-(4-(2-(butylthio)ethoxy)-4-oxobutyl)((2-hydroxyethyl)amino)hexanoate (YK-111)

According to the method for preparing YK-101, 2-octyldecyl 6-((2-hydroxyethyl)amino)hexanoate (130mg, 0.30mmol) and 3-(5-pentyl-dithio)propyl 4-bromobutyrate (200mg, 0.54mmol) were used as raw materials to give the target compound (60mg, 0.08mmol, 27.8%). C₄₀H₇₉NO₅S₂, MS(ES): m/z(M+H⁺)718.2.

¹H NMR (400 MHz, CDCl₃) δ 4.10 (td, *J* = 6.3, 2.1 Hz, 3H), 3.90 (d, *J* = 5.8 Hz, 2H), 3.55 (d, *J* = *5.3* Hz, 2H), 2.75 - 2.56 (m, 6H), 2.50 (d, *J* = 7.4 Hz, 4H), 2.25 (td, *J* = 7.3, 1.4 Hz, 6H), 2.06 - 1.91 (m, 2H), 1.70 - 1.37 (m, 9H), 1.37 - 0.97 (m, 36H), 0.83 (dt, *J* = 10.4, 7.0 Hz, 9H).

### 14. Synthesis of 3-hexylnonyl-6-(6-(2-(butylthio)ethoxy)-6-oxohexyl)((2-hydroxyethyl)amino)hexanoate (YK-112)

The synthesis route is as follows:

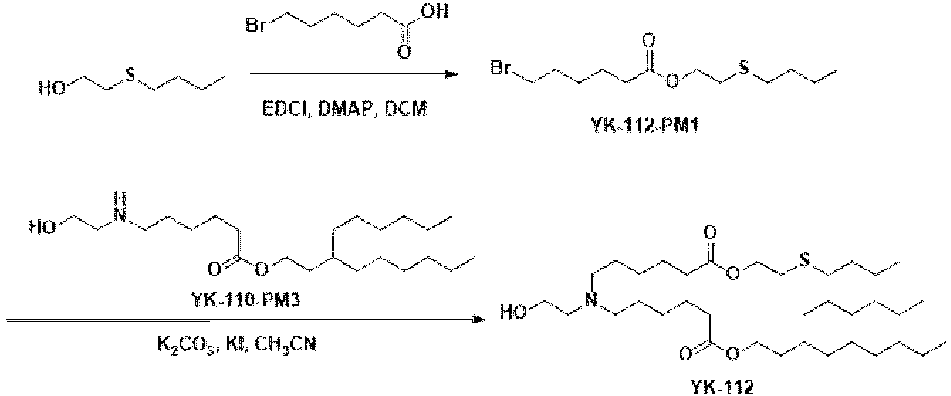

### Step 1: Synthesis of 2-(butylthio)ethyl 6-bromohexanoate (YK-112-PM1):

According to the method for preparing INT-1, 6-bromohexanoic acid(308mg, 1.58mmol) and 2-(butylthio)ethan-1-ol (200mg, 1.49mmol) were used as raw materials and subjected to purification by silica gel chromatography (ethyl acetate/n-hexane) to give 2-(butylthio)ethyl 6-bromohexanoate (0.35g, 1.12mmol, 75.5%).

### Step 2: Synthesis of 3-hexylnonyl 6-(6-(2-(butylthio)ethoxy)-6-oxohexyl)((2-hydroxyethyl)amino)hexanoate (YK-112)

According to the method for preparing YK-101, 3-hexylnonyl-6-((2-hydroxyethyl)amino)hexanoate (200mg, 0.52mmol) and 2-(butylthio)ethyl 6-bromohexanoate (291mg, 0.93mmol) were used as raw materials to give the target compound (67mg, 0.11mmol, 20.9%). C₃₅H₆₉NO₅S, MS(ES): m/z(M+H⁺)616.5.

¹H NMR (400 MHz, CDCl₃) δ 4.26 (t, *J* = 7.0 Hz, 2H), 4.12 (t, *J* = 7.1 Hz, 2H), 3.62 (t, *J* = 5.1 Hz, 2H), 2.77 (t, *J =* 7.0 Hz, 2H), 2.68 (t, *J =* 4.9 Hz, 2H), 2.64 - 2.47 (m, 6H), 2.35 (dt, *J* = 12.7, 7.5 Hz, 4H), 1.79 - 1.49 (m, 12H), 1.49 - 1.13 (m, 28H), 1.04 - 0.81 (m, 9H).

### 15. Synthesis of 2-octyldecyl 6-(6-(2-(butylthio)ethoxy)-6-oxohexyl)((2-hydroxyethyl)amino)hexanoate (YK-113)

The synthesis route is as follows:

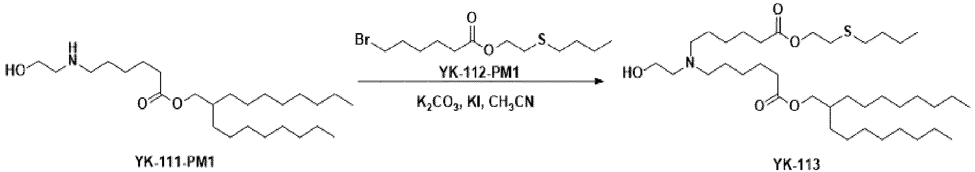

According to the method for preparing YK-101, 2-octyldecyl 6-((2-hydroxyethyl)amino)hexanoate (200mg, 0.52mmol) and 2-(butylthio)ethyl 6-bromohexanoate (287mg, 0.93mmol) were used as raw materials to give the target compound (214mg, 0.33mmol, 62.5%). C₃₈H₇₅NO₅S, MS(ES): m/z(M+H⁺) 658.6.

¹H NMR (400 MHz, CDCl₃) δ 4.15 (t, *J* = 7.0 Hz, 2H), 3.90 (d, *J* = 5.8 Hz, 2H), 3.53 (t, *J =* 5.2 Hz, 2H), 2.66 (t, *J =* 7.0 Hz, 2H), 2.60 (t, *J =* 5.3 Hz, 2H), 2.49 (ddt, *J =* 9.5, 7.8, 2.6 Hz, 6H), 2.25 (q, *J =* 7.3 Hz, 4H), 1.68 - 1.40 (m, 9H), 1.39 - 1.05 (m, 37H), 0.91 - 0.69 (m, 9H).

### 16. Synthesis of 3-hexylnonyl 6-(4-(decyloxy)-4-oxobutyl)((2,3-dihydroxypropyl)amino)hexanoate (YK-114)

The synthesis route is as follows:

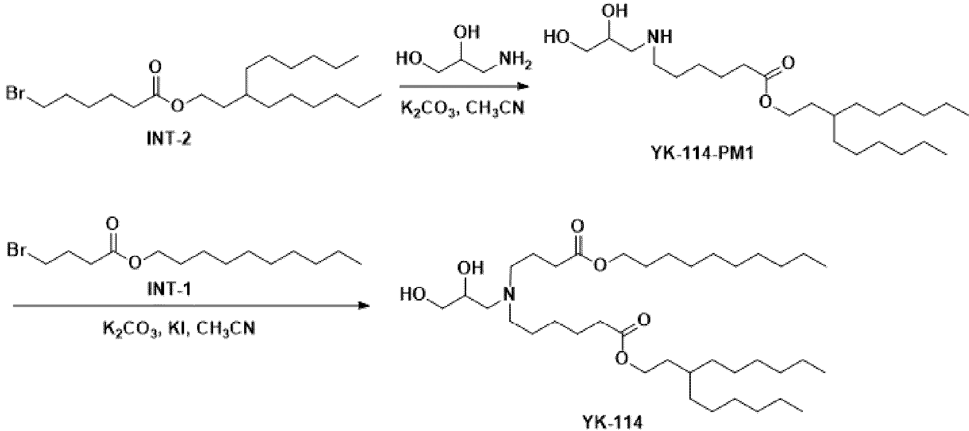

### Step 1: Synthesis of 3-hexylnonyl 6-((2,3-dihydroxypropyl)amino)hexanoate (YK-114-PM1)

According to the method for synthesizing YK-101-PM2, INT-2(1014mg, 2.50mmol) and 3-aminopropan-1,2-diol (273mg, 3.00mmol) were used as raw materials to give 3-hexylnonyl-6-((2,3-dihydroxypropyl)amino)hexanoate 400mg, 0.96mmol, 38.5%). C₂₄H₄₉NO₄, MS(ES): m/z(M+H⁺)416.4.

### Step 2: Synthesis of 3-hexylnonyl 6-(4-(decyloxy)-4-oxobutyl)((-2,3-dihydroxypropyl)amino)hexanoate (YK-114)

According to the method for preparing YK-101, 3-hexylnonyl-6-((2,3-dihydroxypropyl)amino)hexanoate (400mg, 0.96mmol) and INT-1 (554mg, 1.93mmol) were used as raw materials to give the target compound (350mg, 0.55mmol, 56.8%). C₃₈H₇₅NO₆, MS(ES): m/z(M+H⁺)642.5.

¹H NMR (400 MHz, CDCl₃) δ 4.17 - 4.04 (m, 4H), 3.89 (s, 1H), 3.78 (dd, *J* = 11.4, 3.9 Hz, 1H), 3.68 (s, 1H), 3.55 (dd, *J* = 11.4, 4.4 Hz, 1H), 2.88 - 2.50 (m, 6H), 2.45 - 2.22 (m, 4H), 1.91 (d, *J* = 7.8 Hz, 2H), 1.75 - 1.50 (m, 8H), 1.48 - 1.10 (m, 38H), 1.00 - 0.80 (m, 9H).

### 17. Synthesis of 3-hexylnonyl 6-(4-(decyloxy)-4-oxobutyl)((R)-2,3-dihydroxypropyl)amino)hexanoate (YK-115)

The synthesis route is as follows:

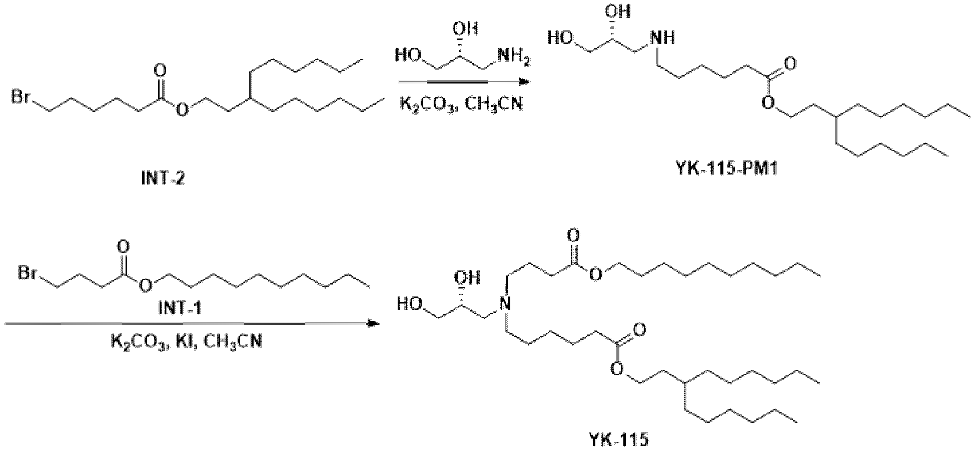

### Step 1: Synthesis of 3-hexylnonyl 6-(((R)2,3-dihydroxypropyl)amino)hexanoate (YK-115-PM1)

According to the method for synthesizing YK-101-PM2, INT-2(890mg, 2.19mmol) and (*R*)-3-aminopropan-1,2-diol (200mg, 2.19mmol) were used as raw materials to give 3-hexylnonyl-6-(((*R*)2,3-dihydroxypropyl)amino)hexanoate 400mg, 0.96mmol, 43.9%). C₂₄H₄₉NO₄, MS(ES): m/z(M+H⁺)416.4.

### Step 2: Synthesis of 3-hexylnonyl 6-(4-(decyloxy)-4-oxobutyl)((R)-2,3-dihydroxypropyl)amino)hexanoate (YK-115)

According to the method for preparing YK-101, 3-hexylnonyl-6-(((*R*)2,3-dihydroxypropyl)amino)hexanoate (200mg, 0.48mmol) and INT-1 (295mg, 0.96mmol) were used as raw materials to give the target compound (125mg, 0.19mmol, 40.6%). C₃₈H₇₅NO₆, MS(ES): m/z(M+H⁺)642.5.

¹H NMR (400 MHz, CDCl₃) δ 4.07 (q, *J =* 7.0 Hz, 4H), 3.88 - 3.64 (m, 2H), 3.48 (dd, *J* = 8.1, 2.6 Hz, 1H), 2.84 - 2.36 (m, 7H), 2.40 - 2.20 (m, 4H), 1.93 - 1.71 (m, 2H), 1.72 - 1.43 (m, 8H), 1.43 - 1.04 (m, 38H), 0.88 (t, *J=* 6.7 Hz, 9H).

### 18. Synthesis of 3-hexylnonyl 6-(4-(decyloxy)-4-oxobutyl)(((S)-2,3-dihydroxypropyl)amino)hexanoate (YK-116)

The synthesis route is as follows:

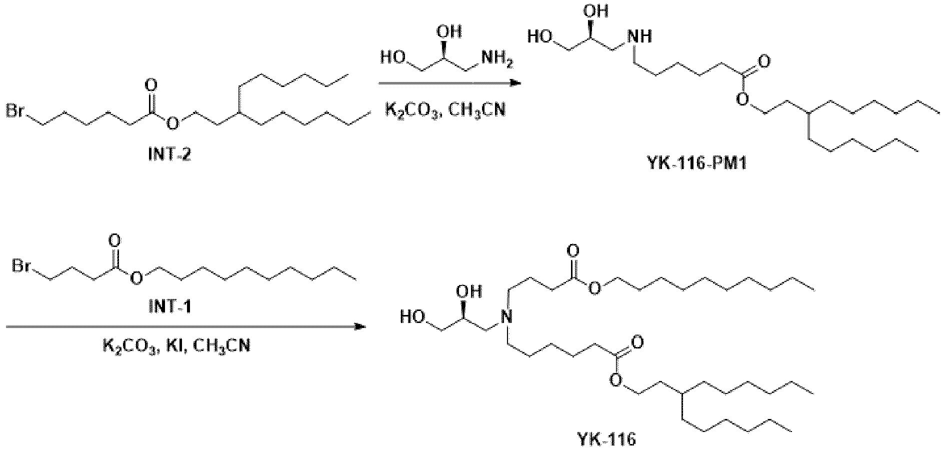

### Step 1: Synthesis of 3-hexylnonyl 6-(((S)2,3-dihydroxypropyl)amino)hexanoate (YK-116-PM1)

According to the method for synthesizing YK-101-PM2, INT-2(890mg, 2.19mmol) and (*S*)-3-aminopropan-1,2-diol (200mg, 2.19mmol) were used as raw materials to give 3-hexylnonyl 6-(((S)2,3-dihydroxypropyl)amino)hexanoate 250mg, 0.60mmol, 27.5%). C₂₄H₄₉NO₄, MS(ES): m/z(M+H⁺)416.5.

### Step 2: Synthesis of 3-hexylnonyl 6-(4-(decyloxy)-4-oxobutyl)(((S)-2,3-dihydroxypropyl)amino)hexanoate (YK-116)

According to the method for preparing YK-101, 3-hexylnonyl 6-(((S)2,3-dihydroxypropyl)amino)hexanoate (200mg, 0.48mmol) and INT-1 (278mg, 0.97mmol) were used as raw materials to give the target compound (205mg, 0.32mmol, 66.5%). C₃₈H₇₅NO₆, MS(ES): m/z(M+H⁺)642.5.

¹H NMR (400 MHz, CDCl₃) δ 4.11 - 3.90 (m, 4H), 3.89 (d, *J* = 7.3 Hz, 1H), 3.78 (d, *J* = 7.3 Hz, 1H), 3.67 (dd, *J* = 11.4, 3.9 Hz, 1H), 3.51 - 3.34 (m, 1H), 2.55 (ddd, *J* = 52.8, 24.9, 9.9 Hz, 6H), 2.36 - 2.15 (m, 4H), 1.80 (d, *J* = 8.7 Hz, 2H), 1.66 - 1.40 (m, 8H), 1.38 - 1.06 (m, 38H), 0.91 - 0.71 (m, 9H).

### 19. Synthesis of 3-hexylnonyl 6-(4-(decyloxy)-4-oxobutyl)(((S)2-hydroxypropyl)amino)hexanoate (YK-117)

The synthesis route is as follows:

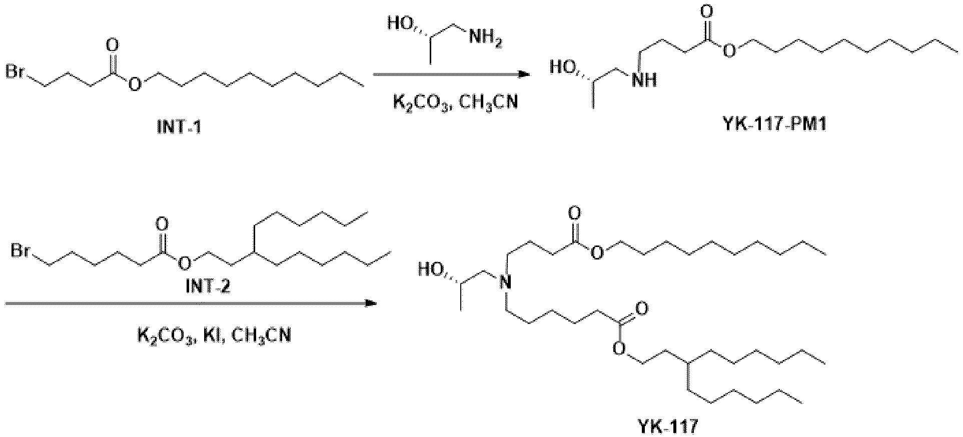

### Step 1: Synthesis of decyl 4-(((S)2-hydroxypropyl)amino)butyrate (YK-117-PM1)

According to the method for synthesizing YK-101-PM2, INT-1(1.03g, 3.37mmol) and (S)-1-aminopropan-2-ol (253mg, 3.37mmol) were used as raw materials to give decyl 4-(((S)2-hydroxypropyl)amino)butyrate 300mg, 1.00mmol, 29.5%). C₁₈H₃₇NO₃, MS(ES): m/z(M+H⁺)302.4.

### Step 2: Synthesis of 3-hexylnonyl 6-(4-(decyloxy)-4-oxobutyl)(((S) 2-hydroxypropyl)amino)hexanoate (YK-117)

According to the method for preparing YK-101, decyl 4-(((S)2-hydroxypropyl)amino)butyrate (151mg, 0.5mmol) and INT-2(205mg, 0.51mmol) were used as raw materials to give the target compound (50mg, 0.08mmol, 16.0%). C₃₈H₇₅NO₅, MS(ES): m/z(M+H⁺)626.6.

¹H NMR (400 MHz, CDCl₃) δ 4.12 - 4.01 (m, 4H), 3.75 - 3.60 (m, 1H), 2.89 - 2.45 (m, 6H), 2.40 - 2.22 (m, 4H), 1.99 - 1.79 (m, 2H), 1.71 - 1.48 (m, 8H), 1.44 - 1.20 (m, 38H), 1.13 (dd, *J* = 18.6, 7.4 Hz, 3H), 0.87 (t, *J =* 6.7 Hz, 9H).

### 20. Synthesis of 3-hexylnonyl 6-(4-(decyloxy)-4-oxobutyl)((3-hydroxybutyl)amino)hexanoate (YK-118)

The synthesis route is as follows:

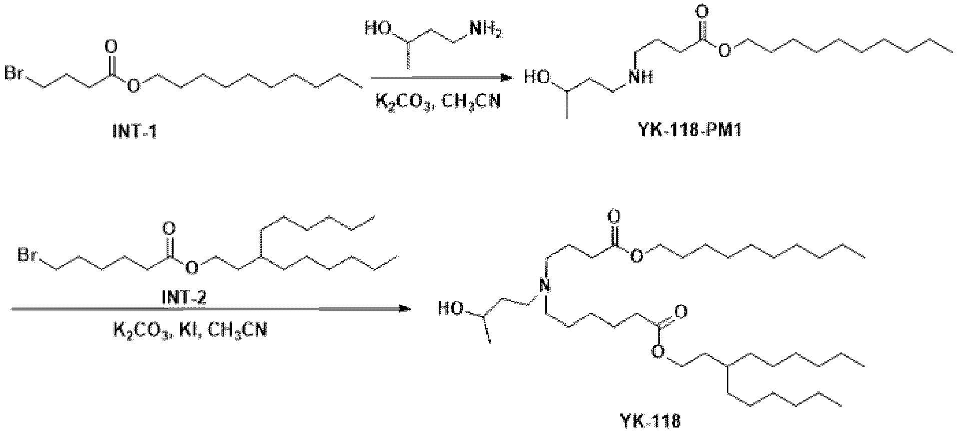

### Step 1: Synthesis of decyl 4-((3-hydroxybutyl)amino)butyrate (YK-118-PM1)

According to the method for synthesizing YK-101-PM2, INT-1(300mg, 0.98mmol) and 4-aminobutan-2-ol (96.2mg, 1.08mmol) were used as raw materials to give decyl 4-((3-hydroxybutyl)amino)butyrate (225mg, 0.71mmol, 72.8%). C₁₈H₃₇NO₃, MS(ES): m/z(M+H⁺)316.4.

### Step 2: Synthesis of 3-hexylnonyl 6-(4-(decyloxy)-4-oxobutyl)((3-hydroxybutyl)amino)hexanoate (YK-118)

According to the method for preparing YK-101, decyl 4-((3-hydroxybutyl)amino)butyrate (200mg, 0.63mmol) and 3-hexylnonyl 6-bromohexanoate (458mg, 1.13mmol) were used as raw materials to give the target compound (80mg, 0.12mmol, 19.8%). C₃₉H₇₇NO₅, MS(ES): m/z(M+H⁺)640.5.

¹H NMR (400 MHz, CDCl₃) δ 4.08 (td, *J =* 7.0, 3.2 Hz, 4H), 4.02 - 3.86 (m, 2H), 3.49 (s, 2H), 3.14 (ddd, *J =* 11.0, 8.1, 2.4 Hz, 2H), 3.07 - 2.87 (m, 4H), 2.41 (t, *J =* 6.7 Hz, 2H), 2.31 (t, *J* = 7.3 Hz, 2H), 2.10 - 1.92 (m, 2H), 1.89 - 1.52 (m, 8H), 1.47 - 1.08 (m, 40H), 0.88 (t, *J* = 6.8 Hz, 9H).

### 21. Synthesis of 3-hexylnonyl 6-(4-(decyloxy)-4-oxobutyl)((3-hydroxy-3-methylbutyl)amino)hexanoate (YK-119)

The synthesis route is as follows:

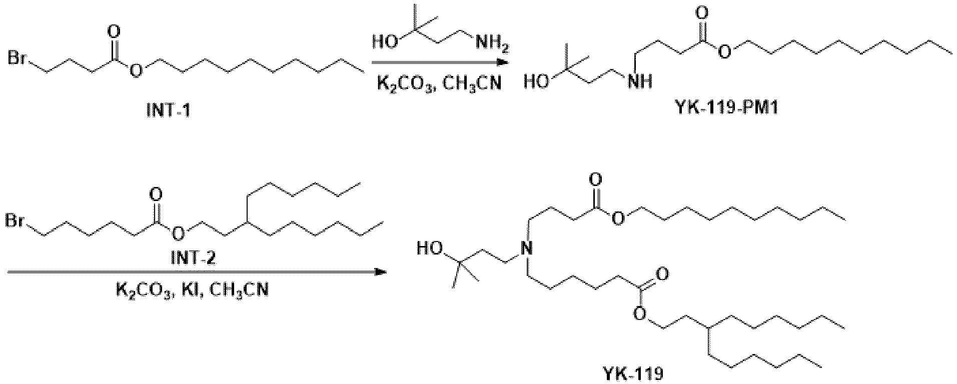

### Step 1: Synthesis of decyl 4-((3-hydroxy-3-methylbutyl)amino)butyrate (YK-119-PM1)

According to the method for synthesizing YK-101-PM2, INT-1(596mg, 1.94mmol) and 4-amino-2-methylbutanol (200mg, 1.94mmol) were used as raw materials to give decyl 4-((3-hydroxy-3-methylbutyl)amino)butyrate (317mg, 0.96mmol, 49.6%). C₁₉H₃₉NO₃, MS(ES): m/z(M+H⁺)330.7.

### Step 2: Synthesis of 3-hexylnonyl 6-(4-(decyloxy)-4-oxobutyl)((3-hydroxy-3-methylbutyl)amino)hexanoate (YK-119)

According to the method for preparing YK-101, decyl 4-((3-hydroxy-3-methylbutyl)amino)butyrate (200mg, 0.61mmol) and 3-hexylnonyl 6-bromohexanoate (492mg, 1.21mmol) were used as raw materials to give the target compound (200mg, 0.31mmol, 50.1%). C₄₀H₇₉NO₅, MS(ES): m/z(M+H⁺)654.4.

¹H NMR (400 MHz, CDCl₃) δ 4.08 (q, *J =* 7.1 Hz, 4H), 2.76 (s, 2H), 2.54 (s, 3H), 2.32 (dt, *J* = 15.0, 7.4 Hz, 4H), 1.85 (s, 2H), 1.72 - 1.45 (m, 10H), 1.45 - 1.02 (m, 44H), 0.89 (t, *J* = 6.8 Hz, 9H).

### 22. Synthesis of 3-hexylnonyl 6-(4-(decyloxy)-4-oxobutyl)((2-hydroxycyclopentyl)amino)hexanoate (YK-120)

The synthesis route is as follows:

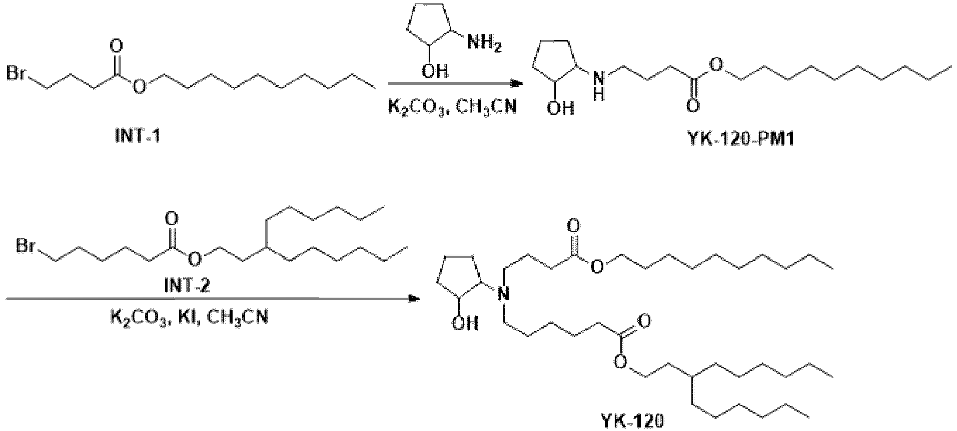

### Step 1: Synthesis of decyl 4-((2-hydroxycyclopentyl)amino)butyrate (YK-120-PM1)

According to the method for synthesizing YK-101-PM2, INT-1(909mg, 2.96mmol) and 2-aminocyclopentan-1-ol (300mg, 2.97mmol) were used as raw materials to give decyl 4-((2-hydroxycyclopentyl)amino)butyrate (580mg, 1.77mmol, 59.8%). C₁₉H₃₇NO₃, MS(ES): m/z(M+H⁺)328.3.

### Step 2: Synthesis of 3-hexylnonyl 6-(4-(decyloxy)-4-oxobutyl)((2-hydroxycyclopentyl)amino)hexanoate (YK-120)

According to the method for preparing YK-101, decyl 4-((2-hydroxycyclopentyl)amino)butyrate (150mg, 0.46mmol) and 3-hexylnonyl 6-bromohexanoate (186mg, 0.46mmol) were used as raw materials to give the target compound (160mg, 0.24mmol, 53.34%). C₄₀H₇₇NO₅, MS(ES): m/z(M+H⁺)652.8.

¹H NMR (400 MHz, CDCl₃) δ 4.30 - 4.15 (m, 1H), 4.15 - 3.86 (m, 4H), 3.61 - 3.30 (m, 1H), 3.10 (s, 1H), 2.70 (d, *J* = 37.2 Hz, 4H), 2.51 - 2.18 (m, 4H), 2.18 - 1.82 (m, 4H), 1.81 - 1.50 (m, 12H), 1.49 - 1.02 (m, 37H), 1.01 - 0.74 (m, 9H).

### 23. Synthesis of 3-hexylnonyl 6-(4-(decyloxy)-4-oxobutyl)(2-(2-(2-hydroxyethoxy)ethoxy-ethyl)amino)hexanoate (YK-121)

The synthesis route is as follows:

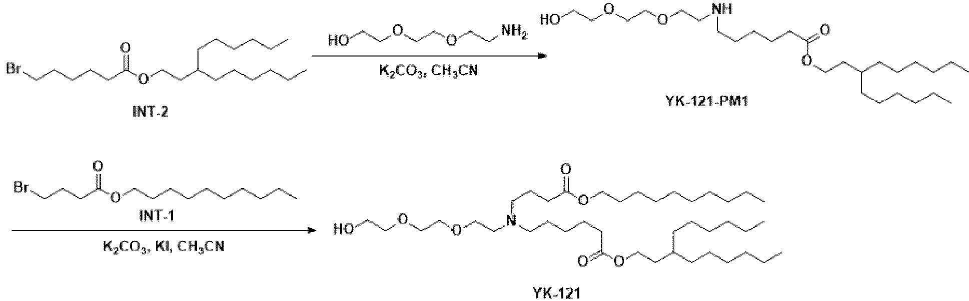

### Step 1: Synthesis of 3-hexylnonyl 6-(2-(2-(2-hydroxyethoxy)ethoxy-ethyl)amino)hexanoate (YK-121-PM1)

According to the method for synthesizing YK-101-PM2, INT-2(500mg, 1.23mmol) and 2-(2-(2-aminoethoxy-ethyl)ethoxy)ethan-1-ol (552mg, 3.70mmol) were used as raw materials to give 3-hexylnonyl 6-(2-(2-(2-hydroxyethoxy)ethoxy-ethyl)amino)hexanoate (519mg, 1.10mmol, 89.1%). C₂₇H₅₅NO₅, MS(ES): m/z(M+H⁺)474.7.

### Step 2: Synthesis of 3-hexylnonyl 6-(4-(decyloxy)-4-oxobutyl)(2-(2-(2-hydroxyethoxy)ethoxy-ethyl)amino)hexanoate (YK-121)

According to the method for preparing YK-101, 3-hexylnonyl 6-(2-(2-(2-hydroxyethoxy)ethoxy-ethyl)amino)hexanoate (100mg, 0.21mmol) and INT-1 (117mg, 0.38mmol) were used as raw materials to give the target compound (72mg, 0.10mmol, 49.0%). C₄₁H₈₁NO₇, MS(ES): m/z(M+H⁺)700.5.

¹H NMR (400 MHz, CDCl₃) δ 5.34 (s, 1H), 4.11 (td, *J* = 7.0, 2.7 Hz, 4H), 3.87 - 3.75 (m, 2H), 3.77 - 3.66 (m, 4H), 3.67 - 3.54 (m, 2H), 3.39 - 2.89 (m, 6H), 2.48 (t, *J* = 6.6 Hz, 2H), 2.35 (t, *J* = 7.3 Hz, 2H), 2.15 (s, 2H), 1.90 (s, 3H), 1.80 - 1.51 (m, 7H), 1.51 - 1.15 (m, 37H), 0.92 (t, *J =* 6.8 Hz, 9H).

### 24. Synthesis of 2-octyldecyl 6-(4-(decyloxy)-4-oxobutyl)(2-(2-(2-hydroxyethoxy)ethoxy-ethyl)amino)hexanoate (YK-122)

The synthesis route is as follows:

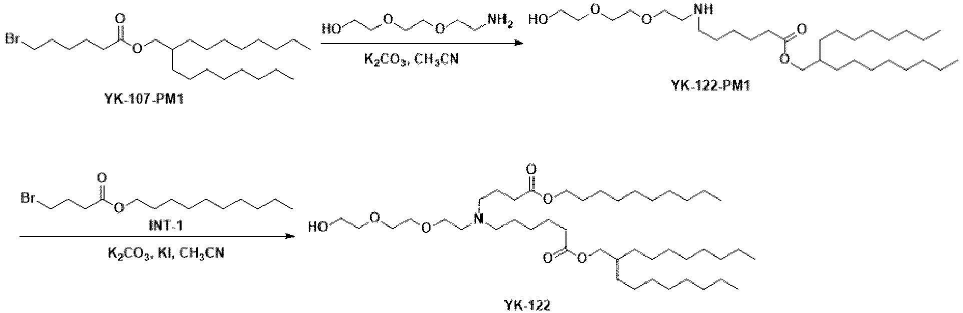

### Step 1: Synthesis of 2-octyldecyl 6-(2-(2-(2-hydroxyethoxy)ethoxy-ethyl)amino)hexanoate (YK-122-PM1)

According to the method for synthesizing YK-101-PM2, YK-107-PM1(500mg, 1.12mmol) and 2-(2-(2-aminoethoxy-ethyl)ethoxy)ethan-1-ol (500mg, 3.35mmol) were used as raw materials to give 2-octyldecyl-6-(2-(2-(2-hydroxyethoxy)ethoxy-ethyl)amino)hexanoate 452mg, 0.88mmol, 78.2%. C₃₀H₆₁NO₅, MS(ES): m/z(M+H⁺)516.7.

### Step 2: Synthesis of 2-octyldecyl 6-(4-(decyloxy)-4-oxobutyl)(2-(2-(2-hydroxyethoxy)ethoxy-ethyl)amino)hexanoate (YK-122)

According to the method for preparing YK-101, 2-octyldecyl 6-(2-(2-(2-hydroxyethoxy)ethoxy-ethyl)amino)hexanoate (200mg, 0.39mmol) and INT-1 (214mg, 0.70mmol) were used as raw materials to give the target compound (105mg, 0.14mmol, 36.3%). C₄₄H₈₇NO₇, MS(ES): m/z(M+H⁺)742.6.

¹H NMR (400 MHz, CDCl₃) δ 4.10 (t, *J* = 6.8 Hz, 2H), 4.00 (d, *J* = 5.8 Hz, 2H), 3.84 - 3.73 (m, 2H), 3.73 - 3.55 (m, 6H), 2.74 (d, *J =* 58.3 Hz, 4H), 2.37 (dt, *J =* 17.7, 7.3 Hz, 5H), 2.05 (s, 2H), 1.88 (s, 4H), 1.76 - 1.51 (m, 7H), 1.31 (d, *J* = 6.8 Hz, 44H), 1.00 - 0.81 (m, 9H).

### 25. Synthesis of 3-hexylnonyl 6-(4-(decyloxy)-4-oxobutyl)(2-(2-hydroxyethoxy-ethyl)amino)hexanoate (YK-123)

The synthesis route is as follows:

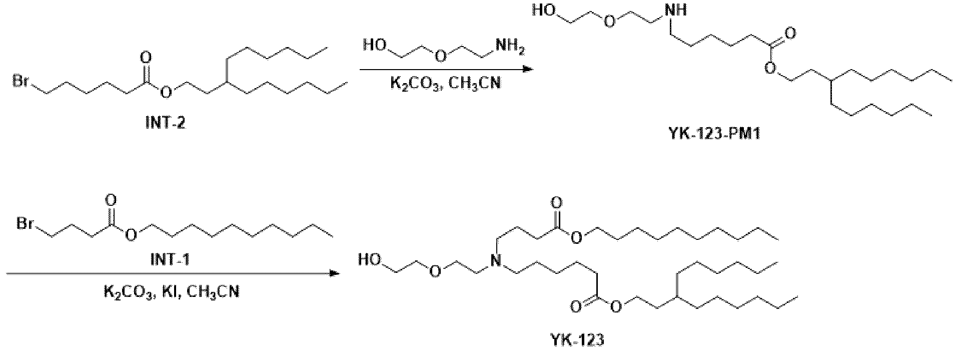

### Step 1: Synthesis of 3-hexylnonyl 6-(2-(2-hydroxyethoxy-ethyl)amino)hexanoate (YK-123-PM1)

According to the method for synthesizing YK-101-PM2, INT-2(500mg, 1.23mmol) and 2-(2-aminoethoxy)ethan-1-ol (338mg, 3.69mmol) were used as raw materials to give 3-hexylnonyl-6-(2-(2-hydroxyethoxy-ethyl)amino)hexanoate 474mg, 1.10mmol, 89.7%. C₂₅H₅₁NO₄, MS(ES): m/z(M+H⁺)430.6.

### Step 2: Synthesis of 3-hexylnonyl 6-(4-(decyloxy)-4-oxobutyl)(2-(2-hydroxyethoxy-ethyl)amino)hexanoate (YK-123)

According to the method for preparing YK-101, 3-hexylnonyl 6-(2-(2-hydroxyethoxy-ethyl)amino)hexanoate (200mg, 0.47mmol) and INT-1 (289mg, 0.94mmol) were used as raw materials to give the target compound (240mg, 0.37mmol, 77.8%). C₃₉H₇₇NO₆, MS(ES): m/z(M+H⁺)656.5.

¹H NMR (400 MHz, CDCl₃) δ 5.34 (s, 1H), 4.11 (dd, *J* = 14.7, 7.1 Hz, 4H), 3.74 (dd, *J =* 5.2, 3.6 Hz, 2H), 3.69 - 3.58 (m, 3H), 2.67 (d, *J =* 59.1 Hz, 5H), 2.35 (dt, *J =* 16.7, 7.4 Hz, 4H), 1.86 (s, 2H), 1.73 - 1.50 (m, 8H), 1.46 - 1.17 (m, 39H), 0.92 (t, *J* = 6.8 Hz, 9H).

### 26. Synthesis of 2-octyldecyl 6-(4-(decyloxy)-4-oxobutyl)(2-(2-hydroxyethoxy-ethyl)amino)hexanoate (YK-124)

The synthesis route is as follows:

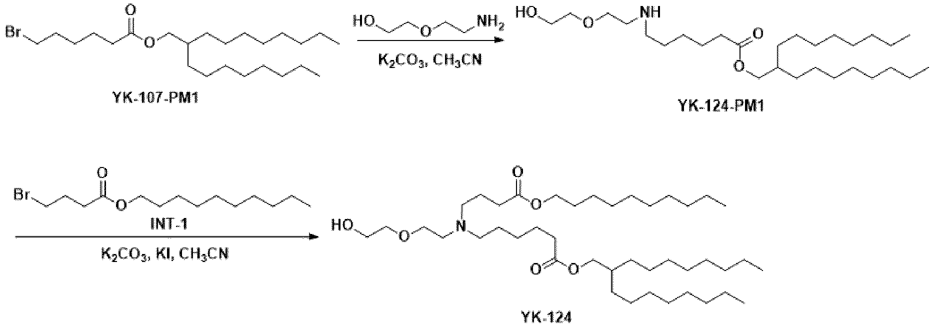

### Step 1: Synthesis of 2-octyldecyl 6-(2-(2-hydroxyethoxy-ethyl)amino)hexanoate (YK-124-PM1)

According to the method for synthesizing YK-101-PM2, YK-107-PM1(260mg, 0.58mmol) and 2- (2-aminoethoxy)ethan-1-ol (183mg, 1.74mmol) were used as raw materials to give 2-octyldecyl-6-(2-(2-hydroxyethoxy-ethyl)amino)hexanoate 132mg, 0.28mmol, 48.2%. C₂₈H₅₇NO₄, MS(ES): m/z(M+H⁺)472.3.

### Step 2: Synthesis of 2-octyldecyl 6-(4-(decyloxy)-4-oxobutyl)(2-(2-hydroxyethoxy-ethyl)amino)hexanoate (YK-124)

According to the method for preparing YK-101, 2-octyldecyl-6-(2-(2-hydroxyethoxy-ethyl)amino)hexanoate (130mg, 0.28mmol) and INT-1(129mg, 0.42mmol) were used as raw materials to give the target compound 109mg, 0.16mmol, 55.8%). C₄₂H₈₃NO₆, MS(ES): m/z(M+H⁺)698.5.

¹H NMR (400 MHz, CDCl₃) δ 3.99 (t, *J* = 6.8 Hz, 2H), 3.89 (d, *J* = 5.8 Hz, 2H), 3.72 - 3.59 (m, 2H), 3.61 - 3.45 (m, 4H), 2.64 (s, 2H), 2.49 (s, 4H), 2.25 (dt, *J* = 11.6, 7.4 Hz, 4H), 1.75 (p, *J* = 7.5 Hz, 2H), 1.67 - 1.34 (m, 7H), 1.34 - 0.98 (m, 45H), 0.90 - 0.68 (m, 9H).

### 27. Synthesis of 3-hexylnonyl 6-(4-(decyloxy)-4-oxobutyl)(2-((4-(dimethylamino)butyryl)oxy)ethyl))amino)hexanoate (YK-125)

The synthesis route is as follows:

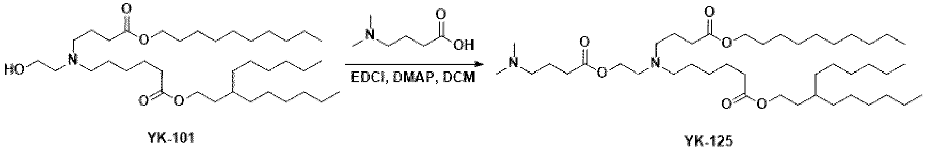

3-Hexylnonyl-6-(4-(decyloxy)-4-oxobutyl)((2-hydroxyethyl)amino)hexanoate (100mg, 0.16mmol) and 4-(dimethylamino)butyric acid (39mg, 0.29mmol) were dissolved in DCM (2mL), and EDCI (98mg, 0.51mmol) and DMAP(20mg, 0.16mmol) were added to the above solution. The mixture was stirred and reacted at 35 °C for 8 hours. After the reaction was completed, the reaction solution was washed with saturated sodium carbonate, washed with saturated brine, and dried over Na₂SO₄. The mixture was filtered and the filtrate was concentrated under reduced pressure in vacuo. The residue was purified by silica gel chromatography (ethyl acetate/n-hexane) to obtain 3-hexylnonyl 6-(4-(decyloxy)-4-oxobutyl)(2-((4-(dimethylamino)butyryl)oxy)ethyl))amino)hexanoate (84mg, 0.11mmol, 72.4%). C₄₃H₈₄N₂O₆, MS(ES): m/z(M+H⁺)725.5.

¹H NMR (400 MHz, CDCl₃) δ 4.23 - 3.90 (m, 6H), 2.70 (t, *J* = 6.3 Hz, 2H), 2.59 - 2.43 (m, 4H), 2.44 - 2.17 (m, 14H), 1.93 - 1.71 (m, 4H), 1.72 - 1.51 (m, 6H), 1.53 - 1.39 (m, 3H), 1.42 - 1.17 (m, 36H), 1.00 - 0.81 (m, 9H).

### 28. Synthesis of 2-octyldecyl 6-(4-(decyloxy)-4-oxobutyl)(2-((4-(dimethylamino)butyryl)oxy)ethyl))amino)hexanoate (YK-126)

The synthesis route is as follows:

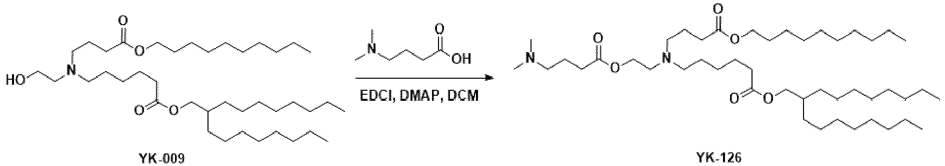

According to the method in CN114044741B, YK -009 270 mg was prepared. According to the method for preparing YK-125, YK-009(120mg, 0.18mmol) and 4-(dimethylamino)butyric acid (29mg, 0.22mmol) were used as raw materials to give the target compound (72mg, 0.09mmol, 52.1%). C₄₆H₉₀N₂O₆, MS(ES): m/z(M+H⁺)767.5.

¹H NMR (400 MHz, CDCl₃) δ 4.00 (dt, *J =* 18.8, 6.5 Hz, 4H), 3.89 (d, *J =* 5.9 Hz, 2H), 2.60 (t, *J* = 6.3 Hz, 2H), 2.46 - 2.33 (m, 4H), 2.32 - 2.09 (m, 13H), 1.70 (dp, *J* = 29.3, 7.4 Hz, 4H), 1.55 (ddd, *J* = 10.1, 7.5, 4.2 Hz, 6H), 1.35 (ddd, *J* = 9.1, 7.2, 5.2 Hz, 3H), 1.31 - 1.00 (m, 45H), 0.91 - 0.68 (m, 9H).

### 29. Synthesis of 9-heptadecyl 8-(8-((3-hexylnonyl)oxy)-8-oxoctyl)-((2-hydroxyethyl)amino)octoate (compound 21)

The synthesis route is as follows:

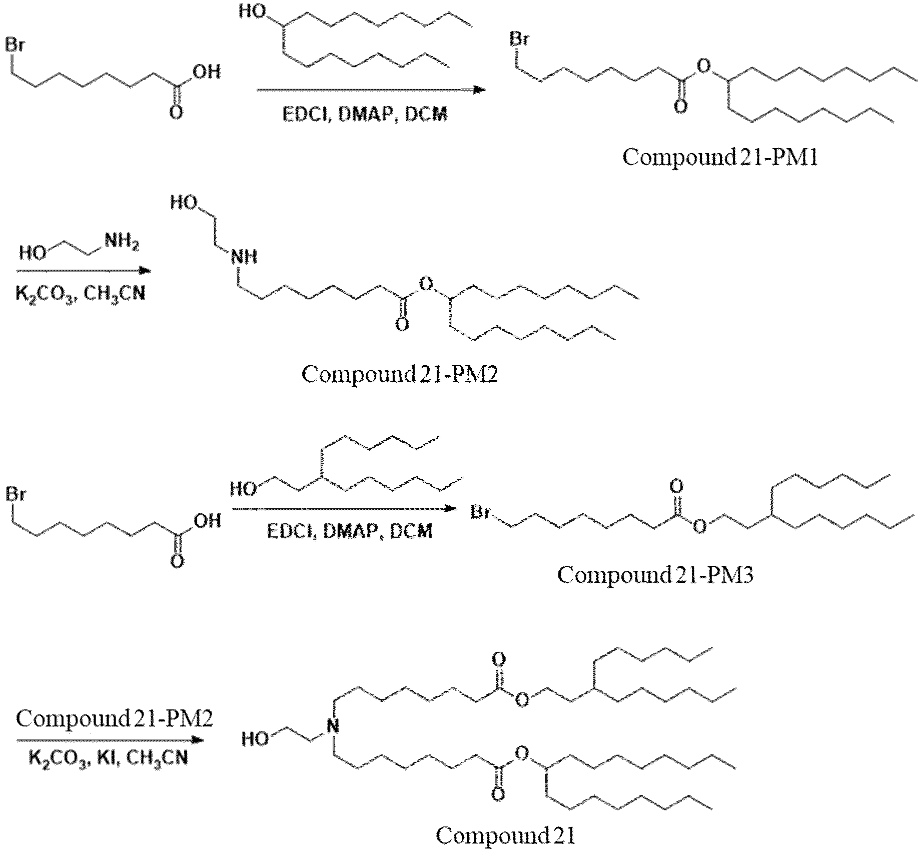

### Step 1: Synthesis of 9-heptadecyl 8-bromooctanoate (compound 21-PM1)

According to the method for preparing INT-1, 9-heptadecanol (1.00g, 3.90mmol) and 8-bromooctanoic acid (1.04g, 4.66mmol) were used as raw materials and subjected to purification by silica gel chromatography (ethyl acetate/n-hexane) to give 9-heptadecyl 8-bromooctanoate (1.20g, 2.60mmol, 66.7%).

### Step 2: Synthesis of 9-heptadecyl 8-((2-hydroxyethyl)amino)octoate (compound 21-PM2)

According to the method for synthesizing YK-101-PM2, 9-heptadecyl 8-bromooctanoate (500mg, 1.08mmol) and ethanolamine (119mg, 3.25mmol) were used as raw materials to give 9-heptadecyl-8-((2-hydroxyethyl)amino)octoate (372mg, 0.84mmol, 78.0%), C₂₇H₅₅NO₃, MS(ES): m/z(M+H⁺)442.3.

### Step 3: Synthesis of 3-hexylnonyl 8-bromooctanoate (compound 21-PM3)

According to the method for preparing INT-1, 3-hexylnonanol (1.00g, 4.38mmol) and 8-bromooctanoic acid (1.17g, 5.25mmol) were used as raw materials and subjected to purification by silica gel chromatography (ethyl acetate/n-hexane) to give 3-hexylnonyl 8-bromooctanoate (1.62g, 3.74mmol, 85.3%).

### Step 4: Synthesis of 9-heptadecyl 8-(8-((3-hexylnonyl)oxy)-8-oxoctyl)-((2-hydroxyethyl)amino)octoate (compound 21)

According to the method for preparing YK-101, 9-heptadecyl 8-((2-hydroxyethyl)amino)octoate (200mg, 0.46mmol) and 3-hexylnonyl 8-bromooctanoate (336mg, 0.82mmol) were used as raw materials to give the target compound (213mg, 0.27mmol, 58.4%). C₅₀H₉₉NO₅, MS(ES): m/z(M+H⁺)794.8.

¹H NMR (400 MHz, CDCl₃) δ 4.90 (p, *J =* 6.3 Hz, 1H), 4.21 - 4.02 (m, 2H), 3.66 (s, 2H), 2.73 (s, 2H), 2.60 (s, 4H), 2.43 - 2.20 (m, 4H), 2.12 - 1.99 (m, 1H), 1.75 - 1.49 (m, 13H), 1.48 - 1.39 (m, 2H), 1.42 - 1.15 (m, 56H), 0.92 (td, *J =* 6.8, 2.2 Hz, 12H).

### 30. Synthesis of bis(3-hexylnonyl)-8, 8'-((2-hydroxyethyl)azadialkyl)dioctanoate (compound 23)

The synthesis route is as follows:

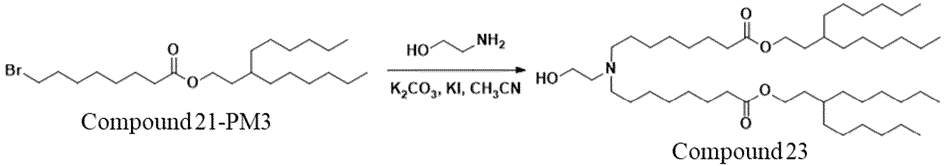

3-Hexylnonyl 8-bromooctanoate (710mg, 1.64mmol) and ethanolamine (40mg, 0.66mmol) were dissolved in acetonitrile (10mL). Potassium carbonate (1.09g, 7.92mmol) and potassium iodide (66mg, 0.39mmol) were added to the above system and the mixture was heated to 70 °C and stirred to react for 20 hours. After the reaction was completed, the reaction solution was cooled to room temperature and then filtered. The filtrate was concentrated at reduced pressure to remove the solvent. The residue was purified by silica gel chromatography (methanol / dichloromethane) to obtain bis(3-hexylnonyl)-8, 8'-((2-hydroxyethyl)azadialkyl)dioctanoate (150mg, 0.20mmol, 29.7%), C₄₈H₉₅NO₅, MS(ES): m/z(M+H⁺)766.5.

¹H NMR (400 MHz, CDCl₃) δ 4.12 (t, *J* = 7.1 Hz, 4H), 3.62 (s, 2H), 2.68 (s, 2H), 2.51 (d, *J* = 25.8 Hz, 4H), 2.32 (t, *J* = 7.5 Hz, 4H), 1.72 - 1.57 (m, 8H), 1.55 - 1.40 (m, 6H), 1.40 - 1.17 (m, 55H), 0.92 (t, *J* = 6.8 Hz, 12H).

### Example 2: Optimization of preparation conditions for lipid nanoparticles (LNP formulations)

### 1. Optimization of the ratio of carrier (liposome) to mRNA

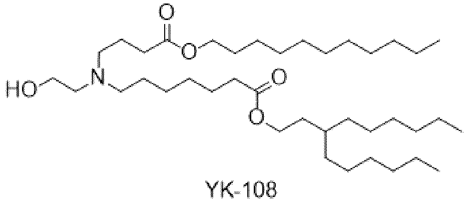

The cationic lipid compound YK-108 synthesized in Example 1 was dissolved in ethanol with DSPC (AVT (Shanghai) Pharmaceutical Technology Co., Ltd.), cholesterol (AVT (Shanghai) Pharmaceutical Technology Co., Ltd.) and DMG-PEG2000 according to a molar ratio of 49: 10: 39.5: 1.5, respectively, to prepare a lipid solution in ethanol. The lipid solution in ethanol was quickly added to citrate buffer (pH=4 to 5) by ethanol injection method, and vortexed for 30s for later use. eGFP-mRNA was diluted in citrate buffer (pH=4 to 5) to give an aqueous mRNA solution. A certain volume of liposome solution and aqueous mRNA solution were used to prepare liposomes at weight ratios of total lipids to mRNA of 5:1, 10:1, 15:1,20:1, 30:1 and 35:1, respectively. The mixtures were sonicated at 25°C for 15min (with an ultrasonic frequency of 40kHz and an ultrasonic power of 800W). The resulting liposomes were diluted to 10 times volume with PBS, and then ultrafiltered with a 300KDa ultrafiltration tube to remove ethanol. Then the volume was fixed to a certain volume with PBS to give an LNP formulation encapsulating eGFP-mRNA using cationic lipid YK-108/DSPC/ cholesterol /DMG-PEG2000 (49:10:39.5:1.5 in molar percentage).

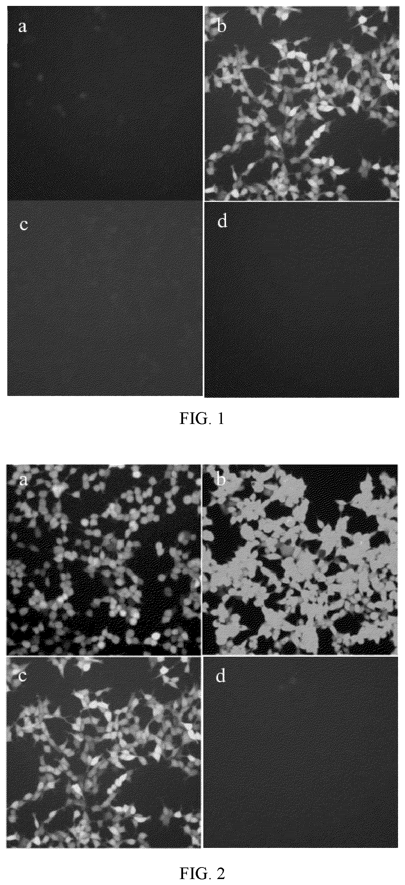

The results of the cell transfection test showed that when the weight ratio of carrier to mRNA was in the range of 10:1 to 30:1, all the transfection effects were good, wherein the ratio of 15:1 had the best transfection effect, and when the weight ratio of carrier to mRNA was 5:1 and 35:1, the transfection effect was poor, which could not be used to carry mRNA. (Fig. 1)

The same results were obtained with LNP formulations prepared from YK-101 and YK-107, which are not shown in the figures.

### 2. Optimization of the ratio of cationic lipid to neutral lipid

The LNP formulation encapsulating eGFP-mRNA was prepared according to the method in 1, wherein the molar ratios of cationic lipid YK-108 and neutral lipid DSPC were 1:1, 3:1, 4:1, 4.9:1, 10:1, 15:1 and 20: 1, respectively.

It can be seen from the cell transfection test that when the molar ratio of cationic lipid to neutral lipid was 1:1 to 15:1, the transfection effect could be achieved, wherein the transfection efficiency was the highest at the molar ratio of cationic lipid to neutral lipid of 4:1, and the ratios of 3:1 and 4.9:1 also had good transfection effect. (FIG. 2)

The same results were obtained with LNP formulations prepared from YK-101 and YK-107, which are not shown in the figures.

### 3. Optimization of the ratio of polymer-conjugated lipid to carrier (liposome)

The LNP formulation encapsulating eGFP-mRNA was prepared according to the method in 1, wherein the cationic lipid in the carrier was YK-108, and the molar ratios of the polymer-conjugated lipid DMG-PEG2000 to the carrier were 0.5%, 1.5%, 3.5%, 5%, 10% and 15%, respectively.

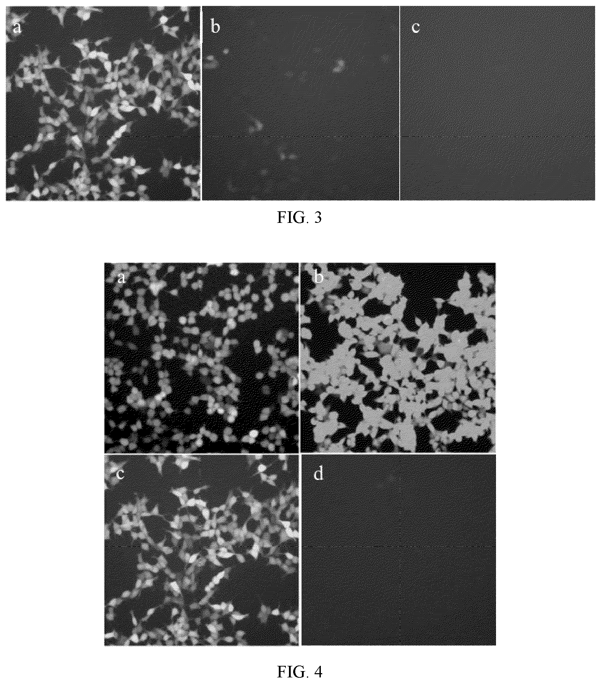

The results of the cell transfection test showed that when the molar ratio of the polymer-conjugated lipid to the carrier was in the range of 0.5% to 10%, the transfection effect were all achieved, wherein the transfection efficiency was the highest when the molar ratio was 1.5%, and the lowest when the molar ratio was 10%. (Fig. 3)

The same results were obtained with LNP formulations prepared from YK-101 and YK-107, which are not shown in the figures.

### 4. Optimization of the proportion of components in the carrier (liposome)

The LNP formulation encapsulating eGFP-mRNA was prepared according to the method in 1, wherein the molar ratios of cationic lipid YK-108, neutral lipid DSPC, structured lipid cholesterol and polymer-conjugated lipid DMG-PEG2000 were 75:5:15:5, 49:10:39.5:1.5, 45:17.5:37:0.5, 40:10:48.5:1.5, 30:10:58.5:1.5 and 25:8:65:2, respectively.

It can be seen from the cell transfection test that transfection was accessible when the molar ratios of cationic lipid, neutral lipid, structured lipid and polymer-conjugated lipid were 75:5:15:5, 49:10:39.5:1.5, 45:17.5:37:0.5, 40:10:48.5:1.5, 30:10:58.5:1.5 and 25:8:65:2, where the transfection effect was the best when the ratio was 40:10:48.5:1.5. Fig. 4 illustrates that it is available to prepare LNP formulations when the molar ratio of cationic lipid, neutral lipid, structured lipid and polymer conjugated lipid is within the range of (25 to 75):(5 to 25):(15 to 65):(0.5 to 10), and the preferred ratio is (35 to 49):(7.5 to 15):(35 to 55):(1 to 5), among which the optimal ratio is 40:10:48.5:1.5.

The same results were obtained with LNP formulations prepared from YK-101 and YK-107, which are not shown in the figures.

### Examples 3: Cell transfection test of LNP formulation of eGFP-mRNA

Cell recovery and passage: 293T cells were recovered, cultured in a culture dish, and passaged to the required number of cells.

Seeding plate: The cells in the culture dish were digested and counted. Cells were spread in a 96-well plate at 10, 000 cells per well and in a 12-well plate at 150, 000 cells per well. Cells were cultured overnight until the cells adhered to the wall.

Cell transfection test: The LNP formulation containing 1.5 µg of eGFP-mRNA prepared in Example 2 (the cationic lipid in the carrier was YK-108) and the Lipofectamin 3000 formulation of eGFP-mRNA were added respectively to the cell culture medium of the 12-well plate, and further incubated for 24 hours. The transfection efficiency of different samples was then investigated according to the fluorescence intensity by observing with a fluorescence microscope.

According to the results of the test, the preparation conditions of lipid nanoparticles (LNP formulation) were finally determined: the ratio of carrier to mRNA was 15:1; the molar ratio of cationic lipid to neutral lipid was 4.9:1; polymer-conjugated lipid accounted for 1.5% of liposome; the molar ratio of cationic lipid, neutral lipid, structured lipid and polymer-conjugated lipid was 49:10:39.5:1.5. At this ratio, various cationic lipids designed in this application and the cationic lipids used in the prior art had good transfection effects (determined by Example 2, some test results are not shown). This condition was used to prepare lipid nanoparticles (LNP formulation) in subsequent tests.

### Example 4: Preparation of lipid nanoparticles (LNP formulation)

**Table 1: The structure of cationic lipids**

| **Name** | **Structure** | **Remark** |
|---|---|---|
| YK-101 | 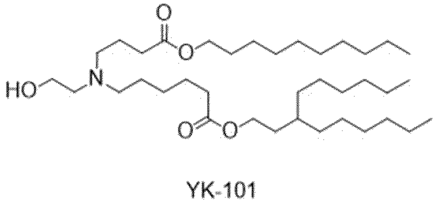 | Synthesized in Example 1 |
| YK-102 | 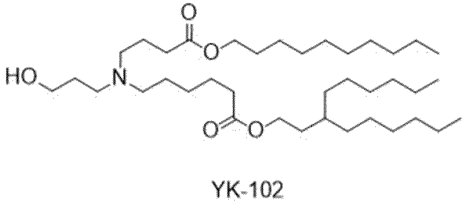 | Synthesized in Example 1 |
| YK-103 | 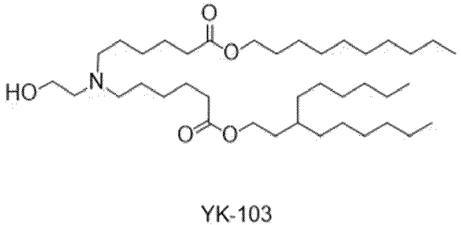 | Synthesized in Example 1 |
| YK-104 | 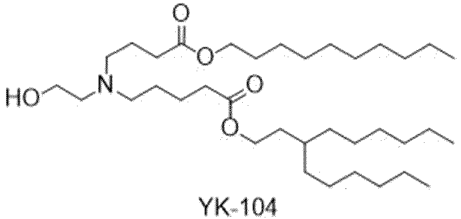 | Synthesized in Example 1 |
| YK-105 | 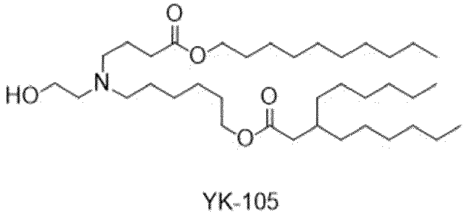 | Synthesized in Example 1 |
| YK-106 | 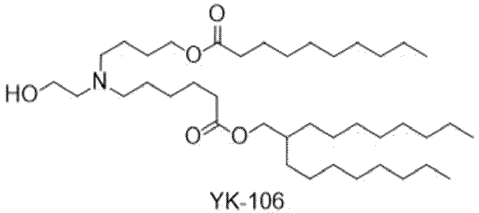 | Synthesized in Example 1 |
| YK-107 | 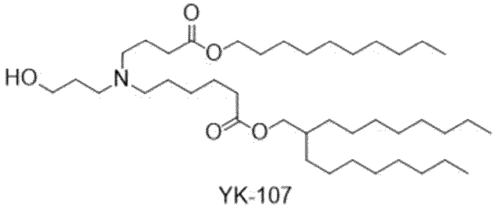 | Synthesized in Example 1 |
| YK-108 | 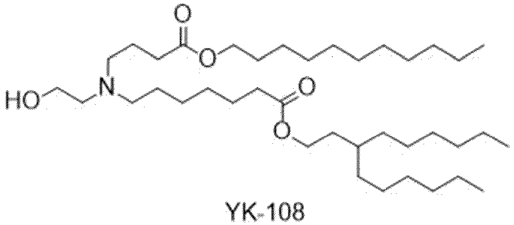 | Synthesized in Example 1 |
| YK-109 | 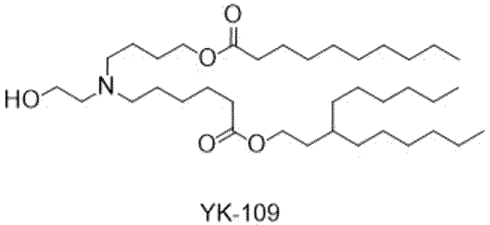 | Synthesized in Example 1 |
| YK-110 | 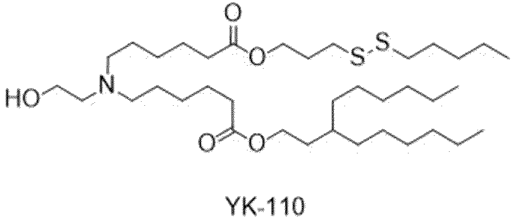 | Synthesized in Example 1 |
| YK-111 | 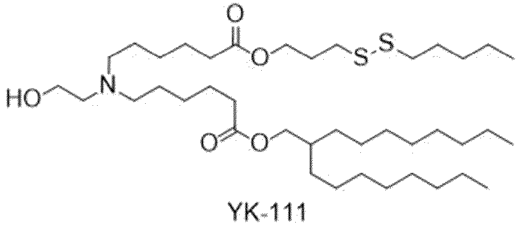 | Synthesized in Example 1 |
| YK- 112 | 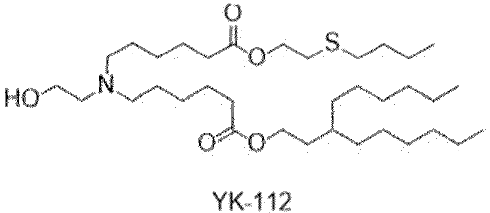 | Synthesized in Example 1 |
| YK-113 | 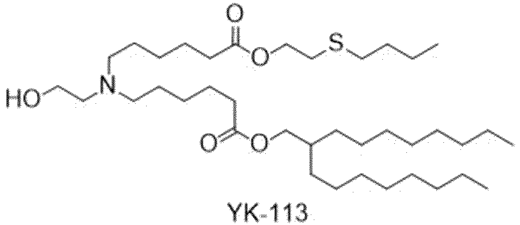 | Synthesized in Example 1 |
| YK-114 | 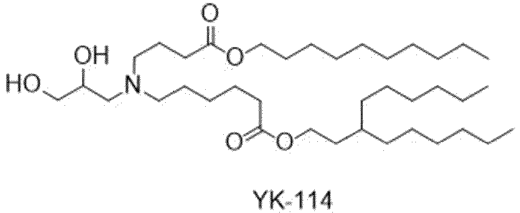 | Synthesized in Example 1 |
| YK-115 | 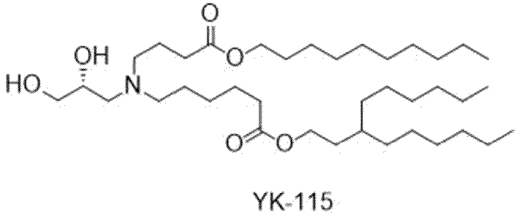 | Synthesized in Example 1 |
| YK-116 | 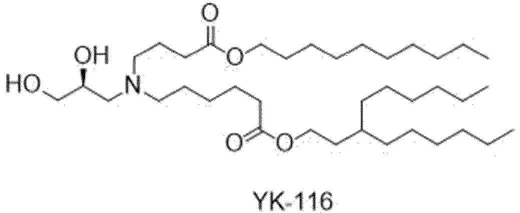 | Synthesized in Example 1 |
| YK-117 | 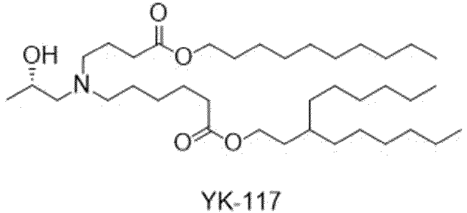 | Synthesized in Example 1 |
| YK-118 | 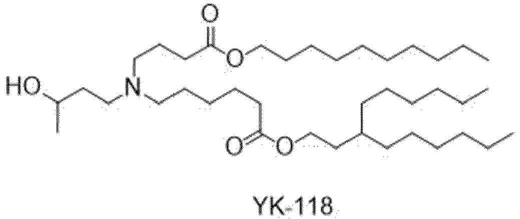 | Synthesized in Example 1 |
| YK-119 | 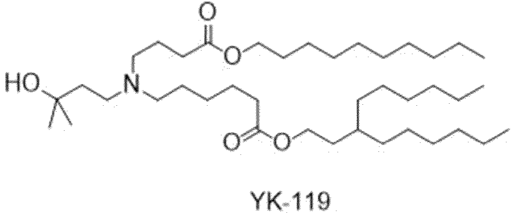 | Synthesized in Example 1 |
| YK-120 | 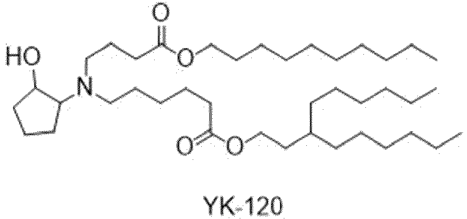 | Synthesized in Example 1 |
| YK-121 | 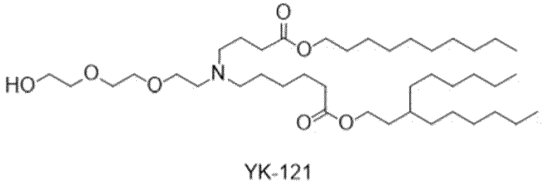 | Synthesized in Example 1 |
| YK-122 | 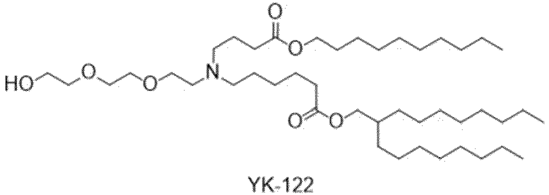 | Synthesized in Example 1 |
| YK-123 | 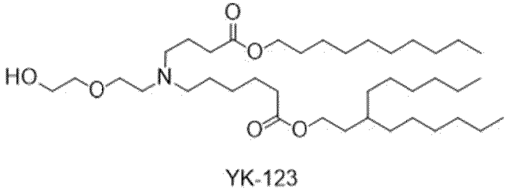 | Synthesized in Example 1 |
| YK-124 | 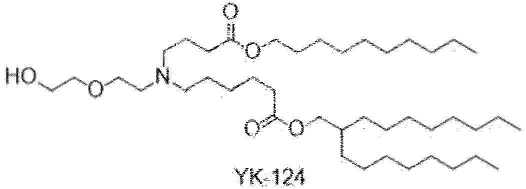 | Synthesized in Example 1 |
| YK-125 | 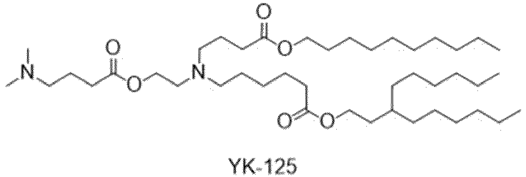 | Synthesized in Example 1 |
| YK-126 | 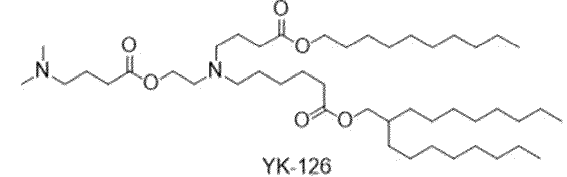 | Synthesized in Example 1 |
| YK-009 | 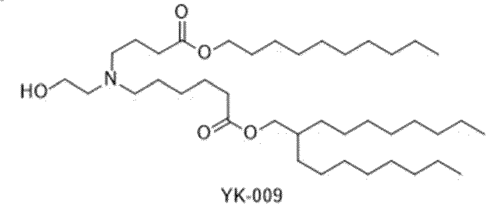 | Synthesized in Example 1, which is YK-009 in CN114044741B |
| Compound 21 | 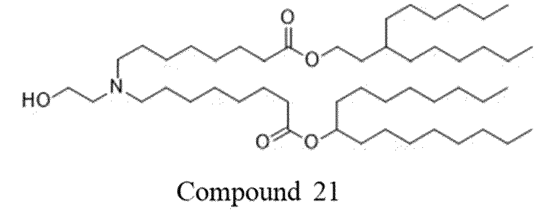 | Synthesized in Example 1, which is compound 21 in WO2021055833A1 |
| Compound 23 | 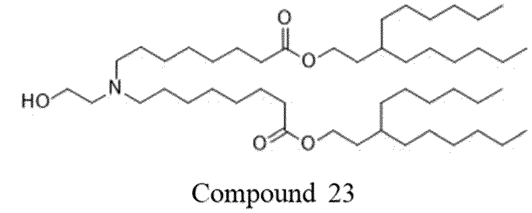 | Synthesized in Example 1, which is compound 23 in WO2021055833A1 |
| SM-102 | 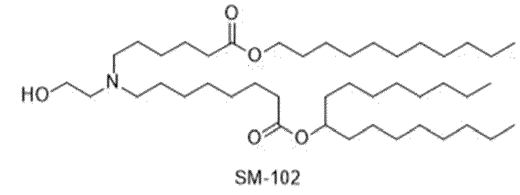 | Purchased from XiaMen Sinopeg Biotech Co., LTD, which is compound 25 in WO2017049245A2 |
| ALC-0315 | 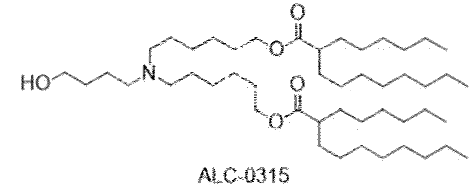 | Purchased from XiaMen Sinopeg Biotech Co., LTD which is compound 3 in CN108368028B |
| HHMA | 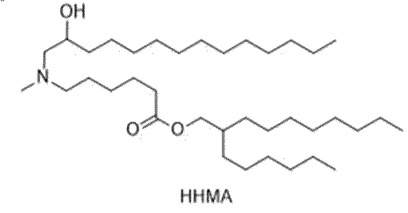 | Purchased from XiaMen Sinopeg Biotech Co., LTD which is compound 1 in CN112979483B |

The cationic lipids listed in Table 1 were dissolved in ethanol with DSPC (AVT (Shanghai) Pharmaceutical Technology Co., Ltd.), cholesterol (AVT (Shanghai) Pharmaceutical Technology Co., Ltd.) and DMG-PEG2000 according to a molar ratio of 49:10:39.5:1.5, respectively, to prepare lipid solution in ethanol. The lipid solution in ethanol was quickly added to citrate buffer (pH=4 to 5) by ethanol injection method, and vortexed for 30s for later use. eGFP-mRNA (from Shanghai Biohub International Trade Co., Ltd) or Fluc-mRNA (from Shanghai Biohub International Trade Co., Ltd) was diluted in citrate buffer (pH=4 to 5) to give an aqueous mRNA solution. A certain volume of liposome solution and mRNA aqueous solution were used to prepare liposomes at a weight ratio of total lipids to mRNA of 15:1. The mixtures were sonicated at 25°C for 15min (with an ultrasonic frequency of 40kHz and an ultrasonic power of 800W). The resulting liposomes were diluted to 10 times volume with PBS, and then ultrafiltered with a 300KDa ultrafiltration tube to remove ethanol. Then the volume was fixed to a certain volume with PBS to give an LNP formulation encapsulating eGFP-mRNA or Fluc-mRNA using cationic lipid/DSPC/cholesterol/DMG-PEG2000 (49:10:39.5:1.5 in molar percentage).

Lipofectamine 3000 transfection reagent is currently widely used for cell transfection. It has very good transfection performance and excellent transfection efficiency, which can improve cell activity, and is suitable for difficult-to-transfect cell types. We selected Lipofectamine 3000 transfection reagent as a control, and prepared Lipofectamin 3000 formulations of eGFP-mRNA or Fluc-mRNA according to the instructions of Lipofectamine 3000 (Invitrogen (Shanghai) Trading Co., Ltd.).

### Example 5: Determination of particle size and polydispersity index (PDI) of lipid nanoparticles

The particle size and polydispersity index (PDI) were determined by dynamic light scattering using Malvern laser particle size analyzer.

10 µL of the liposome solution was weighed, diluted to 1 mL with RNase-free deionized water, and added to the sample pool. Each sample was measured in triplicate. The measurement conditions were: 90° scattering angle, and 25 °C. The test results were as follows:

**Table 2: Particle size and polydispersity index (PDI)**

| **Name** | **Particle size (nm)** | **PDI(%)** |
|---|---|---|
| YK-101 | 191 | 22.4 |
| YK-102 | 166 | 19.9 |
| YK-103 | 179 | 21.5 |
| YK-104 | 200 | 26.9 |
| YK-105 | 109 | 18.6 |
| YK-106 | 140 | 14.4 |
| YK-107 | 96 | 22.8 |
| YK-108 | 164 | 26.5 |
| YK-109 | 205 | 28.2 |
| YK-110 | 150 | 24.9 |
| YK-111 | 144 | 14.1 |
| YK-112 | 147 | 23.1 |
| YK-113 | 147 | 10.4 |
| YK-114 | 112 | 29.5 |
| YK-115 | 167 | 21.8 |
| YK-116 | 143 | 14.3 |
| YK-117 | 187 | 19.2 |
| YK-118 | 187 | 23.2 |
| YK-119 | 98 | 41.3 |
| YK-120 | 142 | 29.7 |
| YK-121 | 192 | 25.1 |
| YK-122 | 205 | 7.7 |
| YK-123 | 246 | 15.4 |
| YK-124 | 216 | 14.7 |
| YK-125 | 220 | 26.4 |
| YK-126 | 260 | 15.1 |
| YK-009 | 205 | 12.1 |
| SM-102 | 142 | 17.4 |
| ALC-0315 | 209 | 15.0 |
| **Compound 21** | 151 | 12.5 |
| **Compound 23** | 140 | 7.7 |
| HMMA | 138 | 12.2 |

The particle size of the nanolipid particles prepared in Example 4 was between 90 and 260 nm, and they can all be used to deliver mRNA. Among them, the particles prepared from YK-107 and YK-119 had the smallest particle size, which were 96nm and 98nm, respectively, and the particles prepared from YK-123 and YK-126 had the largest particle size, which were 246nm and 260nm, respectively. The polydispersity index of all nanolipid particles ranges from 7.7% to 41.3%, among which the smallest was 7.7% for both compound 23 and YK-122, and the largest was 41.3% for YK-119.

### Example 6: In vitro verification of the performance of LNP delivery carriers

Cell recovery and passage: the method was the same as in Example 3.

Seeding plate: the method was the same as in Example 3.

### 1. Fluorescent detection of Fluc-mRNA

An LNP formulation containing 0.3 µg of Fluc-mRNA (the carrier components of the LNP formulation were cationic lipid, neutral lipid, structured lipid and polymer-conjugated lipid with a molar ratio of 49:10:39.5:1.5, wherein the cationic lipid was listed in Table 1) was added to the cell culture medium of a 96-well plate, and further incubated for 24 hours. The corresponding reagent was added according to the instructions of the Gaussia Luciferase Assay Kit, and the fluorescence expression intensity of each well was detected by an IVIS fluorescence detection system. This test verified the transfection efficiencies of LNP formulations in cells, see Tables 3-6 for specific test results.

### Assay results:

**(1) Among a series of designed compounds, the LNP formulations prepared from YK-101, YK-107 and YK-108 had the best cell transfection activities which were significantly improved compared with similar or used cationic lipids in the prior art. For example, the activities of them can be up to 16 times that of SM-102 and 19 times that of compound 23.**

**Table 3 Fluorescence detection results of Fluc-mRNA - 1**

| **Seri al No.** | **Name** | **Relative light unit (RLU)** | **Ratio relative to SM-102** | **Multiples (YK-101)** | **Multiples (YK-107)** | **Multiples (YK-108)** |
|---|---|---|---|---|---|---|
| 1 | YK-101 | 20335193 | 13.25 | 1.00 | 1.10 | 1.21 |
| 2 | YK-107 | 22361210 | 14.58 | 0.91 | 1.00 | 1.10 |
| 3 | YK-108 | 24660507 | 16.07 | 0.82 | 0.91 | 1.00 |
| 4 | YK-009 | 5075954 | 3.31 | 4.01 | 4.41 | 4.86 |
| 5 | SM-102 | 1534210 | 1.00 | 13.25 | 14.58 | 16.07 |
| 6 | ALC-0315 | 2056890 | 1.34 | 9.89 | 10.87 | 11.99 |
| 7 | **Compound 21** | 1389141 | 0,91 | 14.64 | 16.10 | 17.75 |
| 8 | **Compound 23** | 1290824 | 0.84 | 15.75 | 17.32 | 19.10 |
| 9 | HHMA | 2047757 | 1.33 | 9.93 | 10.92 | 12.04 |
| 10 | Lipofectamine 3000 | 1073515 | 0.70 | 18.94 | 20.83 | 22.97 |

### a. Difference in cell transfection activity

Table 3 lists the fluorescence detection results of LNP formulations containing Fluc-mRNA prepared from different cationic lipids. Among them, YK-009 is disclosed in CN114044741B (claim 1), SM-102 is compound 25 disclosed in WO2017049245A2 (page 29 of the specification), ALC-0315 is compound 3 disclosed in CN108368028B (page 24 of the specification), and compound 21 and compound 23 are disclosed in WO2021055833A1 (page 22 of the specification), and HHMA is compound 1 disclosed in CN112979483B (page 12 of the specification). LNPs prepared from these cationic lipids all had good cell transfection effects; Lipofectamine 3000 is currently a widely used cell transfection reagent with good transfection performance.

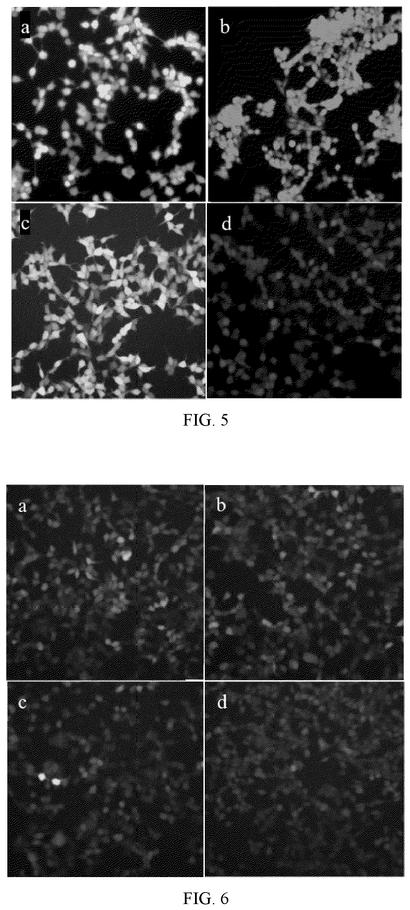

It can be seen from Table 3 and Fig. 5 that the LNP formulations containing Fluc-mRNA prepared from YK-101, YK-107 and YK-108 had the best fluorescence absorptions, with RLU values of 20335193, 22361210 and 24660507 respectively, which can be up to 13.25 times, 14.58 times and 16.07 times of SM-102, 9.89 times, 10.87 times and 11.99 times of ALC-0315, 14.64 times, 16.10 times and 17.75 times that of compound 21, 15.75 times, 17.32 times and 19.10 times that of compound 23, 9.93 times, 10.92 times and 12.04 times that of HHMA, 18.94 times, 20.83 times and 22.97 times that of Lipofectamine 3000, 4.01 times, 4.41 times and 4.86 times that of YK-009.

The data were analyzed using GraphPad Prism software. YK-101, YK-107 and YK-108 were significantly different from SM-102, ALC-0315, compound 21, compound 23, HHMA, Lipofectamine 3000 and YK-009, wherein the transfection efficiencies were significantly improved.

### b. Differences in chemical structure

The chemical structures of YK-101, YK-107 and YK-108 are similar to those of cationic lipids in the prior art, with only a difference of 1-2 C in the G₁, R₁, G₂ or R₂ group.

For example, compared with the chemical structure of SM-102, YK-101 has 2 less C in the G₁ group, 1 less C in the R₁ group, 2 less C in the G₂ group, 2 more C in the single chain of the R₂ group and 2 less C in each single chain of the double chain of the R₂ group, and the other structures are exactly the same; YK-107 has 2 less C in the G₁ group, 1 less C in the R₁ group, 2 less C in the G₂ group, 1 more C in the single chain of the R₂ group, 1 more C in the G₃ group, and the other structures are exactly the same; YK-108 has 2 less C in the G₁ group, 1 less C in the G₂ group, 2 more C in the single chain of the R₂ group and 2 less C in each single chain of the double chain of the R₂ group, and the other structures are exactly the same.

### Brief summary:

Among a series of designed compounds, the LNP formulations prepared from YK-101, YK-107 and YK-108 had the best cell transfection activities which were significantly improved compared with similar or used cationic lipids in the prior art. For example, the activities of them can be up to 16 times that of SM-102 and 19 times that of compound 23. Therefore, compounds with similar chemical structures do not necessarily have similar cell transfection activities, but are very likely to have very significant differences.

**(2) YK-101, YK-107 and YK-108 had the highest cell transfection activities among the compounds with the most similar structures that designed by us, which can be up to 34 times that of other compounds, such as YK-109.**

In order to verify that compounds with similar structures have significantly different activities, we compared the compounds with the closest structures to YK-101, YK-107 and YK-108. The results show that the activities of this series of compounds varied greatly. Among them, YK-101, YK-107 and YK-108 had the highest cell transfection activities, which were 28 times, 31 times and 34 times that of YK-109, which had the lowest activity, indicating significantly improved transfection efficiency.

**Table 4 Fluorescence detection results of Fluc-mRNA - 2**

| **Seri al No.** | **Name** | **Relative light unit (RLU)** | **Ratio relative to SM-102** | **Multiples (YK-101)** | **Multiples (YK-107)** | **Multiples (YK-108)** |
|---|---|---|---|---|---|---|
| 1 | YK-101 | 20335193 | 13.25 | 1.00 | 1.10 | 1.21 |
| 2 | YK-107 | 22361210 | 14.58 | 0.91 | 1.00 | 1.10 |
| 3 | YK-108 | 24660507 | 16.07 | 0.82 | 0.91 | 1.00 |
| 4 | YK-102 | 1464032 | 0.95 | 13.89 | 15.27 | 16.84 |
| 5 | YK-103 | 2312883 | 1.51 | 8.79 | 9.67 | 10.66 |
| 6 | YK-104 | 1693707 | 1.10 | 12.01 | 13.20 | 14.56 |
| 7 | YK-105 | 1593805 | 1.04 | 12.76 | 14.03 | 15.47 |
| 8 | YK-106 | 1628063 | 1.06 | 12.49 | 13.73 | 15.15 |
| 9 | YK-109 | 717431 | 0.47 | 28.34 | 31.17 | 34.37 |
| 10 | SM-102 | 1534210 | 1.00 | 13.25 | 14.58 | 16.07 |
| 11 | ALC-0315 | 2056890 | 1.34 | 9.89 | 10.87 | 11.99 |
| 12 | **Compound 21** | 1389141 | 0.91 | 14.64 | 16.10 | 17.75 |
| 13 | **Compound 23** | 1290824 | 0.84 | 15.75 | 17.32 | 19.10 |
| 14 | HHMA | 2047757 | 1.33 | 9.93 | 10.92 | 12.04 |
| 15 | Lipofectamine 3000 | 1073515 | 0.70 | 18.94 | 20.83 | 22.97 |

### a. Difference in cell transfection activity

As can be seen from Table 4 and Fig. 6, the fluorescence absorption values of LNP formulations prepared from these compounds were very different from those of YK-101, YK-107 and YK-108, wherein YK-101, YK-107 and YK-108 were 13.89 times, 15.27 times and 16.84 times that of YK-102, 8.79 times, 9.67 times and 10.66 times that of YK-103, 12.01 times, 13.20 times and 14.56 times that of YK-104, 12.76 times, 14.03 times and 15.47 times that of YK-105, 12.49 times, 13.73 times and 15.15 times that of YK-106, 28.34 times, 31.17 times and 34.37 times that of YK-109, respectively.

Compounds YK-102, YK-103, YK-104, YK-105, YK-106 and YK-109 also had large differences in activity among them. The fluorescence absorption values of LNP formulations prepared from YK-103, YK-104, YK-105 and YK-106 were 2312883, 1693707, 1593805 and 1628063 respectively, which were slightly better than that of SM-102, and were 1.51 times, 1.10 times, 1.04 times and 1.06 times that of SM-102, respectively; the fluorescence absorption value of YK-102 was 1464032, which was not much different from that of SM-102 and was 0.95 times that of SM-102; the fluorescence absorption value of YK-109 was 717431, which was only 0.47 times that of SM-102. Among this series of compounds, YK-103, which had the highest activity, was 3.2 times that of YK-109, which had the lowest activity.

The data were analyzed using GraphPad Prism software. There is no significant difference between each of YK-102, YK-103, YK-104, YK-105, YK-106 and YK-109 and that of SM-102. YK-101, YK-107 and YK-108 were significantly different from YK-102, YK-103, YK-104, YK-105, YK-106 and YK-109, with significantly improved transfection efficiencies.

### b. Differences in chemical structure

The structures of this series of compounds are very similar, except that individual groups differ by 1-2 C. The structures of YK-101, YK-107 and YK-108 are very similar to those of other compounds; other compounds are also very similar to each other.

For example: Compared with YK-101, YK-104 has only 1 less C in the G₂ group, and the other structures are exactly the same, but the cell transfection activity of YK-101 was 12 times that of YK-104; compared with YK-107, YK-102 only has 1 more C in the single chain of the R₂ group, and 2 less C in each single chain of the double chain of the R₂ group, and the other structures are exactly the same, but the cell transfection activity of YK-107 was 15 times that of YK-102; compared with YK-108, YK-109 only has 1 more C in the G₁ group, 2 less C in the R₁ group, and a different Li group, which is -OC(O)-, and the other structures are exactly the same, but the cell transfection activity of YK-108 was 34 times higher than that of YK-109.

Compared with YK-109, YK-103 only has 1 more C each in G₁ and R₁, and a different L₁, which is -C(O)O-, and the other structures are exactly the same, but the cell transfection activity of YK-103 was 3.2 times higher than that of YK-109.

### Brief summary:

Among a series of compounds with very similar structures we designed, YK-101, YK-107 and YK-108 had the highest cell transfection activities, which were 28 times, 31 times and 34 times higher than YK-109 respectively. There is no correspondence between the structure of a compound and intracellular transfection efficiency. Even a group of compounds with the most similar structures may have very different cell transfection efficiencies. Therefore, it is not easy to screen out cationic lipid compounds with high transfection efficiency from a series of compounds with very similar structures, and multiple designs are required.

**(3) It is very difficult and requires a lot of creative work to screen out compounds with high cell transfection activities.**
**I. YK-101, YK-107 and YK-108 had the highest transfection activities compared to compounds with some minor differences in structure only in the G₁, G₃ or R₁ group. They can be up to 10,000 times higher than other compounds, such as YK-112.**

We further compared the differences in cell transfection activities of YK-101, YK-107 and YK-108 with those of other structurally similar compounds. This series of compounds only has some small differences in the G₁, G₃ or R₁ group such as, 2 C added in the G₁, a side chain group introduced in G₃, or -S- or -S-S- group introduced in R₁. The results showed that the activities of this series of compounds were significantly different from those of YK-101, YK-107 and YK-108. The cell transfection efficiencies of YK-101, YK-107 and YK-108 were significantly higher, which can be up to 10,000 times or more, than those of other compounds.

**Table 5 Fluorescence detection results of Fluc-mRNA - 3**

| **Seri al No.** | **Name** | **Relative light unit (RLU)** | **Ratio relative to SM-102** | **Multiples (YK-101)** | **Multiples (YK-107)** | **Multiples (YK-108)** |
|---|---|---|---|---|---|---|
| 1 | YK-101, | 20335193 | 13.25 | 1.00 | 1.10 | 1.21 |
| 2 | YK-107 | 22361210 | 14.58 | 0.91 | 1.00 | 1.10 |
| 3 | YK-108 | 24660507 | 16.07 | 0.82 | 0.91 | 1.00 |
| 4 | YK-110 | 271783 | 0.18 | 74.82 | 82.28 | 90.74 |
| 5 | YK-111 | 1328301 | 0.87 | 15.31 | 16.83 | 18.57 |
| 6 | YK-112 | 2012 | 0.0013 | 10106.95 | 11113.92 | 12256.71 |
| 7 | YK-113 | 151694 | 0.10 | 134.05 | 147.41 | 162.57 |
| 8 | YK-114 | 1418926 | 0.92 | 14.33 | 15.76 | 17.38 |
| 9 | YK-115 | 1427501 | 0.93 | 14.25 | 15.66 | 17.28 |
| 10 | YK-116 | 1321284 | 0.86 | 15.39 | 16.92 | 18.66 |
| 11 | YK-117 | 695716 | 0.45 | 29.23 | 32.14 | 35.45 |
| 12 | YK-118 | 21310 | 0.0139 | 954.26 | 1049.33 | 1157.23 |
| 13 | YK-119 | 8557 | 0.0056 | 2376.44 | 2613.21 | 2881.91 |
| 14 | YK-120 | 1403572 | 0.91 | 14.49 | 15.93 | 17.57 |
| 15 | SM-102 | 1534210 | 1.00 | 13.25 | 14.58 | 16.07 |
| 16 | ALC-0315 | 2056890 | 1.34 | 9.89 | 10.87 | 11.99 |
| 17 | **Compound 21** | 1389141 | 0.91 | 14.64 | 16.10 | 17.75 |
| 18 | **Compound 23** | 1290824 | 0.84 | 15.75 | 17.32 | 19.10 |
| 19 | HHMA | 2047757 | 1.33 | 9.93 | 10.92 | 12.04 |
| 20 | Lipofectamine 3000 | 1073515 | 0.70 | 18.94 | 20.83 | 22.97 |

### a. Difference in cell transfection activity

Compared with YK-101, YK-107 and YK-108, other compounds only have some minor differences in the G₁, G₃ or R₁ group, but the impact on cell transfection activity is very large, with the highest difference up to more than 10,000 times.

Specifically, it can be seen from Table 5 that the fluorescence absorption values of LNP formulations prepared from YK-111, YK-114, YK-115, YK-116 and YK-120 were similar to that of SM-102, which were 0.87 times, 0.92 times, 0.93 times, 0.86 times and 0.91 times that of SM-102, 0.96 times, 1.02 times, 1.03 times, 0.95 times and 1.01 times that of compound 21, 1.03 times, 1.10 times, 1.11 times, 1.02 times and 1.09 times of that of compound 23, 0.65 times, 0.69 times, 0.70 times, 0.65 times and 0.69 times of HHMA, 1.24 times, 1.32 times, 1.33 times, 1.23 times and 1.31 times that of Lipofectamine 3000, respectively.

The activities of the above five compounds were quite different from those of YK-101, YK-107 and YK-108, wherein YK-101, YK-107 and YK-108 were 15.31 times, 16.83 times and 18.57 times that of YK-111, 14.33 times, 15.76 times and 17.38 times that of YK-114, 14.25 times, 15.66 times and 17.28 times that of YK-115, 15.39 times, 16.92 times and 18.66 times that of YK-116, 14.49 times, 15.93 times and 17.57 times that of YK-120, respectively.

YK-110, YK-113 and YK-117 were quite different from SM-102, which were 0.18 times, 0.10 times and 0.45 times that of SM-102, 0.20 times, 0.11 times and 0.50 times that of compound 21, 0.21 times, 0.12 times and 0.54 times that of compound 23, 0.13 times, 0.07 times and 0.34 times that of HHMA, 0.25 times, 0.14 times and 0.65 times that of Lipofectamine 3000, respectively.

The above three compounds had very different activities from YK-101, YK-107 and YK-108, wherein YK-101, YK-107 and YK-108 were 74.82 times, 82.28 times and 90.74 times that of YK-110, 134.05 times, 147.41 times and 162.57 times that of YK-113, and 29.23 times, 32.14 times and 35.45 times that of YK-117 respectively.

YK-112, YK-118 and YK-119 were very different from SM-102, with significantly reduced activity, which were only 0.0013 times, 0.0139 times and 0.0056 times that of SM-102, 0.0014 times, 0.0153 times and 0.0062 times that of compound 21, 0.0016 times, 0.0165 times and 0.0066 times that of compound 23, 0.0010 times, 0.0104 times and 0.0042 times that of HHMA, 0.0019 times and 0.0199 times and 0.0080 times that of Lipofectamine 3000. The intracellular transfection efficiencies of YK-112, YK-118 and YK-119 were only 1 ‰ - 1 % of that of SM-102.

The above three compounds had very different activities from YK-101, YK-107 and YK-108, wherein YK-101, YK-107 and YK-108 can be up to 10106.95 times, 11113.92 times and 12256.71 times of YK-23, 954.26 times, 1049.33 times and 1157.23 times of YK-118, and 2376.44 times, 2613.21 times and 2881.91 times of YK-119, respectively.

The data were analyzed using GraphPad Prism software. YK-110, YK-112, YK-113, YK-118 and YK-119 were significantly different from SM-102, and YK-101, YK-107 and YK-108 were significantly different from all other compounds. The cell transfection efficiency was significantly improved.

### b. Differences in chemical structure

This series of compounds only has some minor differences in the G₁, G₃ or R₁ groups from YK-101, YK-107 and YK-108, such as 2 C added in G₁ and an introduced side chain group in G₃ or an introduced -S- or -S-S- group in Ri; the structural differences among these compounds are also very small.

For example, compared with YK-101, YK-112 only has 2 more C in G₁ and 4 less C and 1 more S in R₁, but the cell transfection activity of YK-101 can be more than 10,000 times that of YK-112; YK-118 only has a different G₃ group, one more branched methyl group, but the cell transfection activity of YK-101 can be up to 900 times that of YK-118. Compared with YK-107, YK-113 only has 2 more C in G₁, 4 less C and 1 more S in R₁ and one less C in G₃, but the cell transfection activity of YK-107 can be up to 140 times that of YK-113. Compared with YK-108, YK-119 only has one less C in R₁, one less C in G₂, and one more -C(CH₃)₂- group in G₃, but the cell transfection activity of YK019 can be up to 2,800 times that of YK-119.

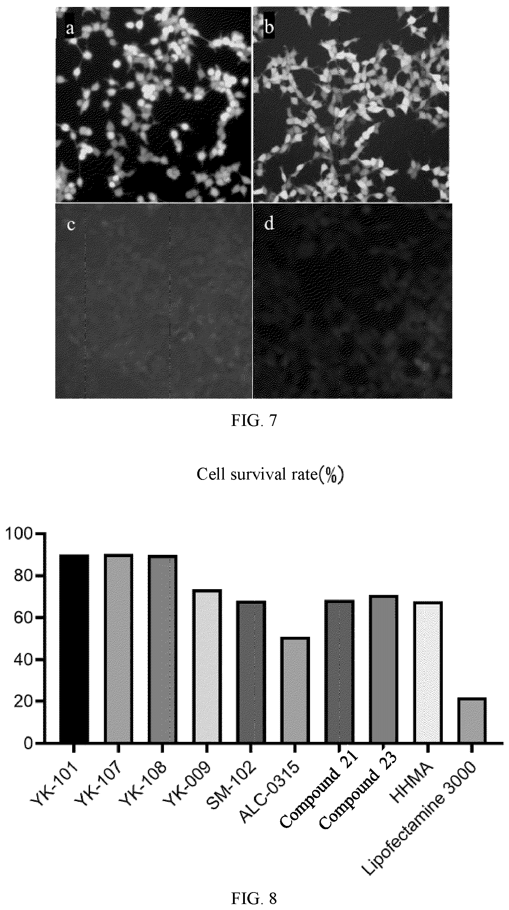

Fig. 7 shows the fluorescence absorption pictures of LNP formulations prepared from YK-101, YK-108, YK-112 and YK-119. It can be seen that compared with YK-101 and YK-108, the fluorescence absorption of YK-112 and YK-119 were very weak.

Among these compounds, YK-110 and YK-111 are only slightly different in the R₂ group. YK-110 has 1 more C in the single chain than YK-111, and has 2 less C in each single chain of the double chain than YK-111, but the cell transfection efficiency of YK-111 was 5 times that of YK-110; YK-112 and YK-113 are only slightly different in the R₂ group. YK-112 has 1 more C in the single chain than YK-113 and 2 less C in each single chain of the double chain than YK-113, but the cell transfection activity of YK-113 can be up to 75 times that of YK-112; YK-117, YK-118 and YK-119 are only slightly different in the G₃ groups, but the cell transfection efficiency of YK-117 was 33 times that of YK-118 and 80 times that of YK-119.

### Brief summary:

Compared with compounds in which two C are added to G₁, a side chain group is introduced in G₃, or -S- or -S-S-groups are introduced in R₁, YK-101, YK-107 and YK-108 had the highest cell transfection activity. For example, YK-101, YK-107 and YK-108 were all more than 10,000 times higher than YK-112, and more than 2,000 times higher than YK-119. Moreover, the activity difference between other compounds with similar structures was also very large. For example, YK-117 was 80 times that of YK-119. It can be seen that small differences in structure can also produce large changes in transfection activity. It is very difficult to screen out compounds with high cell transfection activity, which requires a lot of creative work.

**II. YK-101, YK-107 and YK-108 had the highest transfection activities compared with compounds in which only an ether bond was introduced into the G₃ group or the G₃ group was changed to a 4-(dimethylamino)butyryl group. For example, the activity can be up to more than 300 times higher than that of YK-124.**

The compounds listed in Table 6 differ from YK-101, YK-107 and YK-108 only in that the G₃ group is different, in which an ether bond is introduced or the G₃ group is changed to a 4-(dimethylamino)butyryl group. The cell transfection activity results show that among this series of compounds with similar structures, the cell transfection activities of YK-101, YK-107 and YK-108 were much higher than those of other compounds, which were 240 times, 270 times and 300 times higher than that of YK-124 respectively.

**Table 6 Fluorescence detection results of Fluc-mRNA - 4**

| **Seri al No.** | **Name** | **Relative light unit (RLU)** | **Ratio relative to SM-102** | **Multiples (YK-101)** | **Multiples (YK-107)** | **Multiples (YK-108)** |
|---|---|---|---|---|---|---|
| 1 | YK-101 | 20335193 | 13.25 | 1.00 | 1.10 | 1.21 |
| 2 | YK-107 | 22361210 | 14.58 | 0.91 | 1.00 | 1.10 |
| 3 | YK-108 | 24660507 | 16.07 | 0.82 | 0.91 | 1.00 |
| 4 | YK-121 | 427603 | 0.28 | 47.56 | 52.29 | 57.67 |
| 5 | YK-122 | 1418705 | 0.92 | 14.33 | 15.76 | 17.38 |
| 6 | YK-123 | 807703 | 0.53 | 25.18 | 27.68 | 30.53 |
| 7 | YK-124 | 81596 | 0.05 | 249.22 | 274.05 | 302.23 |
| 8 | YK-125 | 169824 | 0.11 | 119.74 | 131.67 | 145.21 |
| 9 | YK-126 | 219983 | 0.14 | 92.44 | 101.65 | 112.10 |
| 10 | SM-102 | 1534210 | 1.00 | 13.25 | 14.58 | 16.07 |
| 11 | ALC-0315 | 2056890 | 1.34 | 9.89 | 10.87 | 11.99 |
| 12 | **Compound 21** | 1389141 | 0.91 | 14.64 | 16.10 | 17.75 |
| 13 | **Compound 23** | 1290824 | 0.84 | 15.75 | 17.32 | 19.10 |
| 14 | HHMA | 2047757 | 1.33 | 9.93 | 10.92 | 12.04 |
| 15 | Lipofectamine 3000 | 1073515 | 0.70 | 18.94 | 20.83 | 22.97 |

### a. Difference in cell transfection activity

When an ether bond was introduced into the G₃ group of YK-101, YK-107 and YK-108 introduced or the G₃ group was changed to a 4-(dimethylamino)butyryl group, the cell transfection activity was greatly reduced. The activities of YK-101, YK-107 and YK-108 were 240 times, 270 times and 300 times that of YK-124 respectively.

Details are as follows:
As can be seen from Table 6, the fluorescence absorptions of LNP formulations prepared from YK-122 and YK-123 were similar to that of SM-102, which were 0.92 times and 0.53 times that of SM-102, 1.02 times and 0.58 times that of compound 21, 1.10 times and 0.63 times that of compound 23, 0.69 times and 0.39 times that of HHMA, and 1.32 times and 0.75 times that of Lipofectamine 3000, respectively.

The above two compounds were quite different from YK-101, YK-107 and YK-108. The fluorescence absorption values of YK-101, YK-107 and YK-108 were 14.33 times, 15.76 times and 17.38 times that of YK-122, and 25.18 times, 27.68 times and 30.53 times that of YK-123, respectively.

YK-121, YK-125 and YK-126 were quite different from SM-102, which were 0.28 times, 0.11 times and 0.14 times of SM-102, 0.31 times, 0.12 times and 0.16 times of compound 21, 0.33 times, 0.13 times and 0.17 times of compound 23, 0.21 times, 0.08 times and 0.11 times of HHMA, and 0.40 times, 0.16 times and 0.20 times of Lipofectamine 3000, respectively.

The above three compounds were very different from YK-101, YK-107 and YK-108. The fluorescence absorption values of YK-101, YK-107 and YK-108 were 47.56 times, 52.29 times and 57.67 times that of YK-121, 119.74 times, 131.67 times and 145.21 times of YK-125, and 92.44 times, 101.65 times and 112.10 times of YK-126, respectively.

YK-124 had the lowest fluorescence absorption, which was only 0.05 times that of SM-102, 0.06 times that of compound 21, 0.06 times that of compound 23, 0.04 times that of HHMA and 0.08 times that of Lipofectamine 3000. The fluorescence absorption values of YK-101, YK-107 and YK-108 can be up to 249.22 times, 274.05 times and 302.23 times of YK-124 respectively.

The data were analyzed using GraphPad Prism software. There was no significant difference between YK-122 and YK-123 and SM-102, while there was a significant difference between YK-121, YK-125, YK-126 and YK-124. YK-101, YK-107 and YK-108 were significantly different from other compounds, and the transfection efficiencies were significantly improved.

### b. Differences in chemical structure

The structural differences between this series of compounds and YK-101, YK-107 and YK-108 are very small, wherein the only difference is that an ether bond was introduced into the G₃ group introduces or the G₃ group was changed to a 4-(dimethylamino)butyryl group. The structural differences between these compounds are also very small.

For example, compared with YK-101, the three compounds YK-121, YK-123 and YK-125 are different only in the G₃ group; compared with YK-108, the G₃ group is different and the R₁ group and G₂ group each have 1 less C, and the other structures are exactly the same. However, the cell transfection activities were very different, wherein YK-101 was 47 times, 25 times and 120 times that of YK-121, YK-123 and YK-125 respectively. YK-108 was 58 times, 31 times and 145 times that of YK-121, YK-123 and YK-125, respectively.

Compared with YK-122, YK-121 only has a slight difference in the R₂ group. YK-121 has 1 more C than YK-122 in the single chain, and has 2 less C than YK-122 in each single chain of the double chain, but the cell transfection efficiency of YK-122 was 3.3 times that of YK-121. Compared with YK-124, YK-123 has only a slight difference in the R₂ group. YK-123 has 1 more C than YK-124 in the single chain, and has 2 less C than YK-124 in each single chain in the double chain, but in terms of cell transfection efficiency, YK-123 was much higher than YK-124, i.e., 10 times that of YK-124; The difference in the structure of YK-125 and YK-126 is also the same as that between YK-121 and YK-122, that is, only the R₂ group is slightly different. YK-125 has 1 more C than YK-126 in the single chain, and has 2 less C in each single chain in the double chain, but the cell transfection activities of YK-125 and YK-126 were very similar, and YK-126 was only 1.3 times that of YK- 125.

It can be seen from the comparison of the activities of the three pairs of compounds YK-121/YK-122, YK-123/YK-124 and YK-125/YK-126 that although the R₂ groups of YK-121, YK-123 and YK-125 have the same structure changes, that is, the single chain has 1 more C, and each single chain in the double chain has 2 C less, the activity changes were completely different. One was enhanced, one was greatly reduced, and the other one was basically unchanged. Therefore, changing one group to another does not necessarily have the same impact on activity, that is, changes in transfection activity cannot be inferred based on changes in chemical structure.

### Brief summary:

From the above, it can be seen that compared with a series of compounds in which an ether bond was introduced into the G₃ group introduces or the G₃ group was changed to a 4-(dimethylamino)butyryl group, YK-101, YK-107 and YK-108 had the highest cell transfection activities, such as, up to 240 times, 270 times and 300 times that of YK-124 respectively. Moreover, for compounds with similar structures, it is impossible to infer differences in cell transfection activity based on structural differences. Therefore, cationic lipids with high transfection activity cannot be obtained based on simple chemical structure combinations or group substitutions. It is very difficult to screen out compounds with high transfection efficiency, which requires a lot of creative effort.

### Summary:

1) Through various designs of compound structures and a lot of creative work, we screened out cationic lipid compounds with high cell transfection efficiency, such as YK-101, YK-107 and YK-108.

The LNP formulations prepared from YK-101, YK-107 and YK-108 had the strongest cell transfection activities, which were significantly improved compared with similar or used cationic lipids in the prior art. For example, they can be up to 16 times that of SM-102 and 19 times that of compound 23.

YK-101, YK-107 and YK-108 had the highest cell transfection activities among the series of compounds with the most similar structures we designed, which can be up to 34 times that of other compounds, such as YK-109.

YK-101, YK-107 and YK-108 had the highest transfection activities compared to compounds with some minor differences in structure only in the G₁, G₃ or R₁ group. They can be up to 10,000 times higher than other compounds, such as YK-112.

YK-101, YK-107 and YK-108 had the highest transfection activities compared with compounds in which only an ether bond is introduced into the G3 group or the G3 group is changed to a 4-(dimethylamino)butyryl group. For example, they can be up to more than 300 times higher than that of YK-124.
2) There is no correspondence between the structure of a compound and intracellular transfection efficiency. Compounds with small structural differences are likely to have very large differences in transfection efficiency.
3) Changing one identical group to another identical group in different compounds may not necessarily have the same impact on activity, and may even be opposite, that is, changes in activity cannot be inferred based on changes in chemical structure. Therefore, screening out cationic lipid compounds with high transfection efficiency requires multiple designs and a lot of creative work.

### 2. Cell survival rate determination

An LNP formulation containing 1.5 µg of Fluc-mRNA (the carrier component of the LNP formulation were cationic lipid, neutral lipid, structured lipid and polymer-conjugated lipid with a molar ratio of 49: 10: 39.5: 1.5, wherein the cationic lipid was listed in Table 1) and the formulation of Lipofectamine 3000 were added to the cell culture medium of a 96-well plate, and further cultivated for 24 hours. 10 µL of CCK-8 solution was then added to each well, and the culture plate was incubated in an incubator for 1 hour. The absorbance at 450 nm was measured by a microplate reader. The results are shown in Tables 7-10.

### Assay results:

**(1) Among a series of designed compounds, the LNP formulations prepared from YK-101, YK-107 and YK-108 had the lowest cytotoxicities and cell survival rates were significantly improved compared with similar or used cationic lipids in the prior art. For example, YK-107 was 39.43% higher than ALC-0315, 22.28% higher than SM-102, and 22.68% higher than HHMA.**

**Table 7 Cell survival rate - 1**

| **Serial No.** | **Cationic lipid** | **Cell survival rate (%)** |
|---|---|---|
| 1 | YK-101 | 90.12 |
| 2 | YK-107 | 90.45 |
| 3 | YK-108 | 89.86 |
| 4 | YK-009 | 73.57 |
| 5 | SM-102 | 68.17 |
| 6 | ALC-0315 | 51.02 |
| 7 | **Compound 21** | 68.53 |
| 8 | **Compound 23** | 70.91 |
| 9 | HHMA | 67.77 |
| 10 | Lipofectamine 3000 | 21.91 |

### a. Difference in cell survival rate

Table 7 lists the cytotoxicity test results of LNP formulations prepared from different cationic lipid compounds. Among them, YK-009 is disclosed in CN114044741B (claim 1), SM-102 is compound 25 disclosed in WO2017049245A2 (page 29 of the specification), ALC-0315 is compound 3 disclosed in CN108368028B (page 24 of the specification), and compound 21 and compound 23 are disclosed in WO2021055833A1 (page 22 of the specification), and HHMA is compound 1 disclosed in CN112979483B (page 12 of the specification). Liposomes prepared from these cationic lipids all have good cell transfection effect; Lipofectamine 3000 is currently a widely used cell transfection reagent with good transfection performance.

It can be seen from Table 7, the LNP formulations of Fluc-mRNA prepared from YK-101, YK-107 and YK-108 had the highest cell survival rates, which can be up to 90.12%, 90.45% and 89.86% respectively, which were 21.95%, 22.28% and 21.69% higher than SM-102, 39.10%, 39.43% and 38.84% higher than ALC-0315, 21.59%, 21.92 % and 21.33 % higher than compound 21, 19.21%, 19.54% and 18.95% higher than compound 23, 22.35%, 22.68% and 22.09% higher than HHMA, 68.21%, 68.54 % and 67.95% higher than Lipofectamine 3000, 16.55%, 16.88 % and 16.29% higher than YK-009, respectively. (Fig. 8)

The data were analyzed using GraphPad Prism software, wherein YK-101, YK-107 and YK-108 all had significant differences from SM-102, ALC-0315, compound 21, compound 23, Lipofectamine 3000 and YK -009, indicating significantly reduced cytotoxicities.

### b. Differences in chemical structure

The chemical structures of YK-101, YK-107 and YK-108 are similar to those of cationic lipids in the prior art, with only a difference of 1-2 C in the G₁, R₁, G₂ or R₂ group.

### Brief summary:

Among a series of designed compounds, the LNP formulations prepared from YK-101, YK-107 and YK-108 had the lowest cytotoxicities and significantly improved cell survival rates compared with similar or used cationic lipids in the prior art. For example, YK-107 was 39.43% higher than ALC-0315, 22.28% higher than SM-102, and 22.68% higher than HHMA. At the same time, there is no corresponding relationship between the structure of cationic lipid compounds and their toxicity to cells. It does not mean that compounds with very small structural differences must be similar in terms of cytotoxicity, but they are very likely to be very different.

**(2) Among compounds with the most similar structures we designed, that is, only individual groups, such as G₁, G₂ and R₁, differ by 1-2 C, YK-101, YK-107 and YK-108 had the lowest cytotoxicities, with the cell survival rates that can be 45 % higher than that of other compounds, such as YK-103.**

In order to compare the differences in cytotoxicity of compounds with similar structures, we compared YK-101, YK-107 and YK-108 with the compounds with the closest structures. The results showed that the cytotoxicity of this series of compounds varied greatly, with cell survival rates ranging from 45 % to 91%. YK-101, YK-107 and YK-108 had the highest cell survival rates of up to 90.12 %, 90.45% and 89.86 %, respectively, which were 45% higher than other compounds, such as YK-103.

**Table 8 Cell survival rate - 2**

| **Serial No.** | **Cationic lipid** | **Cell survival rate (%)** |
|---|---|---|
| 1 | YK-101 | 90.12 |
| 2 | YK-107 | 90.45 |
| 3 | YK-108 | 89.86 |
| 4 | YK-102 | 51.82 |
| 5 | YK-103 | 45.25 |
| 6 | YK-104 | 73.55 |
| 7 | YK-105 | 78.58 |
| 8 | YK-106 | 59.59 |
| 9 | YK-109 | 69.45 |
| 10 | SM-102 | 68.17 |
| 11 | ALC-0315 | 51.02 |
| 12 | **Compound 21** | 68.53 |
| 13 | **Compound 23** | 70.91 |
| 14 | HHMA | 67.77 |
| 15 | Lipofectamine 3000 | 21.91 |

### a. Difference in cell survival rate

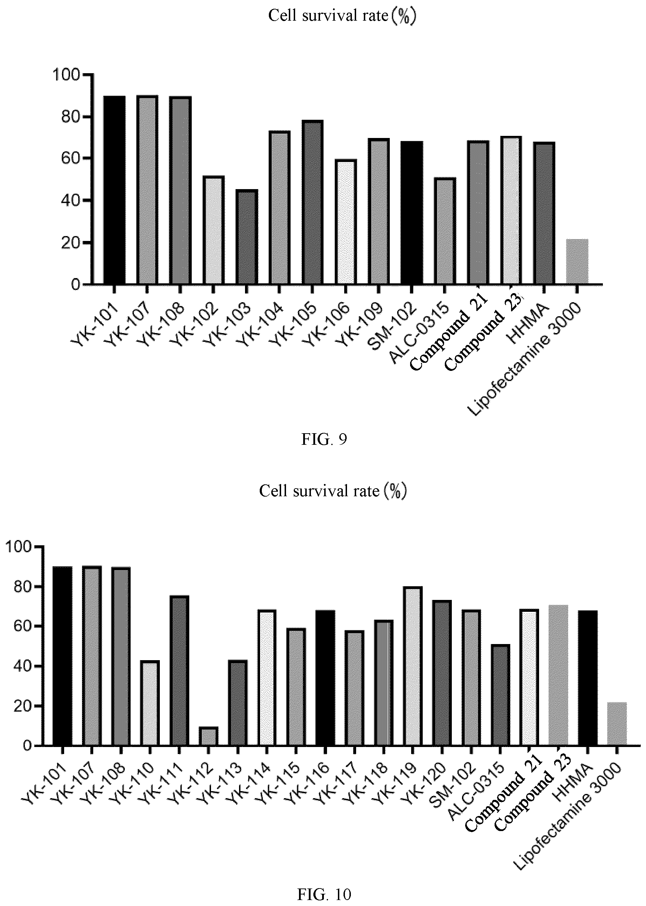

It can be seen from Table 8 that the cytotoxicities of LNP formulations prepared from these compounds varied greatly, among which YK-101, YK-107 and YK-108 had the lowest toxicities and the highest cell survival rates. The lowest cell survival rates were only 45.25% and 51.82% of YK-103 and YK-102. YK-101, YK-107 and YK-108 are 44.87%, 45.20% and 44.61% higher than YK-103 and 38.30%, 38.63% and 38.04% higher than YK-102, respectively. (Fig. 9)

The cell survival rates of YK-106 and YK-109 were slightly higher, which were 59.59% and 69.45% respectively. Compared with YK-101, YK-107 and YK-108, YK-106 was 30.53%, 30.86% and 30.27% lower respectively, YK-109 was 20.67%, 21.00% and 20.41% lower respectively.

YK-104 and YK-105 had higher cell survival rates of 73.55% and 78.58% respectively. However, compared with YK-101, YK-107 and YK-108, YK-104 were 16.57%, 16.90% and 16.31% lower respectively, and YK-105 was 11.54%, 11.87% and 11.28% lower respectively.

The data were analyzed using GraphPad Prism software. Among them, YK-101, YK-107 and YK-108 all had significant differences in cytotoxicity from other compounds, with significantly reduced cytotoxicity.

### b. Differences in chemical structure

This series of compounds only have some small differences from YK-101, YK-107 and YK-108 in individual groups. For example, G₁, G₂ and R₁ differ by 1-2 C; these compounds are also very similar to each other.

### Brief summary:

Among this series of compounds with very similar structures, YK-101, YK-107 and YK-108 had the highest cell survival rates, which were 44.87%, 45.20% and 44.61% higher than that of YK-103 respectively. There is no correspondence between the structure of a compound and its cytotoxicity. Even a group of compounds with the most similar structures may have very different cytotoxicities. Therefore, it is very difficult to screen out compounds with high transfection efficiency and low cytotoxicity from a series of structurally similar compounds.

**(3) Screening out compounds with low cytotoxicity is very difficult and requires a lot of creative effort**
**I. YK-101, YK-107 and YK-108 had the highest cell survival rates compared to compounds with some minor differences in structure only in the G₁, G₃ or R₁ group, which at most 80% higher than that of other compounds, such as YK-112.**

We further compared the differences in cell survival rates between YK-101, YK-107 and YK-108 with other structurally similar compounds. This series of compounds only have some minor differences in the G_{1,} G₃ or R₁ group, such as 2 more C in G₁, an introduced side chain group to G₃ or an introduced -S- or -S-S- group to R₁. The results showed that the cytotoxicities of this series of compounds were very different. YK-101, YK-107 and YK-108 had the highest cell survival rates, which were 80 % higher than that of other compounds, such as YK-112.

**Table 9 Cell survival rate - 3**

| **Serial No.** | **Cationic lipid** | **Cell survival rate (%)** |
|---|---|---|
| 1 | YK-101 | 90.12 |
| 2 | YK-107 | 90.45 |
| 3 | YK-108 | 89.86 |
| 4 | YK-110 | 42.85 |
| 5 | YK-111 | 75.76 |
| 6 | YK-112 | 9.82 |
| 7 | YK- 113 | 43.05 |
| 8 | YK-114 | 68.33 |
| 9 | YK-115 | 59.07 |
| 10 | YK-116 | 67.96 |
| 11 | YK-117 | 57.93 |
| 12 | YK-118 | 63.33 |
| 13 | YK-119 | 80.27 |
| 14 | YK-120 | 73.43 |
| 15 | SM-102 | 68.17 |
| 16 | ALC-0315 | 51.02 |
| 17 | **Compound 21** | 68.53 |
| 18 | **Compound 23** | 70.91 |
| 19 | HHMA | 67.77 |
| 20 | Lipofectamine 3000 | 21.91 |

### a. Difference in cell survival rate

Compared with YK-101, YK-107 and YK-108, other compounds only have some minor differences in the G₁, G₃ or R₁ group, but the impact on cytotoxicity was very large. The cell survival rates of them could be at most 80% lower than those of YK-101, YK-107 and YK-108.

As can be seen from Table 9, the cell survival rates of YK-111, YK-114, YK-116, YK-119 and YK-120 were quite different from those of YK-101, YK-107 and YK-108, wherein YK-111 was 14.36%, 14.69% and 14.10% lower respectively, YK-114 was 21.79%, 22.12% and 21.53% lower respectively, YK-116 was 22.16%, 22.49% and 21.90% lower respectively, YK-119 was 9.85%, 10.18% and 9.59% lower respectively, and YK-120 was 16.69%, 17.02% and 16.43% lower respectively. (Fig. 10)

The cell survival rates of YK-113, YK-115, YK-117 and YK-118 were 43.05%, 59.07%, 57.93% and 63.33% respectively. Compared with YK-101, YK-107 and YK-108, YK-113 was 47.07%, 47.40% and 46.81% lower respectively, YK-115 was 31.05%, 31.38% and 30.79% lower respectively, YK-117 was 32.19%, 32.52% and 31.93% lower respectively, YK-118 was 26.79%, 27.12 % and 26.53% lower respectively.

YK-112 had the most cytotoxicity, with a cell survival rate of only 9.82 %, which was 80.30%, 80.63% and 80.04% lower than YK-101, YK-107 and YK-108 respectively.

The data were analyzed using GraphPad Prism software. Among them, YK-101, YK-107 and YK-108 all had significant differences in cytotoxicity from other compounds, with significantly reduced cytotoxicities.

### b. Differences in chemical structure

This series of compounds only has some minor differences in the G₁, G₃ or R₁ groups from YK-101, YK-107 and YK-108, such as 2 more C in G₁ and an introduced side chain group in G₃ or an introduced -S- or -S-S- group in Ri; the structural differences among these compounds are also very small.

### Brief summary:

Compared with compounds in which 2 C were added in G₁, a side chain group wasintroduced in G₃, or a -S- or -S-S- groups were introduced in R₁, YK-101, YK-107 and YK-108 had the highest cell survival rates. For example, YK-101, YK-107 and YK-108 were all 80% higher than YK-112, and 40 % higher than YK-110 and YK-113. Moreover, even small differences in structure can have a great impact on cytotoxicity, so screening out compounds with low cytotoxicity is very difficult, which requires a lot of creative work.

**II. YK-101, YK-107 and YK-108 had the lowest cytotoxicities compared with compounds in which only an ether bond is introduced into the G₃ group, or the G₃ group is changed to a 4-(dimethylamino)butyryl group in the structure. For example, cell survival rate of them can be up to 60% higher than YK-126.**

Cytotoxicity results showed that some compounds that have the structural difference from YK-101, YK-107 and YK-108 only in that an ether bond was introduced into the G₃ group or the G₃ group was changed to a 4-(dimethylamino)butyryl group had increased cytotoxicities over YK-101, YK-107 and YK-108. For example, YK-126 had cell survival rate reduced by 60 %.

**Table 10 Cell survival rate - 4**

| **Serial No.** | **Cationic lipid** | **Cell survival rate (%)** |
|---|---|---|
| 1 | YK-101 | 90.12 |
| 2 | YK-107 | 90.45 |
| 3 | YK-108 | 89.86 |
| 4 | YK-121 | 73.11 |
| 5 | YK-122 | 79.15 |
| 6 | YK-123 | 76.68 |
| 7 | YK-124 | 77.56 |
| 8 | YK-125 | 35.39 |
| 9 | YK-126 | 25.03 |
| 10 | SM-102 | 68.17 |
| 11 | ALC-0315 | 51.02 |
| 12 | **Compound 21** | 68.53 |
| 13 | **Compound 23** | 70.91 |
| 14 | HHMA | 67.77 |
| 15 | Lipofectamine 3000 | 21.91 |

### a. Difference in cell survival rate

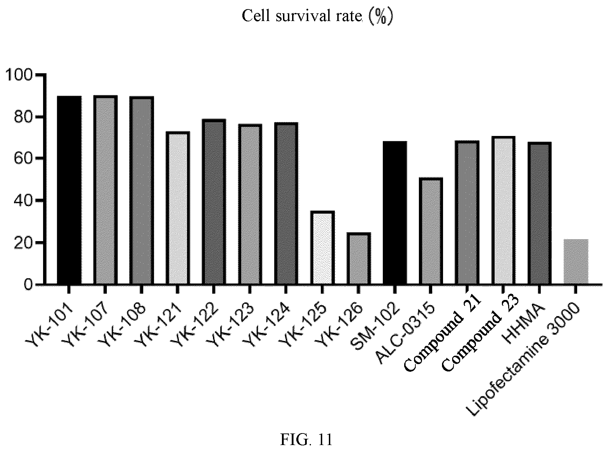

It can be seen from Table 10, when G₃ of YK-101, YK-107 and YK-108 introduces an ether bond or is changed to a 4-(dimethylamino)butyryl group, the cytotoxicity changes greatly. The survival rates of some compounds were not very different from that of YK-101, YK-107 and YK-108; for some compounds, such as YK-126, the cell survival rate was 60% lower than that of YK-101, YK-107 and YK-108. (Fig. 11)

Specifically, the cell survival rates of YK-121, YK-122, YK-123 and YK-124 were 73.11%, 79.15%, 76.68% and 77.56% respectively. Compared with YK-101, YK-107 and YK-108, YK-121 was 17.01%, 17.34% and 16.75% lower respectively, YK-122 was 10.97%, 11.30% and 10.71% lower respectively, YK-123 was 13.44%, 13.77% and 13.18% lower respectively, and YK-124 was 12.56%, 12.89% and 12.30% lower respectively.

The cell survival rates of YK-125 and YK-126 were only 35.39% and 25.03% respectively. Compared with YK-101, YK-107 and YK-108, YK-125 was 54.73%, 55.06% and 54.47% lower respectively, and YK-126 was 65.09%, 65.42% and 64.83% lower respectively.

Compared with YK-122 and YK-124, the cell survival rate of YK-126 was 54.12% and 52.53% lower respectively.

The data were analyzed using GraphPad Prism software. Among them, YK-101, YK-107 and YK-108 all had significant differences from YK-122, YK-123, YK-124, YK-125 and YK-126 with significantly reduced cytotoxicities.

### b. Differences in chemical structure

The structural differences between this series of compounds and YK-101, YK-107 and YK-108 are very small, which are only in that an ether bond is introduced into the G₃ group or the G₃ group is changed to a 4-(dimethylamino)butyryl group. At the same time, the structural differences between these compounds are also small.

### Brief summary:

Compared with a series of compounds in which an ether bond is introduced into the G₃ group or the G₃ group is changed to a 4-(dimethylamino)butyryl group, YK-101, YK-107 and YK-108 had the lowest cytotoxicities. For example, the cell survival rates of them were 54.73%, 55.06% and 54.47% higher than YK-125, and 65.09%, 65.42% and 64.83% higher than YK-126 respectively. The cytotoxicities of this series of compounds also differ greatly. For example, YK-126 had a cell survival rate 54.12% and 52.53% lower than YK-122 and YK-124, respectively. Therefore, the cytotoxicity cannot be predicted based on chemical structure. It is very difficult to screen out compounds with high transfection efficiency and low cytotoxicity, which requires a lot of creative work.

### Summary:

1) We measured the cell survival rate of LNP formulations prepared from a series of designed compounds and screened out cationic lipid compounds with low cytotoxicity, such as YK-101, YK-107 and YK-108.

The LNP formulations prepared from YK-101, YK-107 and YK-108 had the lowest cytotoxicities and significantly reduced cytotoxicities compared with similar or used cationic lipids in the prior art. For example, the cell survival rate of YK-107 was 39.43% higher than ALC-0315, 22.28% higher than SM-102, and 22.68% higher than HHMA.

YK-101, YK-107 and YK-108 had the lowest cytotoxicities among the compounds we designed that have the most similar structures, that is, only individual groups, such as Gi, G₂ and R₁, differ by 1-2 C. The cell survival rates of the three compounds can be 45% higher than that of other compounds, such as YK-103.

YK-101, YK-107 and YK-108 had the lowest cytotoxicities compared to compounds with some minor differences in structure only in the G₁, G₃ or R₁ group. The cell survival rates of the three compounds can be 80% higher than that of other compounds, such as YK-112.

YK-101, YK-107 and YK-108 had the lowest cytotoxicities compared with compounds in which only an ether bond is introduced into the G3 group or the G3 group is changed to a 4-(dimethylamino)butyryl group. For example, the cell survival rates of the three can be 60% higher than YK-126.
2) In addition, there is no correspondence between the structure of a compound and its cytotoxicity. Even compounds with small structural differences will likely have very large differences in cytotoxicity.
3) Cytotoxicity cannot be predicted based on chemical structure. It is very difficult to screen out compounds with high transfection efficiency and low cytotoxicity, which requires a lot of creative work.

### Example 7: In vivo validation of the performance of cationic lipid delivery carriers

In addition, we also verified the protein expression and duration of mRNA in mice delivered by the cationic lipid delivery carriers we designed. *In vivo* tests further proved that our LNP delivery carriers can effectively deliver mRNA into the body and make the mRNA express efficiently and sustainably.

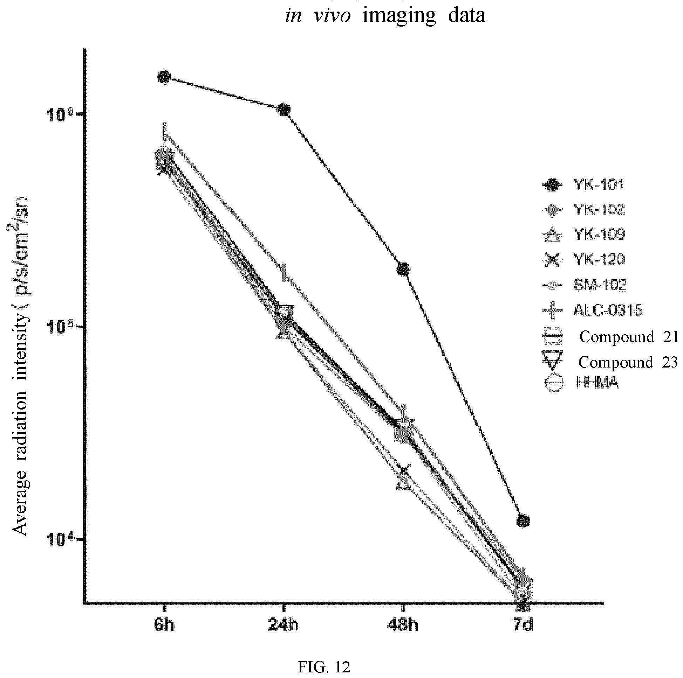

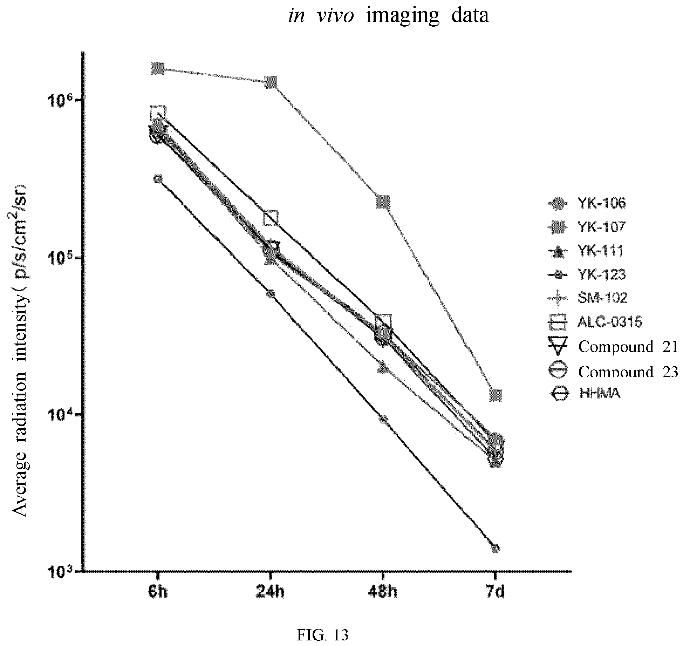

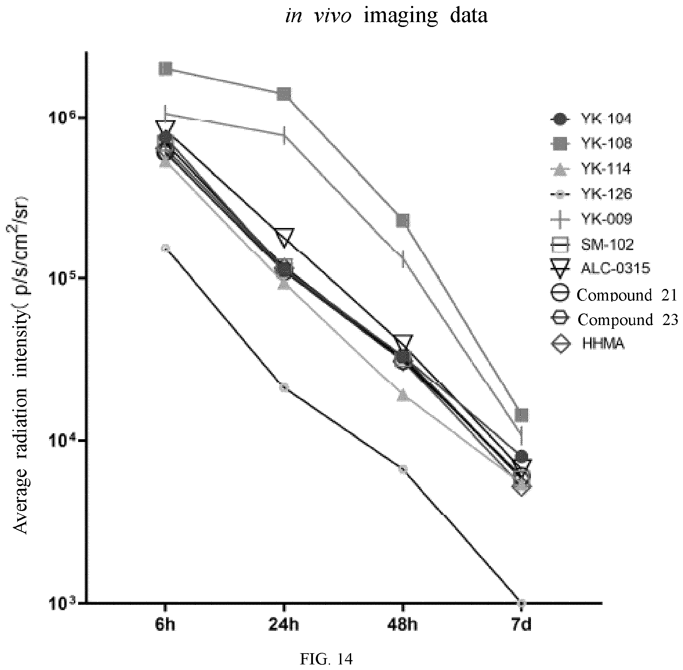

The LNP formulation containing 10 µg Fluc-mRNA was injected intramuscularly into female BALB/C mice aged 4-6 weeks old and weighed 17-19 g, and the mice were intraperitoneally injected with fluorescent imaging substrate at specific time points after administration (6h, 24h, 48h and 7d), where the mice were allowed to move freely for 5 minutes, and then the average radiation intensity (corresponding to fluorescence expression intensity) of the protein expression of the mRNA carried by the LNP in the mice was detected by the IVIS Spectrum small animal *in vivo* imaging device. The test results are shown in Tables 11 to 14 and Figures 12 to 14.

### Assay results:

**(1) Among the designed series of compounds, the LNP formulations prepared from YK-101, YK-107 and YK-108 had high and sustained expression of mRNA in mice, which were improved significantly compared with similar or used cationic lipids in the prior art. For example, the expression amount of YK-108 can be** up **to 11 times that of SM-102, and 12 times that of compound 21 and compound 23. The expression of mRNA in mice was consistent with the cell transfection activity.**

**Table 11 Test data in the in vivo imaging in mice - 1**

| **Serial No.** | **Cationic lipid** | **Time** | | | |
|---|---|---|---|---|---|
| | | **6h** | **24h** | **48h** | **7d** |
| 1 | YK-101 | 1496240 | 1056210 | 185905 | 12165 |
| 2 | YK-107 | 1601250 | 1302640 | 226170 | 13288 |
| 3 | YK-108 | 2017280 | 1406010 | 230360 | 14194 |
| 4 | YK-009 | 1055200 | 770050 | 132100 | *10665* |
| 5 | SM-102 | 689570 | 117750 | 31900 | 5834 |
| 6 | ALC-0315 | 830590 | 179680 | 39015 | 6643 |
| 7 | **Compound 21** | 606680 | 110210 | 31062 | 6024 |
| 8 | **Compound 23** | 599850 | 112670 | 33060 | 5863 |
| 9 | HHMA | 639280 | 115530 | 30852 | 5235 |

### a. Expression differences in mice

Table 11 lists the mRNA expression intensities of LNP formulations containing Fluc-mRNA prepared from different cationic lipids at different times in mice. Among them, YK-009 is disclosed in CN114044741B (claim 1), SM-102 is compound 25 disclosed in WO2017049245A2 (page 29 of the specification), ALC-0315 is compound 3 disclosed in CN108368028B (page 24 of the specification), and compound 21 and compound 23 are disclosed in WO2021055833A1 (page 22 of the specification), and HHMA is compound 1 disclosed in CN112979483B (page 12 of the specification). These cationic lipids can be used to prepare carriers for delivering mRNA.

It can be seen from Table 11 that the LNP formulations containing Fluc-mRNA prepared from YK-101, YK-107 and YK-108 had high and sustained expression of mRNA in mice.

The average radiation intensity at 6h of YK-101, YK-107 and YK-108 were 1496240, 1601250 and 2017280 respectively, which were 2.2 times, 2.3 times and 2.9 times that of SM-102 respectively, 1.8 times, 1.9 times and 2.4 times that of ALC-0315, 2.5 times, 2.6 times and 3.3 times that of compound 21, 2.5 times, 2.7 times and 3.4 times that of compound 23, 2.3 times, 2.5 times and 3.2 times that of HHMA.

At 24 h, they were 1056210, 1302640 and 1406010 respectively, which were 9.0 times, 11.1 times and 11.9 times that of SM-102, 5.9 times, 7.2 times and 7.8 times that of ALC-0315, 9.6 times, 11.8 times and 12.8 times that of compound 21, 9.4 times, 11.6 times and 12.5 times that of compound 23, and 9.1 times, 11.3 times and 12.2 times that of HHMA.

At 48 h, they were 185905, 226170 and 230360 respectively, which were 5.8 times, 7.1 times and 7.2 times that of SM-102, 4.8 times, 5.8 times and 5.9 times that of ALC-0315, 6.0 times, 7.3 times and 7.4 times that of compound 21, 5.6 times, 6.8 times and 7.0 times that of compound 23, and 6.0 times, 7.3 times and 7.5 times that of HHMA.

At 7 d, they were 12165, 13288 and 14194 respectively, which were 2.1 times, 2.3 times and 2.4 times that of SM-102, 1.8 times, 2.0 times and 2.1 times that of ALC-0315, 2.0 times, 2.2 times and 2.4 times that of compound 21, 2.1 times, 2.3 times and 2.4 times that of compound 23, and 2.3 times, 2.5 times and 2.7 times that of HHMA.

The data were analyzed using GraphPad Prism software. Among them, YK-101, YK-107 and YK-108 had significant differences from SM-102, ALC-0315, compound 21, compound 23, HHMA and YK-009 at each time, with significantly improved expression amount and duration.

### b. Differences in chemical structure

The chemical structures of YK-101, YK-107 and YK-108 are similar to those of cationic lipids in the prior art, with only a difference of 1-2 C in the G_{1,} R₁, G₂ or R₂ group.

### Brief summary:

Among a series of designed compounds, the LNP formulations prepared from YK-101, YK-107 and YK-108 had the highest and sustained expressions of mRNA in mice, which were significantly improved compared with the expression amounts of similar or used cationic lipids in the prior art at 6h, 24h, 48h and 7d. For example, YK-108 can be up to 11 times that of SM-102, and 12 times that of compound 21 and compound 23. The expression of mRNA in mice was consistent with the results of the cell transfection assay in Example 6. Moreover, there is no correspondence between the structure of the cationic lipid compound and the expression of mRNA in mice. It is not that LNP preparations prepared from cationic lipids with very small structural differences must be similar in terms of mRNA expression in mice, but are very likely to be very different.

(2) Among compounds we designed with the most similar structures, that is, only individual groups such as G₁, G₂ and R₁ differ by 1-2 C, LNP formulations **prepared from YK-101, YK-107 and YK-108 had the highest amount and the longest duration of mRNA expressions in mice. The expression amount of YK-108 can be up to 14 times that of other compounds, such as YK-109. The expression of mRNA in mice was consistent with the cell transfection activity.**

In order to compare the differences in expression intensity and duration of mRNA in mice delivered by the delivery carriers prepared from cationic lilpids with the most similar structures, YK-101, YK-107 and YK-108 were compared with the compounds with the closest structures, i.e. YK-102, YK-104, YK-106 and YK-109. The results show that the LNP formulations prepared by this series of compounds had very different expressions of mRNA in mice. Among them, YK-101, YK-107 and YK-108 had the highest expression amounts and the longest durations with the expression amounts that can be up to 11 times, 13 times and 14 times that of YK-109 respectively.

**Table 12 Test data in the in vivo imaging in mice - 2**

| **Serial No.** | **Cationic lipid** | **Time** | | | |
|---|---|---|---|---|---|
| | | **6h** | **24h** | **48h** | **7d** |
| 1 | YK-101 | 1496240 | 1056210 | 185905 | 12165 |
| 2 | YK-107 | 1601250 | 1302640 | 226170 | 13288 |
| 3 | YK-108 | 2017280 | 1406010 | 230360 | 14194 |
| 4 | YK-102 | 652680 | 100422 | 30507 | 6477 |
| 5 | YK-104 | 747000 | 113940 | 33095 | 7969 |
| 6 | YK-106 | 683840 | 105776 | 32940 | 7052 |
| 7 | YK-109 | 625040 | 94720 | 18496 | 4948 |
| 8 | SM-102 | 689570 | 117750 | 31900 | 5834 |
| 9 | ALC-0315 | 830590 | 179680 | 39015 | 6643 |
| 10 | **Compound 21** | 606680 | 110210 | 31062 | 6024 |
| 11 | **Compound 23** | 599850 | 112670 | 33060 | 5863 |
| 12 | HHMA | 639280 | 115530 | 30852 | 5235 |

### a. Expression differences in mice

It can be seen from from Table 12, among this series of compounds with the closest structures, with only a difference of 1-2 C in individual groups, the LNP formulations prepared from YK-101, YK-107 and YK-108 had the highest amount and the longest duration of mRNA expressions in mice.

The average radiation intensities of YK-101, YK-107 and YK-108 at 6h were 2.3 times, 2.5 times and 3.1 times that of YK-102, respectively, 2.0 times, 2.1 times and 2.7 times that of YK-104, 2.2 times, 2.3 times and 2.9 times that of YK-106, and 2.4 times, 2.6 times and 3.2 times that of YK-109.

At 24 h, they were 10.5 times, 13.0 times and 14.0 times that of YK-102, 9.3 times, 11.4 times and 12.3 times that of YK-104, 10.0 times, 12.3 times and 13.3 times that of YK-106, and 11.2 times, 13.8 times and 14.8 times that of YK-109.

At 48 h, they were 6.1 times, 7.4 times and 7.6 times that of YK-102, 5.6 times, 6.8 times and 7.0 times that of YK-104, 5.6 times, 6.9 times and 7.0 times that of YK-106, and 10.1 times, 12.2 times and 12.5 times that of YK-109.

At 7 d, they were 1.9 times, 2.1 times and 2.2 times of YK-102, 1.5 times, 1.7 times and 1.8 times that of YK-104, 1.7 times, 1.9 times and 2.0 times that of YK-106, and 2.5 times, 2.7 times and 2.9 times that of YK-109.

The data were analyzed using GraphPad Prism software. Among them, YK-101, YK-107 and YK-108 were significantly different from other compounds at each time, with significantly improved expression intensities and durations.

### b. Differences in chemical structure

The structural differences of this series of compounds are very small compared with YK-101, YK-107 and YK-108 with only slightly different individual groups, such as, G_{1,} G₂ and R₁ that differ by 1-2 C.

### Brief summary:

Among a group of this series of compounds with very similar structures, the LNP formulations prepared from YK-101, YK-107 and YK-108 had the highest expression intensity and longest duration of mRNA expressions in mice, which, for example, at 24 hours, were 11.2 times, 13.8 times and 14.8 times that of YK-109 respectively; at 7d, were 2.5 times, 2.7 times and 2.9 times that of YK-109 respectively. The expression of mRNA in mice was consistent with the results of the cell transfection assay in Example 6. Moreover, there is no correspondence between the expression of mRNA in mice and the structure of cationic lipids. Even for LNP formulations prepared from a group of compounds with the most similar structures, the degree and duration of mRNA expression in mice are likely to be very different. Therefore, it is very difficult to screen out compounds with high and sustained expression in animals from a series of cationic lipid compounds with similar structures.

**(3) It is very difficult to screen out cationic lipid compounds with high and sustained expressions of mRNA in animals, which requires a lot of creative work.**

**I. YK-101, YK-107 and YK-108 had the highest amounts and durations of mRNA expression in mice compared with compounds that have some minor differences in structure only in the G₁, G₃ or R₁ group. For example, the expression amount of YK-108 can be 15 times higher than that of YK-114. The expression of mRNA in mice was consistent with the cell transfection activity.**

We further compared the differences in mRNA expression in mice between LNP formulations prepared from YK-101, YK-107 and YK-108 with other structurally similar compounds. This series of compounds only have some minor differences in the G₁, G₃ or **R₁** group, such as 2 more C in G₁, a side chain groups introduced in G₃, or a -S- or -S-S- group introduced in R₁. The results showed that the expressions of mRNA in mice were very different. YK-101, YK-107 and YK-108 had the highest amounts and sustained expression. The expression amount of YK-108 was 15 times higher than other compounds, such as YK-114.

**Table 13 Test data in the in vivo imaging in mice - 3**

| **Serial No.** | **Cationic lipid** | **Time** | | | |
|---|---|---|---|---|---|
| | | **6h** | **24h** | **48h** | **7d** |
| 1 | YK-101 | 1496240 | 1056210 | 185905 | 12165 |
| 2 | YK-107 | 1601250 | 1302640 | 226170 | 13288 |
| 3 | YK-108 | 2017280 | 1406010 | 230360 | 14194 |
| 4 | YK-111 | 661800 | 99777 | 20312 | 5050 |
| 5 | YK-114 | 535760 | 93565 | 19004 | 5457 |
| 6 | YK-120 | 559000 | 94779 | 20832 | 5052 |
| 7 | SM-102 | 689570 | 117750 | 31900 | 5834 |
| 8 | ALC-0315 | 830590 | 179680 | 39015 | 6643 |
| 9 | **Compound 21** | 606680 | 110210 | 31062 | 6024 |
| 10 | **Compound 23** | 599850 | 112670 | 33060 | 5863 |
| 11 | HHMA | 639280 | 115530 | 30852 | 5235 |

### a. Expression differences in mice

It can be seen from Table 13 that among this series of compounds with some minor differences in the chemical structures only in the G₁, G₃ or R₁ groups, LNP formulations prepared from YK-101, YK-107 and YK-108 had the highest amount and duration of expressions of mRNA in mice.

The average radiation intensities of YK-101, YK-107 and YK-108 at 6h were 2.3 times, 2.4 times and 3.0 times that of YK-111, 2.8 times, 3.0 times and 3.8 times that of YK-114, 2.7 times, 2.9 times and 3.6 times that of YK-120 respectively.

At 24 h, they were 10.6 times, 13.1 times and 14.1 times that of YK-111, 11.3 times, 13.9 times and 15.0 times that of YK-114, and 11.1 times, 13.7 times and 14.8 times that of YK-120.

At 48 h, they were 9.2 times, 11.1 times and 11.3 times that of YK-111, 9.8 times, 11.9 times and 12.1 times that of YK-114, and 8.9 times, 10.9 times and 11.1 times that of YK-120.

At 7d, they were 2.4 times, 2.6 times and 2.8 times that of YK-111, 2.2 times, 2.4 times and 2.6 times that of YK- 114, and 2.4 times, 2.6 times and 2.8 times that of YK-120.

The data were analyzed using GraphPad Prism software. Among them, YK-101, YK-107 and YK-108 were significantly different from other compounds at each time, with significantly improved expression effects and durations.

### b. Differences in chemical structure

This series of compounds only has some minor differences in the G₁, G₃ or R₁ groups from YK-101, YK-107 and YK-108, such as 2 more C in G₁ and an introduced side chain group in G₃ or an introduced -S- or -S-S- group in R₁.

### Brief summary:

Compared with compounds with only small differences in structure, such as the 2 C added in G_{1,} a side chain group introduced in G₃, or a -S-S- group introduced in R₁, the LNP formulations prepared from YK-101, YK-107 and YK-108 had the highest amount and duration of expressions of mRNA in mice. For example, YK-101, YK-107 and YK-108 at most were up to 11 times, 13 times and 15 times higher than YK-114 respectively. The expression of mRNA in mice was consistent with the results of the cell transfection assay in Example 6. Moreover, even small differences in structure will have a great impact on the expression of mRNA in mice. Therefore, it is very difficult to screen out cationic lipid compounds with high and sustained expression of mRNA in mice, which requires a lot of creative work.

**II. YK-101, YK-107 and YK-108 had the highest amount and the longest duration of expressions of mRNA in mice compared with compounds in which only an ether bond is introduced into the G3 group or the G3 group is changed to a 4-(dimethylamino)butyryl group. For example, compared with YK-126, the expression amount of YK-108 can be up to 67 times. The expression of mRNA in mice was consistent with the cell transfection activity.**

**Table 14 Test data in the in vivo imaging in mice - 4**

| **Serial No.** | **Cationic lipid** | **Time** | | | |
|---|---|---|---|---|---|
| | | **6h** | **24h** | **48h** | **7d** |
| 1 | YK-101 | 1496240 | 1056210 | 185905 | 12165 |
| 2 | YK-107 | 1601250 | 1302640 | 226170 | 13288 |
| 3 | YK-108 | 2017280 | 1406010 | 230360 | 14194 |
| 4 | YK-123 | 317640 | 58728 | 9336 | 1414 |
| 5 | YK-126 | 155160 | 20939 | 6656 | 877 |
| 6 | SM-102 | 689570 | 117750 | 31900 | 5834 |
| 7 | ALC-0315 | 830590 | 179680 | 39015 | 6643 |
| 8 | **Compound 21** | 606680 | 110210 | 31062 | 6024 |
| 9 | **Compound 23** | 599850 | 112670 | 33060 | 5863 |
| 10 | HHMA | 639280 | 115530 | 30852 | 5235 |

### a. Expression differences in mice

It can be seen from Table 14 that compared with compounds with only a small difference in structure in that an ether bond is introduced into the G₃ group or the G₃ group is changed to a 4-(dimethylamino)butyryl group, the LNP formulations prepared from YK-101, YK-107 and YK-108 had the highest degree and duration of expressions of mRNA in mice.

The average radiation intensities of YK-101, YK-107 and YK-108 at 6h were 4.7 times, 5.0 times and 6.4 times that of YK-123, and 9.6 times, 10.3 times and 13.0 times that of YK-126, respectively.

At 24 h, they were 18.0 times, 22.2 times and 23.9 times that of YK-123, and 50.4 times, 62.2 times and 67.1 times that of YK-126.

At 48 h, they were 19.9 times, 24.2 times and 24.7 times that of YK-123, and 27.9 times, 34.0 times and 34.6 times that of YK-126.

At 7d, they were 8.6 times, 9.4 times and 10.0 times that of YK-123, and 13.9 times, 15.2 times and 16.2 times that of YK-126.

The data were analyzed using GraphPad Prism software. YK-101, YK-107 and YK-108 had significant differences from YK-123 and YK-126 at each time point, with significantly improved expression effects and durations.

### b. Differences in chemical structure

YK-123 and YK-126 only have some minor differences in the G₃ group from YK-101, YK-107 and YK-108, in that, for example, an ether bond is introduced into the G₃ group or the G₃ group is changed to a 4-(dimethylamino)butyryl group.

### Brief summary:

Compared with compounds with only small differences in structure, such as the introduction of an ether bond or change to 4-(dimethylamino)butyryl group in G₃, the LNP formulations prepared from YK-101, YK-107 and YK-108 had the highest and the longest duration of expressions of mRNA in mice, which were 50 times, 62 times and 67 times higher than that of YK-126 respectively. The expression of mRNA in mice was consistent with the results of the cell transfection assay in Example 6. It is impossible to predict the expression effect of mRNA in mice based on the chemical structure. It is very difficult to screen out cationic lipid compounds with high and sustained expressions of mRNA in mice, which requires a lot of creative work.

### Summary:

1) We conducted a testing of performance of *in vivo* delivery of carriers in animals on LNP formulations prepared from a series of designed compounds, and screened out cationic lipid compounds with high and sustained expression of mRNA in mice, such as YK-101, YK -107 and YK-108.

The LNP formulations prepared from YK-101, YK-107 and YK-108 had high and sustained expressions of mRNA in mice, which were significantly higher than similar or used cationic lipids in the prior art. For example, YK-108 can be up to 11 times that of SM-102, and 12 times that of compound 21 and compound 23.

YK-101, YK-107 and YK-108 had the highest mRNA expression amounts and durations of mRNA in mice among the compounds we designed that have the most similar structures, that is, only individual groups, such as G₁, G₂ and R₁, differ by 1-2 C. The expression amount of YK-108 can be 14 times higher than that of other compounds, such as YK-109.

YK-101, YK-107 and YK-108 had the highest mRNA expression amounts and durations of mRNA in mice compared to compounds with some minor differences in structure only in the G₁, G₃ or R₁ group. For example, the expression amount of YK-108 can be up to 15 times that of YK-114.

YK-101, YK-107 and YK-108 had the highest mRNA expression amounts and durations of mRNA in mice compared with compounds in which only an ether bond is introduced into the G3 group or the G3 group is changed to a 4-(dimethylamino)butyryl group. For example, the expression amount of YK-108 can be up to 67 times higher than that of YK-126.
2) In addition, there is no correspondence between the structure of cationic lipids and the high expression and sustained expression of mRNA in mice. Even if the structural differences of cationic lipid compounds are very small, the expression *in vivo* of mRNA in animals of the LNP formulations prepared from them will be likely to be very different.
3) It is impossible to predict whether mRNA will be highly and continuously expressed in animals based on the chemical structure of cationic lipids. Therefore, it is very difficult to screen out cationic lipid compounds with high and sustained expression of mRNA, which requires a lot of creative work.

### Conclusions:

1. Among a series of designed compounds, LNP preparations prepared from YK-101, YK-107 and YK-108 had significantly improved cell transfection activities, significantly reduced cytotoxicities, and significantly improved expression amounts and durations of mRNA in mice, compared with similar or used cationic lipids in the prior art. For example, the cell transfection activity of YK-108 can be up to 16 times that of SM-102 and 19 times that of compound 23; the cell survival rates of the three were 39% higher than ALC-0315 and 22% higher than SM-102 and HHMA; in terms of the expression amount of mRNA in mice, YK-108 was 11 times that of SM-102, and 12 times that of compound 21 and compound 23.

Among a series of compounds with little difference in chemical structure we designed, LNP formulations prepared from YK-101, YK-107 and YK-108 had significantly improved cell transfection activities and significantly reduced cytotoxicities and significantly increased amount and duration of expression of mRNA in mice compared with other compounds. The structure of this series of compounds only differs by 1-2 C in individual groups, or by some minor differences in the G₁, G₃, and R₁ groups, or by that an ether bond is introduced into the G₃ group or the G₃ group is changed to 4-(dimethylamino)butyryl group, but the cell transfection activities of YK-101, YK-107 and YK-108 can be up to more than 10,000 times that of YK-112, the cell survival rates of YK-101, YK-107 and YK-108 were 80% higher than YK-112, and the mRNA expression amount of YK-108 in mice can be up to 67 times that of YK-126.
2. There is no obvious corresponding relationship between the structure of cationic lipid compound and intracellular transfection efficiency, toxicity to cells, and the high expression and sustained expression in animals of mRNA in an LNP formulation prepared from the cationic lipid compound. Compounds with small structural differences are likely to have very large differences in transfection efficiency and/or toxicity to cells and high intracellular expression. For example, for the compounds YK-108 and YK-109 of the present application, the cell transfection efficiency of YK-108 was 34 times that of YK-109, the toxicity to transfected cells of YK-108 was 20 % lower than that of YK-109, and the expression of mRNA in mice of YK-108 can be up to 15 times that of YK-109; YK-101, YK-107 and YK-108 had cell transfection activities that were more than 10,000 times higher than that of YK-112; the expression of mRNA carried by compound YK-107 in animals was 62 times that of YK-126. Therefore, it is very difficult to screen out suitable cationic lipid compounds that can simultaneously have high transfection efficiency and low toxicity to cells, as well as high and sustained expression of mRNA in mice.
3. Through unique design and extensive screening, the present disclosure discovered some compounds, such as YK-101, YK-107, YK-108, YK-103, YK-104, YK-105 and YK-106, can deliver nucleic acids with high cell transfection efficiencies, low cytotoxicities, and high expression and sustained expression in animals, compared with other compounds in the prior art, achieving unexpected technical effects.

## Claims

1. A compound of formula (I) 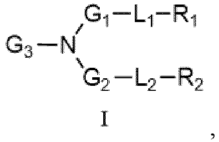 or an N-oxide, a solvate, a pharmaceutically acceptable salt or a stereoisomer thereof, wherein
G₁ is C_{1~6} alkylene;
G₂ is C_{2~8} alkylene;
Li is -C(O)O- or -OC(O)-;
L₂ is -C(O)O- or -OC(O)-;
R₁ is C_{6~15} linear alkyl or R₃-S-R₄ or R₅-S-S-R₆;
R₂ is C_{12~25} branched alkyl;
G₃ is: HO-R₇- or (CH₃)₂N(CH₂)₃C(O)O(CH₂)₂-;
wherein R₃ is C_{1~7} alkylene; R₄ is C_{1~7} alkyl;
R₅ is C_{1~7} alkylene; R₆ is C_{1~7} alkyl;
R₇ is C_{2~8} alkylene, C₅₋₆ cycloalkylene, -CH₂CH(OH)CH₂-, -(CH₂CH₂O)ₘ-(CH₂)ₙ- or -(CH₂)ₘ-O-(CH₂)ₙ-, where m and n are each independently 1, 2, 3 or 4.

2. The compound of formula (I) or an N-oxide, a solvate, a pharmaceutically acceptable salt or a stereoisomer thereof according to claim 1, wherein G₁ is unsubstituted C_{2~5} alkylene.

3. The compound of formula (I) or an N-oxide, a solvate, a pharmaceutically acceptable salt or a stereoisomer thereof according to claim 2, wherein G₁ is unsubstituted C₃ alkylene.

4. The compound of formula (I) or an N-oxide, a solvate, a pharmaceutically acceptable salt or a stereoisomer thereof according to claim 2, wherein G₁ is unsubstituted C₅ alkylene.

5. The compound of formula (I) or an N-oxide, a solvate, a pharmaceutically acceptable salt or a stereoisomer thereof according to claim 2, wherein G₁ is unsubstituted C₄ alkylene.

6. The compound of formula (I) or an N-oxide, a solvate, a pharmaceutically acceptable salt or a stereoisomer thereof according to any one of the preceding claims, wherein G₂ is unsubstituted C_{4~6} alkylene.

7. The compound of formula (I) or an N-oxide, a solvate, a pharmaceutically acceptable salt or a stereoisomer thereof according to claim 6, wherein G₂ is unsubstituted C₅ alkylene.

8. The compound of formula (I) or an N-oxide, a solvate, a pharmaceutically acceptable salt or a stereoisomer thereof according to claim 6, wherein G₂ is unsubstituted C₆ alkylene.

9. The compound of formula (I) or an N-oxide, a solvate, a pharmaceutically acceptable salt or a stereoisomer thereof according to claim 6, wherein G₂ is unsubstituted C₄ alkylene.

10. The compound of formula (I) or an N-oxide, a solvate, a pharmaceutically acceptable salt or a stereoisomer thereof according to any one of claims 1 to 9, wherein Li is -C(O)O-.

11. The compound of formula (I) or an N-oxide, a solvate, a pharmaceutically acceptable salt or a stereoisomer thereof according to any one of claims 1 to 9, wherein Li is -OC(O)-.

12. The compound of formula (I) or an N-oxide, a solvate, a pharmaceutically acceptable salt or a stereoisomer thereof according to any one of claims 1 to 11, wherein L₂ is -C(O)O-.

13. The compound of formula (I) or an N-oxide, a solvate, a pharmaceutically acceptable salt or a stereoisomer thereof according to any one of claims 1 to 11, wherein L₂ is -OC(O)-.

14. The compound of formula (I) or an N-oxide, a solvate, a pharmaceutically acceptable salt or a stereoisomer thereof according to any one of claims 1 to 13, wherein R₁ is unsubstituted C_{8~12} linear alkyl.

15. The compound of formula (I) or an N-oxide, a solvate, a pharmaceutically acceptable salt or a stereoisomer thereof according to claim 14, wherein R₁ is unsubstituted C₁₀ linear alkyl.

16. The compound of formula (I) or an N-oxide, a solvate, a pharmaceutically acceptable salt or a stereoisomer thereof according to claim 14, wherein R₁ is unsubstituted C₁₁ linear alkyl.

17. The compound of formula (I) or an N-oxide, a solvate, a pharmaceutically acceptable salt or a stereoisomer thereof according to claim 14, wherein R₁ is unsubstituted C₉ linear alkyl.

18. The compound of formula (I) or an N-oxide, a solvate, a pharmaceutically acceptable salt or a stereoisomer thereof according to claim 14, wherein R₁ is unsubstituted C₈ linear alkyl.

19. The compound of formula (I) or an N-oxide, a solvate, a pharmaceutically acceptable salt or a stereoisomer thereof according to any one of claims 1 to 13, wherein R₃ is -CH₂CH₂-.

20. The compound of formula (I) or an N-oxide, a solvate, a pharmaceutically acceptable salt or a stereoisomer thereof according to claim 19, wherein R₄ is C₄ linear alkyl.

21. The compound of formula (I) or an N-oxide, a solvate, a pharmaceutically acceptable salt or a stereoisomer thereof according to any one of claims 1 to 13, wherein R₅ is -CH₂CH₂CH₂-.

22. The compound of formula (I) or an N-oxide, a solvate, a pharmaceutically acceptable salt or a stereoisomer thereof according to claim 21, wherein R₆ is C₅ linear alkyl.

23. The compound of formula (I) or an N-oxide, a solvate, a pharmaceutically acceptable salt or a stereoisomer thereof according to any one of the preceding claims, wherein R₂ is unsubstituted C_{14~22} branched alkyl.

24. The compound of formula (I) or an N-oxide, a solvate, a pharmaceutically acceptable salt or a stereoisomer thereof according to claim 23, wherein R₂ is unsubstituted C₁₅ branched alkyl.

25. The compound of formula (I) or an N-oxide, a solvate, a pharmaceutically acceptable salt or a stereoisomer thereof according to claim 23, wherein R₂ is unsubstituted C₁₈ branched alkyl.

26. The compound of formula (I) or an N-oxide, a solvate, a pharmaceutically acceptable salt or a stereoisomer thereof according to claim 23, wherein R₂ is unsubstituted C₁₄ branched alkyl.

27. The compound of formula (I) or an N-oxide, a solvate, a pharmaceutically acceptable salt or a stereoisomer thereof according to claim 23, wherein R₂ is:

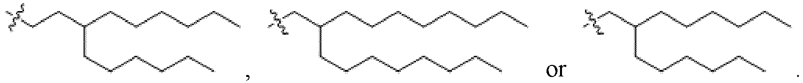

28. The compound of formula (I) or an N-oxide, a solvate, a pharmaceutically acceptable salt or a stereoisomer thereof according to any one of the preceding claims, wherein R₇ is: -(CH₂)₂-, -(CH₂)₃-, -CH₂CH(OH)CH₂-, -CH(CH₃)CH₂-, -CH(CH₃)(CH₂)₂-, -C(CH₃)₂(CH₂)₂-, -(CH₂)₂O(CH₂)₂O(CH₂)₂-, -(CH₂)₂O(CH₂)₂- or

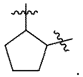

29. The compound of formula (I) or an N-oxide, a solvate, a pharmaceutically acceptable salt or a stereoisomer thereof according to claim 28, wherein R₇ is: -(CH₂)₂-.

30. The compound of formula (I) or an N-oxide, a solvate, a pharmaceutically acceptable salt or a stereoisomer thereof according to claim 28, wherein R₇ is: -(CH₂)₃-.

31. The compound of formula (I) or an N-oxide, a solvate, a pharmaceutically acceptable salt or a stereoisomer thereof according to claim 1, wherein the compound of formula (I) has one of the following structures:

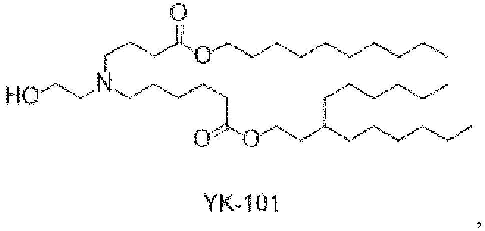

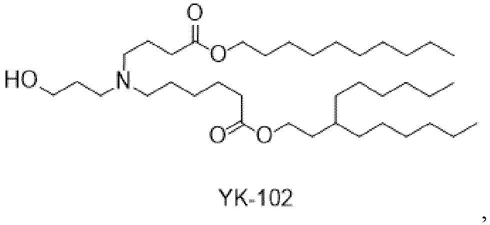

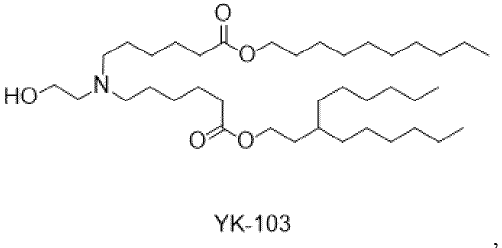

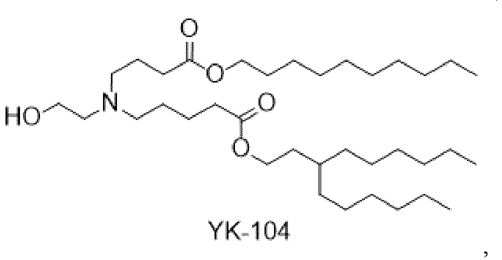

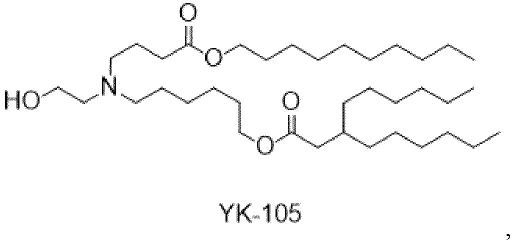

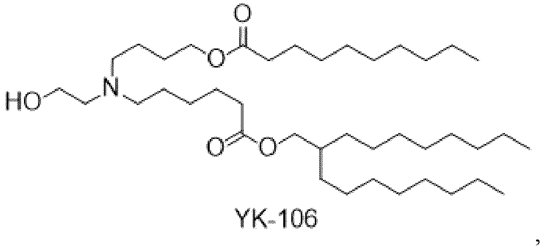

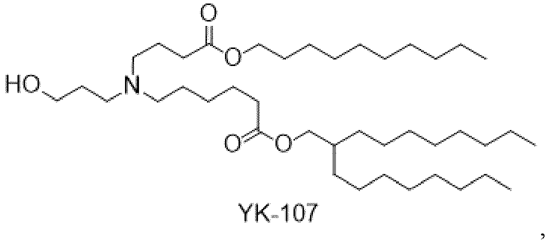

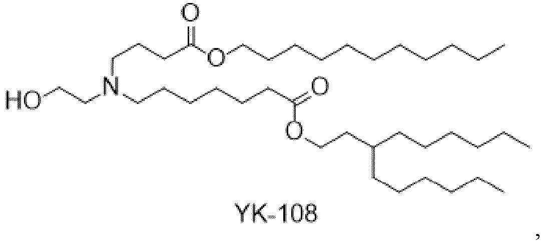

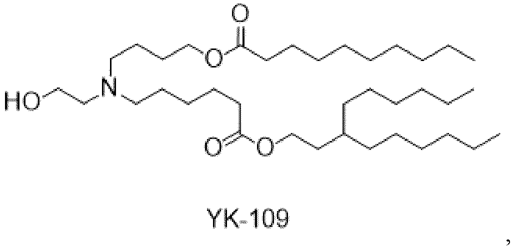

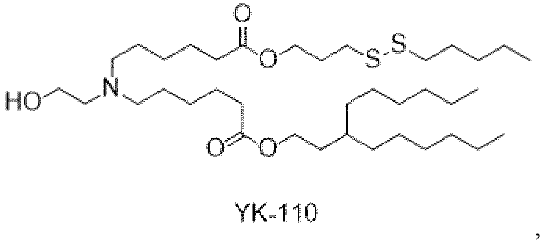

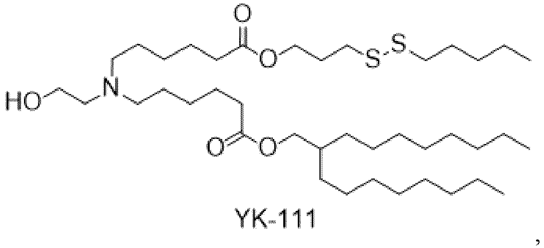

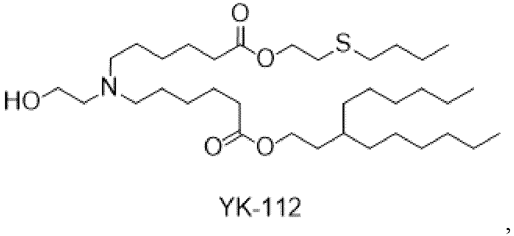

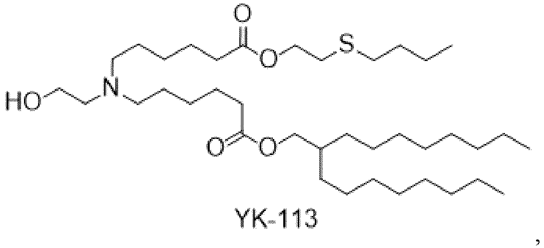

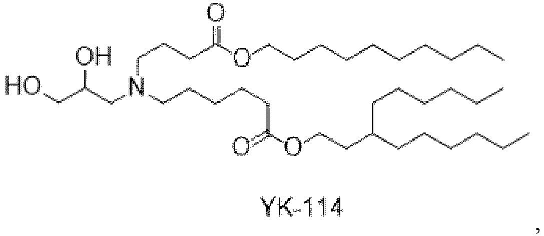

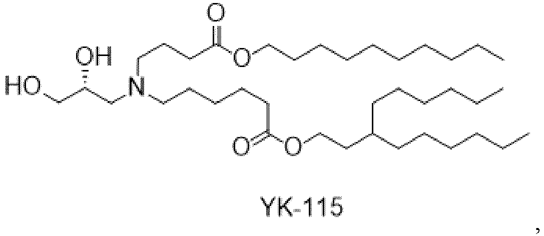

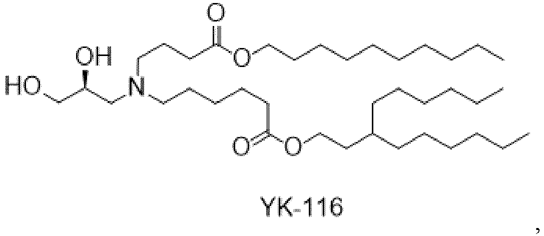

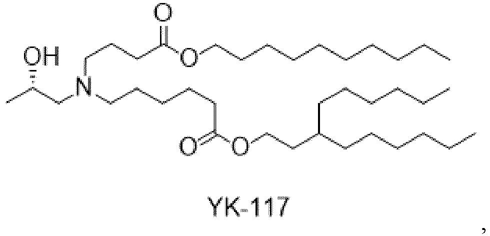

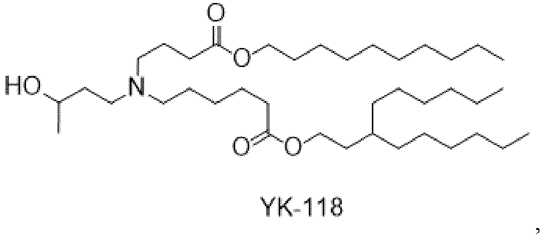

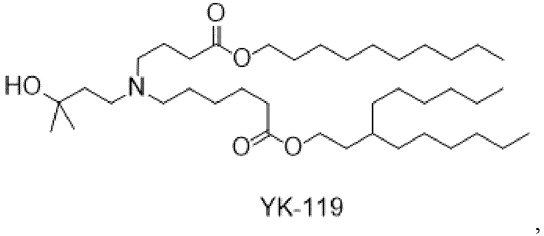

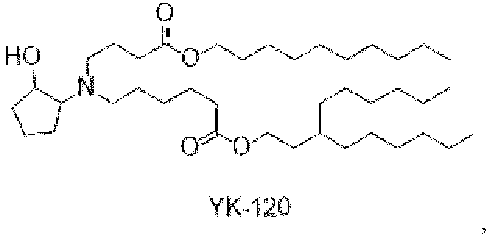

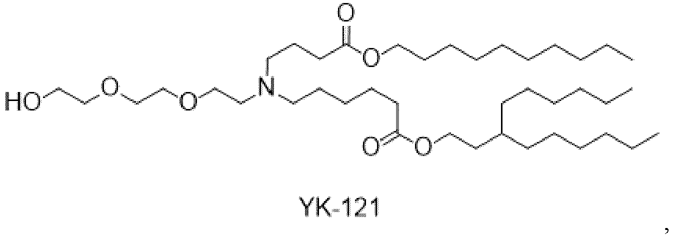

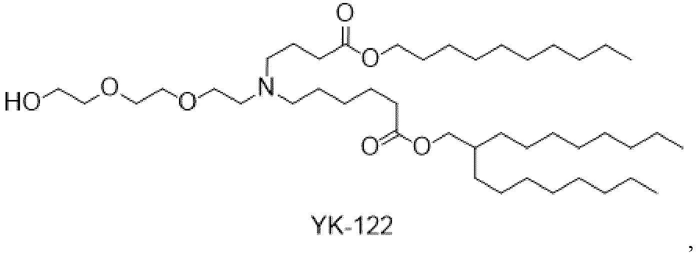

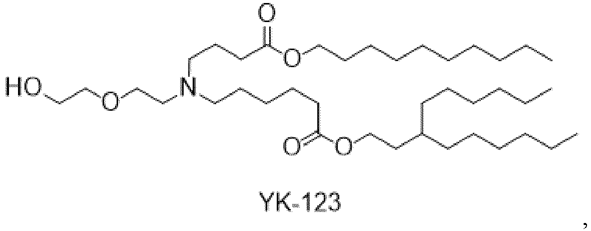

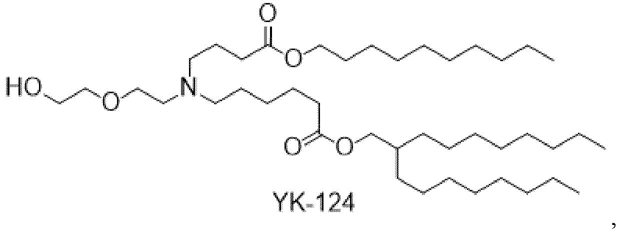

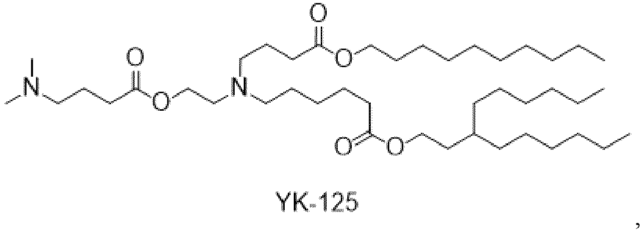

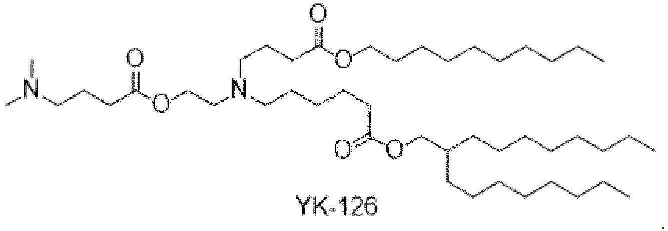

32. The compound of formula (I) or an N-oxide, a solvate, a pharmaceutically acceptable salt or a stereoisomer thereof according to claim 31, wherein the compound of formula (I) is compound YK-101 with the following structure:

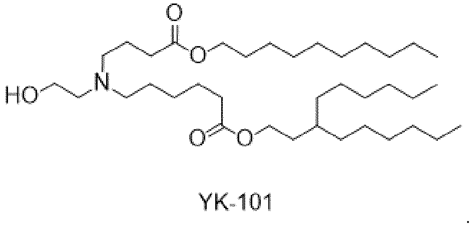

33. The compound of formula (I) or an N-oxide, a solvate, a pharmaceutically acceptable salt or a stereoisomer thereof according to claim 31, wherein the compound of formula (I) is compound YK-107 with the following structure:

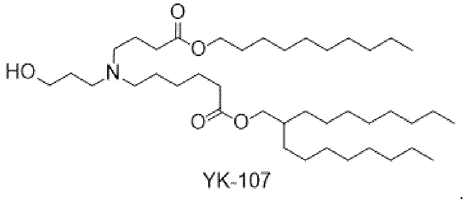

34. The compound of formula (I) or an N-oxide, a solvate, a pharmaceutically acceptable salt or a stereoisomer thereof according to claim 31, wherein the compound of formula (I) is compound YK-108 with the following structure:

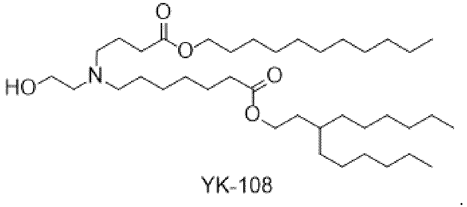

35. A composition comprising a carrier, wherein the carrier comprises a cationic lipid, and the cationic lipid comprises the compound of formula (I) or an N-oxide, a solvate, a pharmaceutically acceptable salt or a stereoisomer thereof according to any one of the preceding claims.

36. The composition according to claim 35, wherein the molar ratio of the cationic lipid to the carrier is 25% to 75%.

37. The composition according to any one of claims 35 to 36, wherein the carrier further comprises a neutral lipid.

38. The composition according to claim 37, wherein the molar ratio of the cationic lipid to the neutral lipid is 1:1 to 15:1, preferably 4:1.

39. The composition according to any one of claims 37 to 38, wherein the neutral lipid comprises one or more of phosphatidylcholine, phosphatidylethanolamine, sphingomyelin, ceramide, sterol and a derivative thereof.

40. The composition according to any one of claims 37 to 39, wherein the neutral lipid is selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and a a mixture thereof.

41. The composition according to claim 40, wherein the neutral lipid is DOPE and/or DSPC.

42. The composition according to any one of claims 35 to 41, wherein the carrier further comprises a structured lipid.

43. The composition according to claim 42, wherein the molar ratio of the cationic lipid to the structured lipid is from 0.6:1 to 3:1.

44. The composition according to any one of claims 42 to 43, wherein the structured lipid is selected from one or more of cholesterol, nonsterol, sitosterol, ergosterol, campesterol, stigmasterol, brassicasterol, tomatine, tomatine, ursolic acid, alpha-tocopherol, and corticosteroid.

45. The composition according to any one of claims 42 to 44, wherein the structured lipid is cholesterol.

46. The composition according to any one of claims 35 to 45, wherein the carrier further comprises a polymer-conjugated lipid.

47. The composition according to claim 46, wherein the molar ratio of the polymer-conjugated lipid to the carrier is 0.5% to 10%, preferably 1.5%.

48. The composition according to any one of claims 46 to 47, wherein the polymer-conjugated lipid is selected from one or more of PEG-modified phosphatidylethanolamine, PEG-modified phosphatidic acid, PEG-modified ceramide, PEG-modified dialkylamine, PEG-modified diacylglycerol, and PEG-modified dialkylglycerol.

49. The composition according to any one of claims 46 to 48, wherein the polymer-conjugated lipid is selected from one or more of distearoyl phosphatidylethanolamine polyethylene glycol 2000 (DSPE-PEG2000), dimyristoylglycero-3-methoxy polyethylene glycol 2000 (DMG-PEG2000) and methoxypolyethylene glycol ditetradecylacetamide (ALC-0159).

50. The composition according to any one of claims 35 to 49, wherein the carrier comprises a neutral lipid, a structured lipid and a polymer-conjugated lipid, and the molar ratio of the cationic lipid, the neutral lipid, the structured lipid and the polymer-conjugated lipid is (25 to 75):(5 to 25):(15 to 65):(0.5 to 10).

51. The composition according to claim 50, wherein the molar ratio of the cationic lipid, the neutral lipid, the structured lipid, and the polymer-conjugated lipid is (30 to 49):(7.5 to 15):(35 to 55):(1 to 5).

52. The composition according to any one of claims 50 to 51, wherein the molar ratio of the cationic lipid, the neutral lipid, the structured lipid, and the polymer-conjugated lipid is 40:10:48.5:1.5.

53. The composition according to any one of claims 35 to 52, wherein the composition is a nanoparticle formulation which has an average particle size of 10 nm to 300 nm and a polydispersity coefficient less than or equal to 50%.

54. The composition according to claim 53, wherein the nanoparticle formulation has an average particle size of 90 nm to 260 nm and a polydispersity coefficient less than or equal to 40 %.

55. The composition according to any one of claims 35 to 54, wherein the cationic lipid further comprises one or more other ionizable lipid compound(s).

56. The composition according to any one of claims 35 to 55, further comprising a therapeutic agent or a prophylactic agent.

57. The composition according to claim 56, wherein the mass ratio of the carrier to the therapeutic agent or prophylactic agent is 10:1 to 30:1.

58. The composition according to claim 57, wherein the mass ratio of the carrier to the therapeutic agent or prophylactic agent is 12.5:1 to 20:1.

59. The composition according to claim 58, wherein the mass ratio of the carrier to the therapeutic agent or prophylactic agent is 15: 1.

60. The composition according to any one of claims 56 to 59, wherein the therapeutic agent or prophylactic agent comprises one or more of a nucleic acid molecule, a small molecule compound, a polypeptide or a protein.

61. The composition according to any one of claims 56 to 60, wherein the therapeutic agent or prophylactic agent is a vaccine or compound capable of eliciting an immune response.

62. The composition according to any one of claims 56 to 61, wherein the therapeutic agent or prophylactic agent is a nucleic acid.

63. The composition according to any one of claims 56 to 62, wherein the therapeutic agent or prophylactic agent is ribonucleic acid (RNA).

64. The composition according to any one of claims 56 to 62, wherein the therapeutic agent or prophylactic agent is deoxyribonucleic acid (DNA).

65. The composition according to claim 63, wherein the RNA is selected from the group consisting of small interfering RNA (siRNA), asymmetric interfering RNA (aiRNA), microRNA (miRNA), Dicer-substrate RNA (dsRNA), small hairpin RNA (shRNA), messenger RNA (mRNA), and a mixture thereof.

66. The composition according to claim 65, wherein the RNA is mRNA.

67. The composition according to any one of claims 35 to 66, wherein the composition further comprises one or more of pharmaceutically acceptable excipients or diluents.

68. Use of the compound of formula (I), or an N-oxide, a solvate, a pharmaceutically acceptable salt or a stereoisomer thereof according to any one of claims 1 to 34, or the composition according to any one of claims 35 to 67 in the manufacture of a nucleic acid medicine, a gene vaccine, a small molecule medicine, a polypeptide or a protein medicine.

69. Use of the compound of formula (I), or an N-oxide, a solvate, a pharmaceutically acceptable salt or a stereoisomer thereof according to any one of claims 1 to 34, or the composition according to any one of claims 35 to 67 in the manufacture of a medicament for treating a disease or condition in a mammal in need thereof.

70. The use according to claim 69, wherein the disease or condition is **characterized by** dysfunctional or abnormal protein or polypeptide activity.

71. The use according to any one of claims 69 to 70, wherein the disease or condition is selected from the group consisting of infectious diseases, cancer and proliferative diseases, genetic diseases, autoimmune diseases, diabetes, neurodegenerative diseases, cardiovascular and renovascular diseases, and metabolic diseases.

72. The use according to claim 71, wherein the infectious disease is selected from the group consisting of diseases caused by coronavirus, influenza virus or HIV virus, infantile pneumonia, Rift Valley fever, yellow fever, rabies, and various herpes.

73. The use according to any one of claims 69 to 72, wherein the mammal is a human.

74. The use according to any one of claims 69 to 73, wherein the composition is administered intravenously, intramuscularly, intradermally, subcutaneously, intranasally or by inhalation.

75. The use according to claim 74, wherein the composition is administered subcutaneously.

76. The use according to any one of claims 69 to 75, wherein the therapeutic agent or prophylactic agent is administered to the mammal at a dose of about 0.001 mg/kg to about 10 mg/kg.
